# EUROPEAN PATENT APPLICATION

(11) **EP 2 599 858 A2**
(43) Date of publication of application: **05.06.2013**
(21) Application number: 12198243.3
(22) Date of filing: 29.01.2007
(51) Int. Cl.: C12N 5/00, A61L 27/18, A61L 27/50, D01D 5/00

(54) **Biomimetic scaffolds**

(30) Priority: 27.01.2006 US 763111 P; 09.06.2006 US 804350 P; 30.11.2006 US 861780 P
(62) Divisional of application: 07710373.7
(71) Applicant: The Regents of The University of California, Oakland, CA 94607-5200 (US)
(72) Inventor: Li, Song, Orinda, CA 94563 (US); Patel, Shyam, Oakland, CA 94610 (US); Hashi, Craig, Berkeley, CA 94703 (US); Huang, Ngan Fong, Mountain View, CA 94043 (US); Kurpinski, Kyle, Berkeley, CA 94703 (US)
(74) Representative: Harrison Goddard Foote

(57) **Abstract**

The invention provides a composition comprising a nanofiber polymer in which the fibers of the nanofiber polymer are aligned, and a molecule is covalently attached, either directly or through a linker, to the nanofiber polymer. This molecule is capable of either covalently or non-covalently attaching to a member selected from an extracellular matrix component, a growth factor, and combinations thereof. The invention also provides methods of making the composition and methods of using the compositions to add new tissue to a subject, such as a human.

## Description

### CROSS-REFERENCE TO RELATED APPLICATIONS

This application claims priority to United States Provisional Patent Application Serial No. 60/861,780 filed on November 30, 2006, 60/804,350 filed on June 9, 2006 and 60/763,111, filed on January 27, 2006, which is herein incorporated by reference in its entirety for all purposes.

### BACKGROUND OF THE INVENTION

There is a need in the art for compositions that can replace or improve biological functions in a subject. There is also a need in the art for compositions which can promote the growth of new tissue or replace damaged tissue in a subject. These and other needs are addressed by the inventions described herein.

### SUMMARY OF THE INVENTION

In a first aspect, the invention provides a composition comprising a first fibrous polymer scaffold, wherein the fiber or fibers of the first fibrous polymer scaffold are aligned. In an exemplary embodiment, wherein the first fibrous polymer scaffold has a length which is a member selected from about 0.01 cm to about 20 cm, about 0.05 cm to about 5 cm, about 0.5 cm to about 5 cm, about 1 cm to about 5 cm, about 2 cm to about 5 cm, about 1 cm to about 3 cm, about 2 cm to about 10 cm, and about 5 cm to about 15 cm. In another exemplary embodiment, the composition has a shape which is a member selected from a sheet, a conduit, a filled conduit and a rod. In another exemplary embodiment, the composition has a shape which is a member selected from a conduit, a filled conduit and a rod. In another exemplary embodiment, the composition has a rod shape. In another exemplary embodiment, said first fibrous polymer scaffold is essentially aligned in a direction which is a member selected from longitudinal and circumferential. In another exemplary embodiment, the first fibrous polymer scaffold has a seam. In another exemplary embodiment, the first fibrous polymer scaffold is seamless. In another exemplary embodiment, the first fibrous polymer scaffold is monolithically formed. In another exemplary embodiment, at least one of the fibers of the first fibrous polymer scaffold comprises a polymer or subunit which is a member selected from an aliphatic polyester, a polyalkylene oxide, polydimethylsiloxane, polycaprolactone, polylysine, collagen, laminin, fibronectin, elastin, alginate, fibrin, hyaluronic acid, proteoglycans, polypeptides and combinations thereof. In another exemplary embodiment, the aliphatic polyester is a member selected from lactic acid (D- or L-), lactide, poly(lactic acid), poly(lactide) glycolic acid, poly(glycolic acid), poly(glycolide), glycolide, poly(lactide-co-glycolide), poly(lactic acid-co-glycolic acid) and combinations thereof. In another exemplary embodiment, at least one of the fibers of the first fibrous polymer scaffold comprises poly(lactide-co-glycolide) (PLGA).

In another exemplary embodiment, the polyalkylene oxide is a member selected from polyethylene oxide, polypropylene oxide and combinations thereof. In another exemplary embodiment, the invention further comprises a cell. In another exemplary embodiment, the cell is embedded within, or is on the surface of the first fibrous polymer scaffold. In another exemplary embodiment, the cell is a member selected from a stem cell and a progenitor cell. In another exemplary embodiment, the cell is a member selected from an adult vascular cell, vascular progenitor cell, vascular stem cell, adult muscle cell, muscle progenitor cell, muscle stem cell, adult neural cell, neural progenitor cell, neural stem cell, Schwann cell, fibroblast cell, adult skin cell, skin progenitor cell, and skin stem cell. In another exemplary embodiment, the invention further comprises a molecule which is covalently attached, either directly or through a linker, to said first fibrous polymer scaffold, and said molecule is capable of either covalently or non-covalently attaching to a member selected from an extracellular matrix component, a growth factor, a differentiation factor and combinations thereof. In another exemplary embodiment, the molecule is covalently attached through a linker, and said linker is a member selected from di-amino poly(ethylene glycol), poly(ethylene glycol) and combinations thereof. In another exemplary embodiment, the molecule is a member selected from heparin, heparan sulfate, heparan sulfate protcoglycan, and combinations thereof. In another exemplary embodiment, the extracellular matrix component is a member selected from laminin, collagen, fibronectin, elastin, vitronectin, fibrinogen, polylysine and combinations thereof. In another exemplary embodiment, the growth factor is a member selected from acidic fibroblast growth factor, basic fibroblast growth factor, nerve growth factor, brain-derived neurotrophic factor, insulin-like growth factor, platelet derived growth factor, transforming growth factor beta, vascular endothelial growth factor, epidermal growth factor, keratinocyte growth factor and combinations thereof. In another exemplary embodiment, the differentiation factor is a member selected from stromal cell derived factor, sonic hedgehog, bone morphogenic proteins, notch ligands, Wnt and combinations thereof. In another exemplary embodiment, said first fibrous polymer scaffold has a conduit, filled conduit or rod shape, and wherein said polymer is seamless.

In another exemplary embodiment, the composition is produced by applying a polymer solution comprising a polymer to a rotating mandrel. In another exemplary embodiment, said polymer scaffold has a sheet, conduit or filled conduit shape and is produced by an electrospinning process comprising a rotating mandrel with at least one non-conducting region. In another exemplary embodiment, said polymer scaffold has a rod shape and is produced by an electrospinning process comprising a rotating mandrel with an air gap.

In another exemplary embodiment the invention provides a pharmaceutical composition comprising:(a) a composition described herein; and(b) a pharmaceutically acceptable excipient. In another exemplary embodiment, the composition is a rod or a conduit and wherein at least one of the fibers of the first fibrous polymer scaffold comprises poly(lactide-co-glycolide) (PLGA). In another exemplary embodiment, the composition has a length of between about 0.5 cm and 50 cm. In another exemplary embodiment, the invention further comprises a sleeve which surrounds the first fibrous polymer scaffold. In another exemplary embodiment, the sleeve comprises a second fibrous polymer scaffold, and said second fibrous polymer scaffold is aligned or has a random orientation. In another exemplary embodiment, the invention further comprises a first sleeve which surrounds a first end of the first fibrous polymer scaffold and a second sleeve which surrounds a second end of the first fibrous polymer scaffold.

In another aspect the invention provides a method of treating an injury in a subject, said method comprising:(i) applying a composition described herein to a site of interest for said subject, in an amount, and under conditions, sufficient to treat said injury. In another exemplary embodiment, the said injury is a member selected from a severed nerve, a damaged nerve, a severed muscle, a damaged muscle, a severed blood vessel, a damaged blood vessel, a skin wound and bruised skin. In another exemplary embodiment, the injury involves a severed nerve, said first fibrous polymer scaffold has a conduit, filled conduit or rod shape comprising a first end and a second end, and said severed nerve comprises a first nerve stump and a second nerve stump, said applying comprises:(ii) attaching said first end of said composition to said first nerve stump; and (iii) attaching said second end of said composition to said second nerve stump.

In another exemplary embodiment, the said injury involves a damaged nerve, and said applying comprises a member selected from: (ii) wrapping the composition described herein around said damaged nerve, wherein said composition has a sheet shape. In another exemplary embodiment, the injury involves a damaged nerve, and said applying comprises a member selected from: (ii) inserting the composition into said damaged nerve, wherein said first fibrous polymer scaffold has a rod, conduit or filled conduit shape. In another exemplary embodiment, the invention provides a method of enhancing nerve growth in a subject, said method comprising: (i) applying the composition described herein to a nerve site of interest in said subject, in an amount, and under conditions, sufficient to enhance nerve growth. In another exemplary embodiment, the injury involves cut skin or bruised skin, said first fibrous polymer scaffold has a sheet shape, and said applying comprises: (i) attaching said composition to said cut skin; thereby treating said injury. In another aspect, the invention provides a method of enhancing skin growth in a subject, wherein said first fibrous polymer scaffold has a sheet shape, said method comprising:(i) applying the composition described herein to a skin site of interest in said subject, in an amount, and under conditions, sufficient to enhance skin growth.

In another exemplary embodiment, the injury involves a severed blood vessel, said first fibrous polymer scaffold has a conduit or filled conduit shape comprising a first end and a second end, and said severed blood vessel comprises a first vessel stump and a second vessel stump, said applying comprises: (ii) attaching said first end of said composition to said first vessel stump; and (iii) attaching said second end of said composition to said second vessel stump. In another aspect, the invention provides a method of enhancing blood vessel growth in a subject, said method comprising: (i) applying the composition described herein to a vessel site of interest in said subject, in an amount, and under conditions, sufficient to enhance blood vessel growth

In another exemplary embodiment, the injury involves a severed muscle, said first fibrous polymer scaffold has a conduit, filled conduit or rod shape comprising a first end and a second end, and said severed muscle comprises a first muscle stump and a second muscle stump, said applying comprises: (ii) attaching said first end of said composition to said first muscle stump; and (iii) attaching said second end of said composition to said second muscle stump. In another exemplary embodiment, the injury involves a damaged muscle, and said applying comprises a member selected from: (ii) wrapping the composition described herein around said damaged muscle, wherein said composition has a sheet shape. In another exemplary embodiment, the injury involves a damaged muscle, and said applying comprises a member selected from: (ii) inserting the composition into said damaged muscle, wherein said first fibrous polymer scaffold has a rod, conduit or filled conduit shape. In another aspect the invention provides a method of enhancing muscle growth in a subject, said method comprising:(i) applying the composition described herein to a muscle site of interest in said subject, in an amount, and under conditions, sufficient to enhance muscle growth. In another aspect the invention provides a method of making the composition described herein. In another exemplary embodiment, said method comprising:(i) subjecting a fiber or fibers to an electrospinning process, thereby making said composition. In another exemplary embodiment, wherein said electrospinning process comprises a rotating mandrel having an air gap or at least one non-conducting region.

In the second aspect, the invention provides a mandrel for an electrospinning apparatus, comprising: a first electrically conducting region; a second electrically conducting region; and a non-electrically conducting region extending between the first and the second electrically conducting region, wherein the non-electrically conducting region is dimensioned and configured to receive a fibrous polymer for the formation of a first fibrous polymer scaffold. In another exemplary embodiment, said non-electrically conducting region is a sleeve which is placed around the mandrel. In another exemplary embodiment, said non-electrically conducting region is a member selected from tape, electrical tape, teflon, and plastic. In another exemplary embodiment, said non-electrically conducting region interconnects the two conducting mandrel regions. In another exemplary embodiment, said non-electrically conducting region is a discrete portion extending between the two conducting mandrel regions. In another exemplary embodiment, said non-electrically conducting region is a member selected from teflon and plastic. In another exemplary embodiment, said non-electrically conducting region has a diameter that is a member selected from larger and smaller than said electrically conducting region.

In the third aspect, the invention provides a mandrel for an electrospinning apparatus, comprising: a first electrically conducting region and a second electrically conducting region, wherein an air gap located between the first and the second electrically conducting region forms a non-conducting region between the first and the second electrically conducting region. In another exemplary embodiment, the invention further comprising: a first non-electrically conducting sleeve which is positioned over at least part of the first electrically conducting portion, and a second non-electrically conducting sleeve which is positioned over at least part of the second electrically conducting portion. In another exemplary embodiment, the mandrel with a non-conducting region is in combination with an electrospinning system. In another exemplary embodiment, the mandrel with an air gap is in combination with an electrospinning system.

### BRIEF DESCRIPTION OF THE DRAWINGS

**FIG. 1** refers to a schematic of the electrospinning apparatus with mandrel **56A.**

**FIG. 2** refers to a perspective view of the electrospinning apparatus with mandrel **56A.**

**FIG. 2A** refers to a portion of the electrospinning apparatus with mandrel **56A** forming polymer scaffold **90.**

**FIG. 2B** refers to a portion of the electrospinning apparatus with mandrel **56A** forming polymer scaffold **90.**

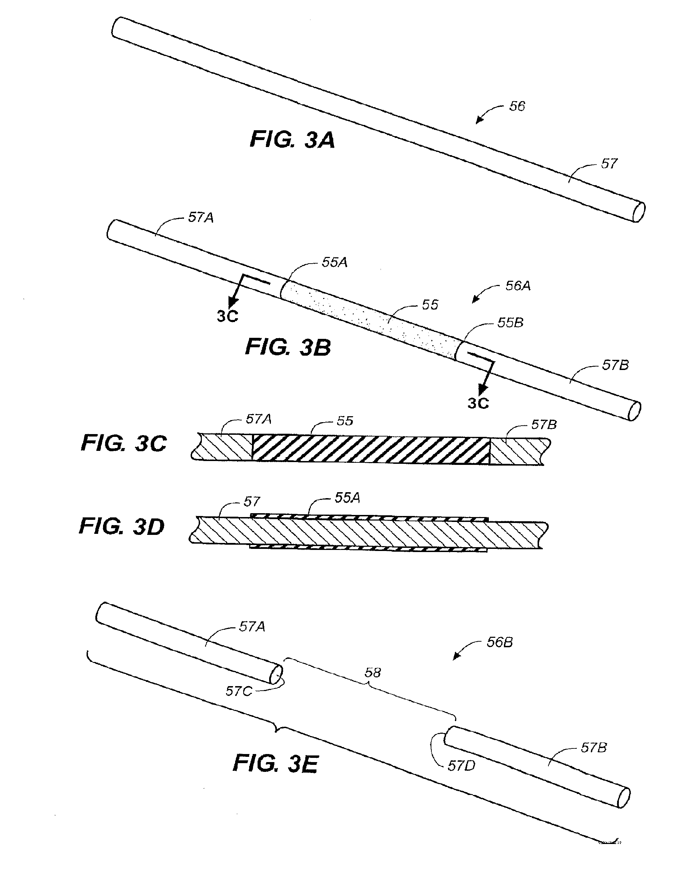

**FIG. 3** illustrates various mandrel designs used for fabricating fibrous polymer scaffolds. (A) mandrel **56** in which the entire surface is conducting; (B) mandrel **56A** with a first conducting region **57A,** a second conducting region **57B,** a non-conducting region **55,** a first interface **55A,** and a second interface **55B;** (C) cross section of mandrel **56A** in which non-conducting region **55** interconnects the two conducting mandrel regions; (D) cross section of mandrel **56A** in which non-conducting region **55A** is a sleeve which covers a portion of the surface of the conducting portion **57;** (E) mandrel **56B** with a first conducting region **57A,** a second conducting region **57B,** a first conducting region face **57C,** a second conducting region face **57D.** The conduction regions are separated by an air gap **58.**

**FIG. 4A** is an illustration of a conduit polymer scaffold composed of longitudinally aligned micro/nanofibers. **FIG. 4B** is an illustration of a rod polymer scaffold composed of longitudinally aligned micro/nanofibers. Note: fiber dimensions not drawn to scale.

**FIG. 5A** is an illustration showing a cross section of the conduit in **FIG. 4A****.** **FIG. 5B** is an illustration showing a cross section of the rod in **FIG. 4****B.**

**FIG. 6** refers to a schematic of the electrospinning apparatus with mandrel **56B.**

**FIG. 7** refers to a perspective view of the electrospinning apparatus with mandrel **56B.**

**FIG. 7A** refers to a portion of the electrospinning apparatus with mandrel **56B** forming polymer scaffold **92.**

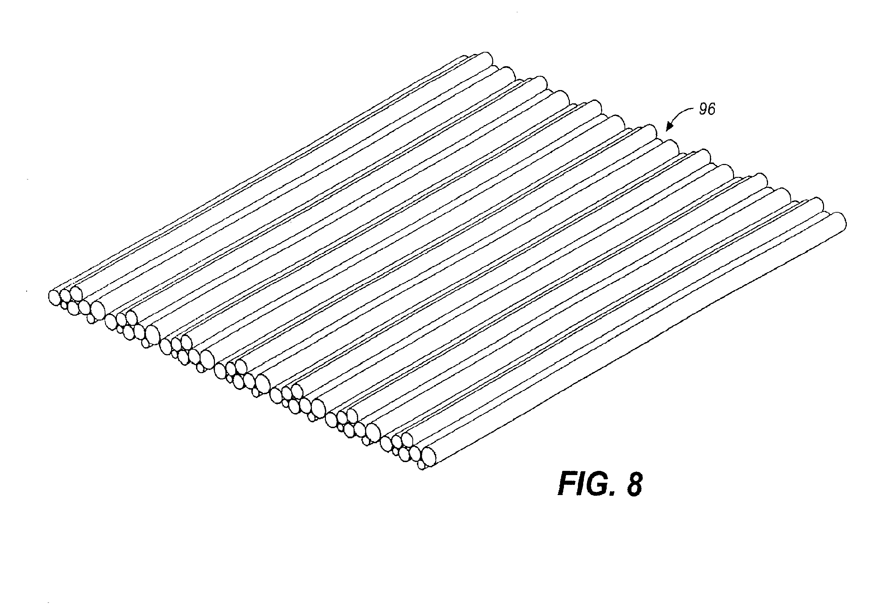

**FIG. 8** is an illustration of a longitudinally aligned polymer scaffold sheet **96.**

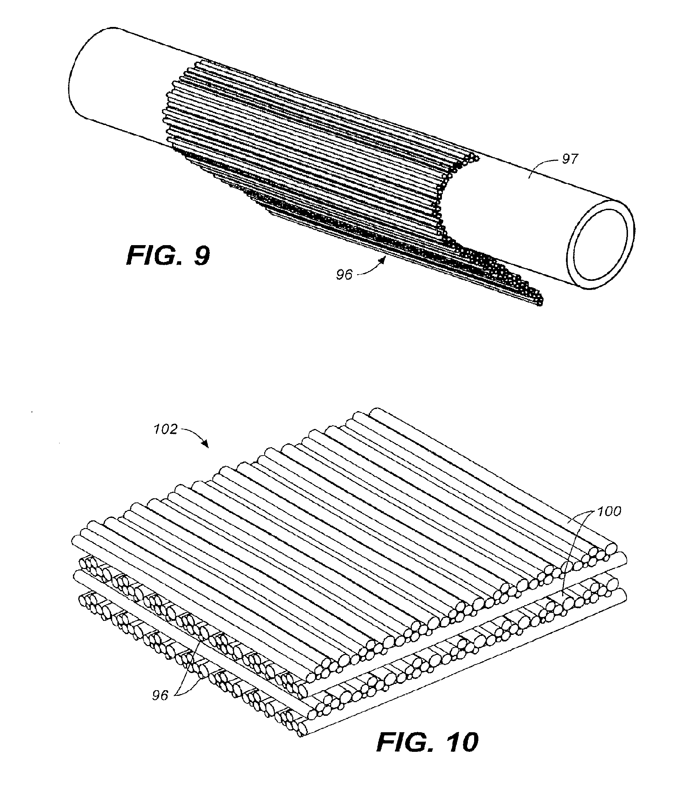

**FIG. 9** is a schematic diagram showing the rolling process for creating a fibrous polymer conduit scaffold with a seam from an aligned polymer scaffold sheet. Here a longitudinally aligned polymer scaffold sheet **96** is rolled around a rod **97** and later sutured or adhered.

**FIG. 10** is an illustration of a 'criss-cross' sheet **102** which comprises aligned sheets **96** and **100.**

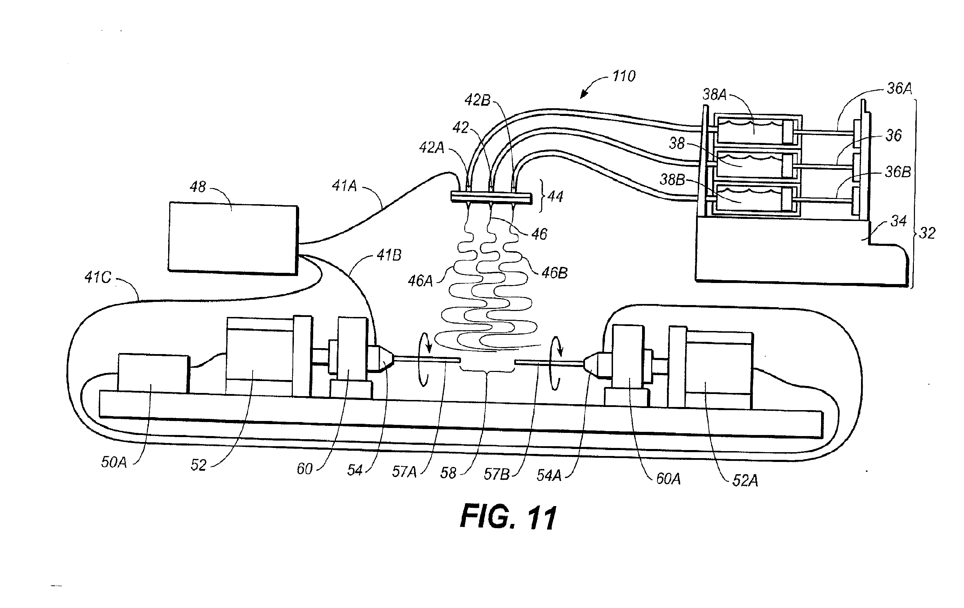

**FIG. 11** refers to a schematic of a multiple spinneret electrospinning apparatus **110** with mandrel **56B.** The polymer solutions **38, 38A** and **38B** contain the polymer dissolved in a solvent, are contained within syringe assemblies **36, 36A** and **36B,** respectively. The syringe assemblies are part of a syringe pump assembly **32** in which a computer **34** controls the rate at which the polymer solution exits the syringe by controlling pressure or flow rate. Optionally, different flow rates can be provided and controlled to selected spinnerets. The flow rate will change depending on the desired physical characteristics of the polymer scaffold, i.e., membrane thickness, fiber diameter, pore size, membrane density, etc.

The syringe pump assembly **32** feeds the polymer solutions to spinnerets **42, 42A** and **42B** that sit on a platform **44.** The spinnerets have a tip geometry which allows for jet formation and transportation, without interference. A charge in the range of about 10 to about 30 kV is applied to the spinnerets by a high voltage power supply **48** through wire **41A.**

A mandrel **56B** (which, as mentioned in **FIG. 3B**, includes **57A, 57B** and **58)** is positioned below the spinnerets **42, 42A** and **42B** such that an electric field is created between the charged spinneret and the mandrel **56A.** The electric field causes a jet of the polymer solution to be ejected from the spinnerets and spray towards the mandrel **56B,** forming micron or nanometer diameter filaments or fibers **46, 46A** and **46B.** The drill chucks are grounded using ground wires **41B** and **41C.**

The mandrel **56B** is attached to a first drill chuck **54** (attached to a non-conducting bearing **60)** and a second drill chuck **54A** (attached to a non-conducting bearing **60A)** which is connected to a motor **52.** The motor **52** is linked to a speed control **50A** which controls the rate at which the motor spins the mandrel **56B.** Optionally, different spin rates can be provided. The spin rate will change depending on the desired physical characteristics of the polymer scaffold, i.e., membrane thickness, fiber diameter, pore size, membrane density, etc.

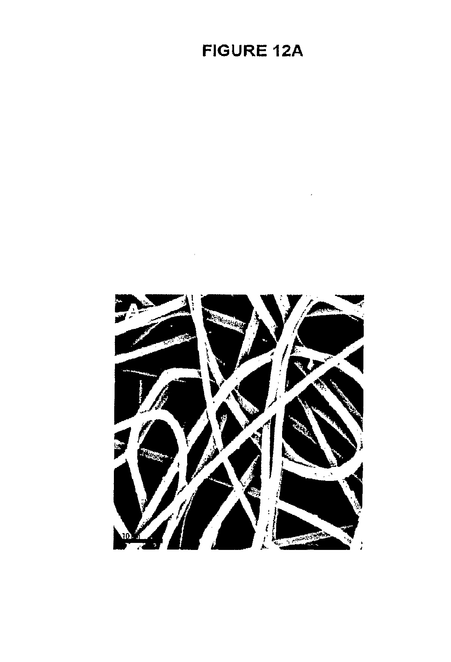

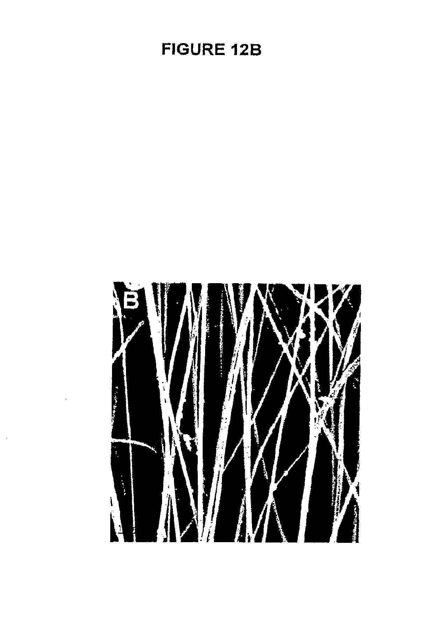

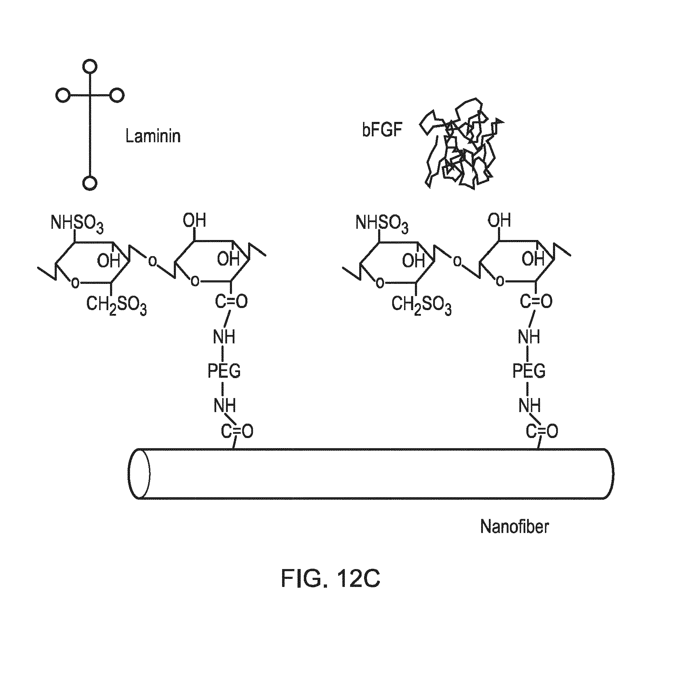

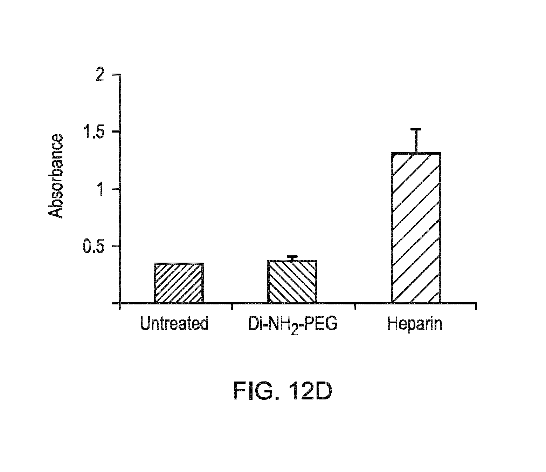

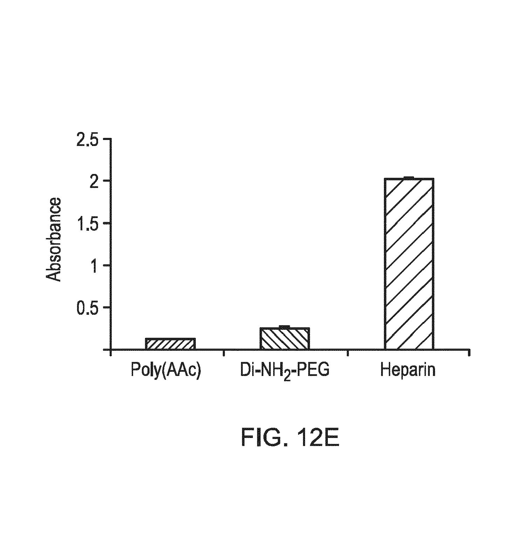

**FIG. 12** SEM images of unaligned (A) and aligned (B) PLLA nanofibcrs. (C) Illustration showing chemical modification of PLLA nanofibers with heparin and noncovalent attachment of bFGF and laminin. A modified ELISA technique was used to show the relative levels of bFGF attachment on untreated, di-NH₂-PEG modified and heparin functionalized PLLA nanofibers (D) and poly(acrylic acid) coated polystyrene surfaces (E).

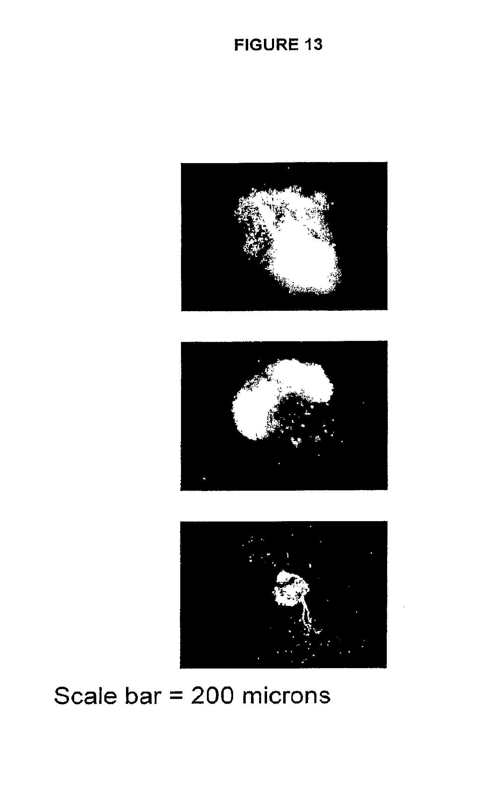

**FIG. 13** Neurite extension from DRG tissue on unaligned nanofibers. Immunofluorescent staining of neurofilaments was used to visualize neurite extension from DRG tissue on untreated, LAM and LAM+bFGF unaligned nanofibcrs after 6 days of *ex vivo* culture. Scale bar = 200 µm.

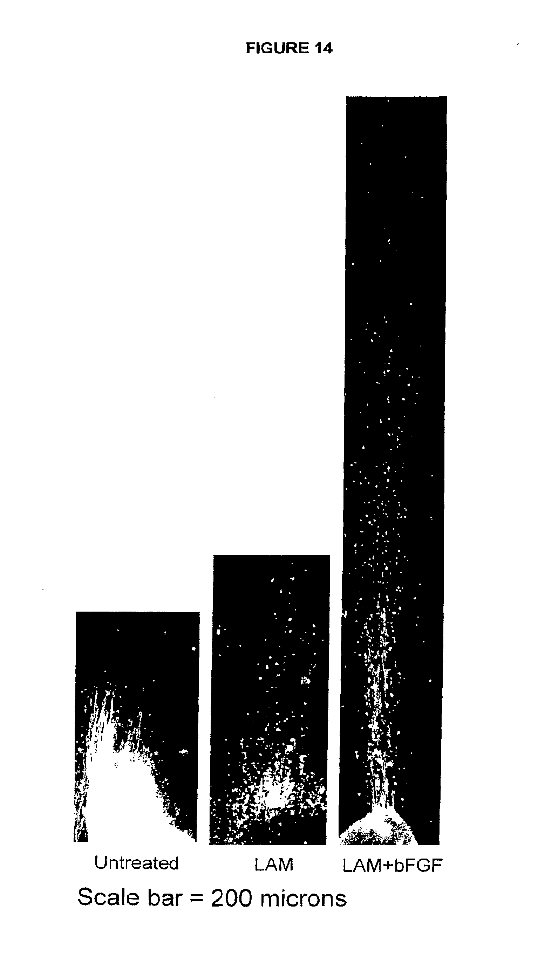

**FIG. 14** Neurite extension from DRG tissue on aligned nanofibers. Immunofluorescent staining of neurofilaments was used to visualize neurite extension from DRG tissue on untreated, LAM and LAM+bFGF aligned nanofibers after 6 days of *ex vivo* culture. Nanofibers were aligned in the vertical direction. Scale bar = 200 µm.

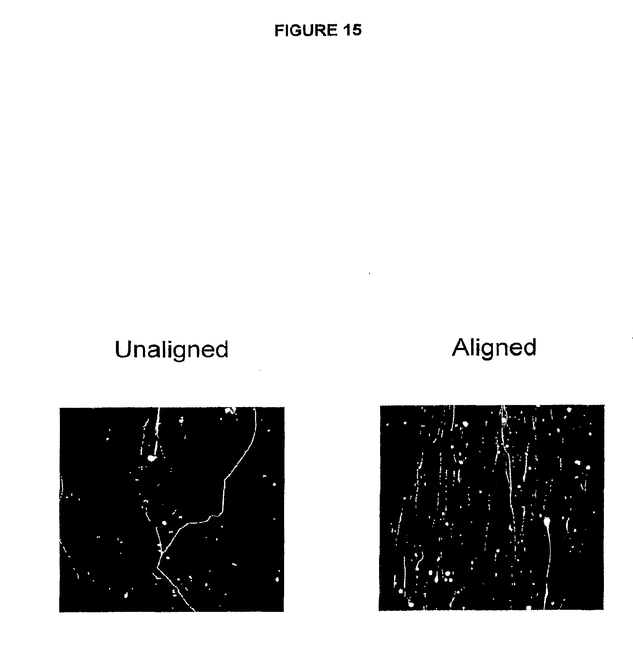

**FIG. 15** High magnification confocal microscopy images of neurite morphology on unaligned and aligned LAM+bFGF PLLA nanofibers. Aligned nanofibers were in vertical direction.

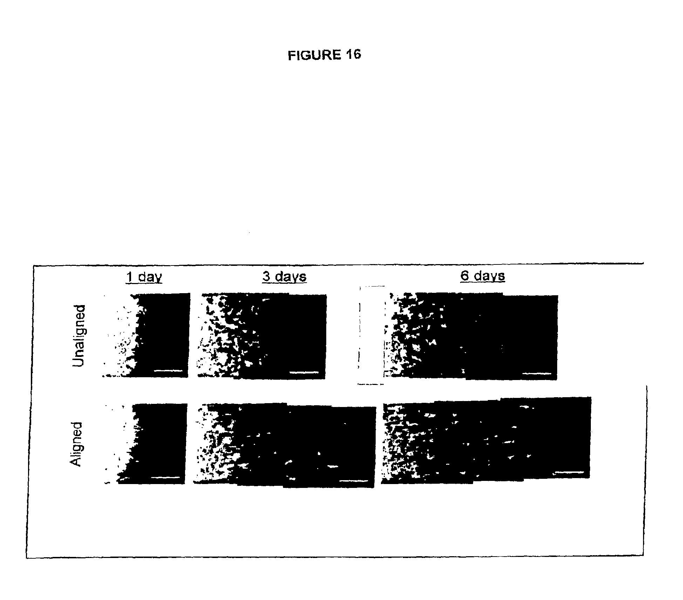

**FIG. 16** Human mesenchymal stem cells were cultured as pellets on PLLA micro/nanofiber membranes for 1, 3, or 6 days. Cells on unaligned micro/nanofibers show gradual migration over time and random alignment. Cells on aligned micro/nanofibers display enhanced migration in the direction of the fibers at 3 and 6 days as well as overall alignment with the fiber direction. Scale bars are 200 µm.

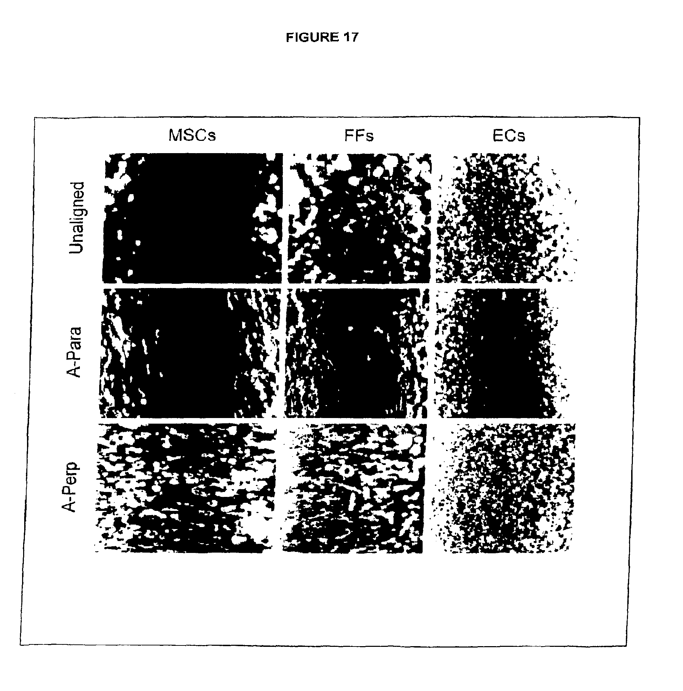

**FIG. 17** Wound healing model on micro/nanofiber scaffolds with various cell types. MSCs: mesenchymal stem cells after 2 days. FFs: foreskin fibroblasts after 5 days. ECs: endothelial cells after 1 day. On unaligned micro/nanofiber scaffolds, wound coverage ranges from minimal (MSC sample) to moderate (EC sample). When micro/nanofibers are aligned parallel to wound long axis (A-Para), cell migration into wound is heavily impaired. Cell migration and wound coverage are greatest when micro/nanofibers are aligned perpendicular to wound long axis.

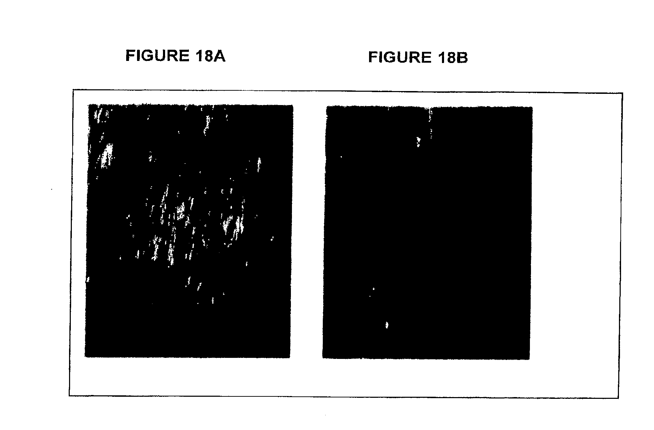

**FIG. 18** Fluorescent staining for actin (phalloidin) and nuclei (propidium iodide) demonstrating similar cytoskclctal structure between A) smooth muscle cell orientation of native *in vivo* common carotid artery and B) aligned nanofiber polymer sheet seeded with human smooth muscle cells.

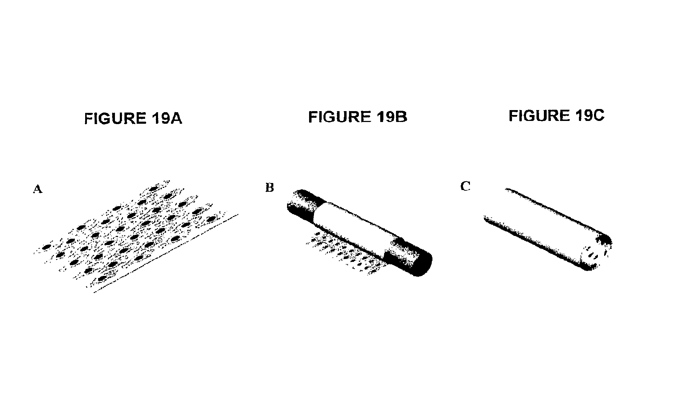

**FIG. 19** Construction of nanofibrous, stem cell embedded vascular graft. A) Stem cells are seeded onto an aligned sheet of biodegradable nanofibers. B) A tubular structure is created by rolling the sheet around the rod. C) The rod is removed and sutures are used maintain the shape of the graft.

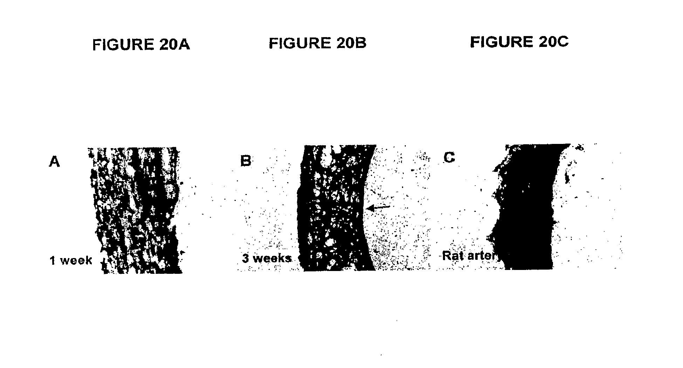

**FIG. 20** Verhoeff's Staining of the cross-sections of vascular grafts and rat artery. Collagen (red) and clastin (black) fiber production is significantly improved from 1 to 3 weeks. By 3 weeks, the tissue engineered vascular graft has strong similarities to the native rat artery.

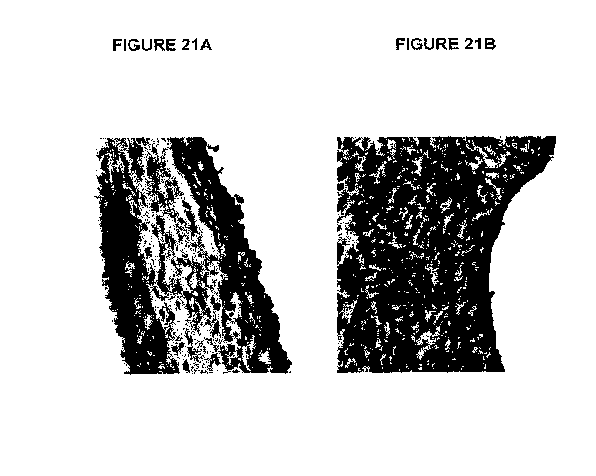

**FIG. 21** Immunohistochemical staining (brown) of the cross-sections for GD31 (an endothelial cell marker) in vascular grafts after 3-week implantation. A) Rat artery. B) Vascular graft after 3 weeks.

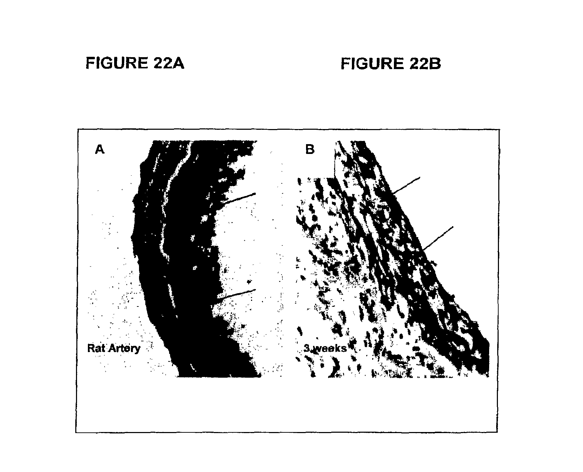

**FIG. 22** Immunohistochemical staining (brown) of cross-sections for α-actin (smooth muscle marker) in vascular grafts after 3 week-implantation. A) Rat artery. B) Vascular graft after 3 weeks.

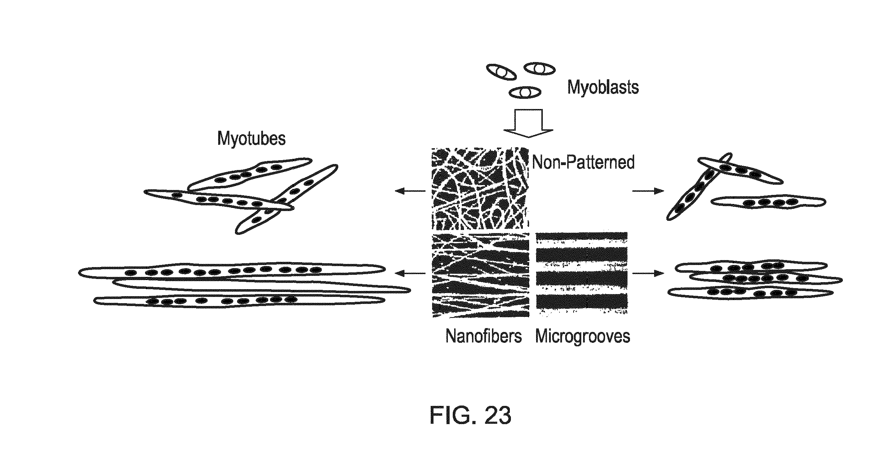

**FIG. 23** When myoblasts are grown on nonaligned or non-patterned surfaces, the myotubes form in a random manner. When myoblasts are grown on aligned nanofibers or micropatterned surfaces, the myotubes form in an aligned manner.

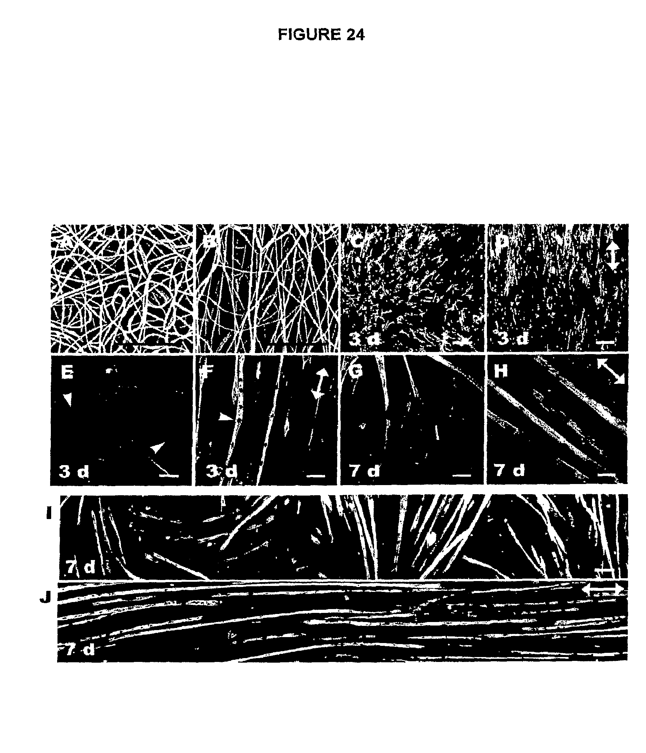

**FIG. 24** Myoblast alignment and myotube assembly on an aligned PLLA nanofibrous scaffold. SEM images show the structure of (A) randomly-oriented and (B) aligned nanofibrous scaffolds, followed by F-actin immunofluorescent staining of myoblasts on (C) randomly-oriented and (D) aligned nanofibrous scaffolds after 3 days in differentiation media. Immunofluorescence staining of skeletal MHC was performed to show myotubes on random (E, G) and aligned (F, H) nanofibrous scaffolds at 3 days (E, F) and 7 days (G, H). Low magnification merged images of skeletal MHC staining on (I) randomly-oriented and (J) aligned substrates after 7 days showing the global alignment and length of myotubes. Arrows indicate direction of nanofibers. Arrow heads in E and F indicate nuclei. Scale bars are 50 µm (A-H) and 100 µm (I, J), respectively.

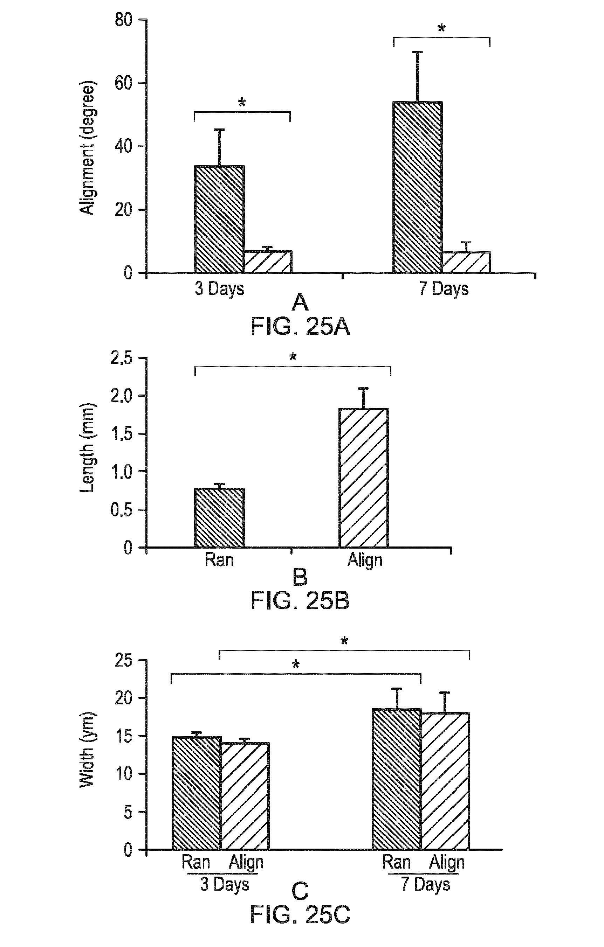

**FIG. 25** **Quantification of myotube organization and morphology on aligned nanofibrous substrates.** (A) Angle of myotube alignment in reference to nanofiber direction. (B) Myotube length after 7 days. (C) Myotube width after 7 days. * indicates statistically significant difference (P<0.05).

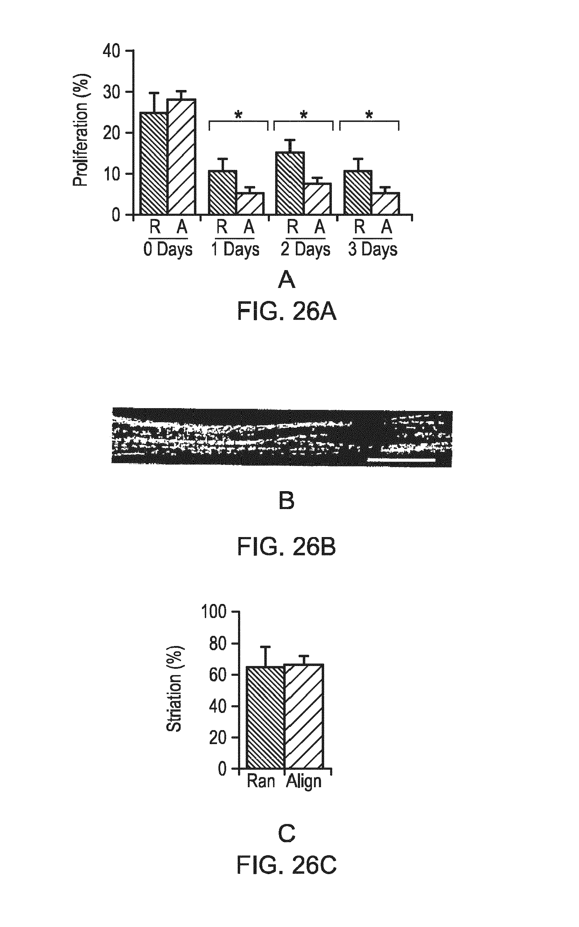

**FIG. 26** **Quantification of myoblast proliferation and myotubes striation on aligned nanofibrous scaffolds.** (A) BrdU incorporation for cell proliferation (R, Ran; A, Align). (B) Immunofluorescence staining of anti-MHC showing a striated myotube on aligned nanofibrous scaffold (Scale bar: 20 µm). (C) Quantification of the percentage of striated cells after 7 days. * indicates statistically significant difference (P<0.05).

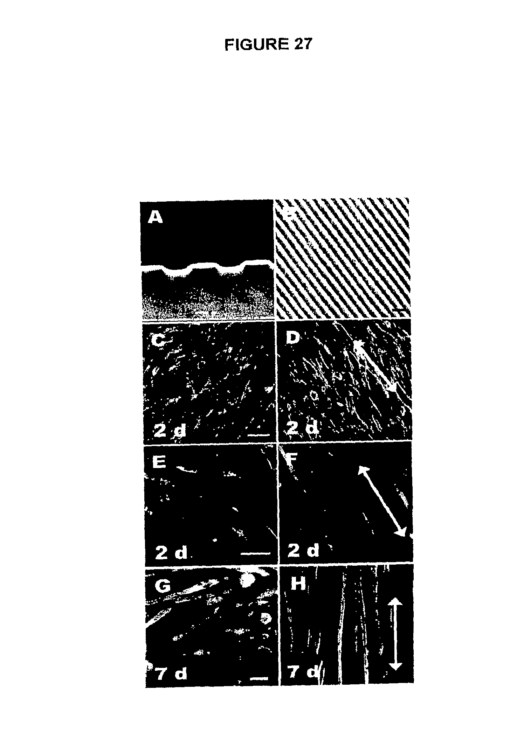

**FIG. 27** **Myoblast alignment and myotube organization on a micropatterned PDMS substrate.** A micropatterned PDMS substrate is shown by (A) SEM (side view) and. (B) phase contrast microscopy. F-actin distribution after 2 days in differentiation media is shown on (C) non-patterned and (D) micropatterned substrates. Immunofluorescent staining of skeletal MHC was performed to show cell fusion on non-patterned (E, G) and micropatterned (F, H) membranes after 2 days (E-F) and 7 days (G-H). Arrows indicate direction of microgrooves. Scale bars are 5 µm (A), 20 µm (B) and 50 µm (C-H) respectively.

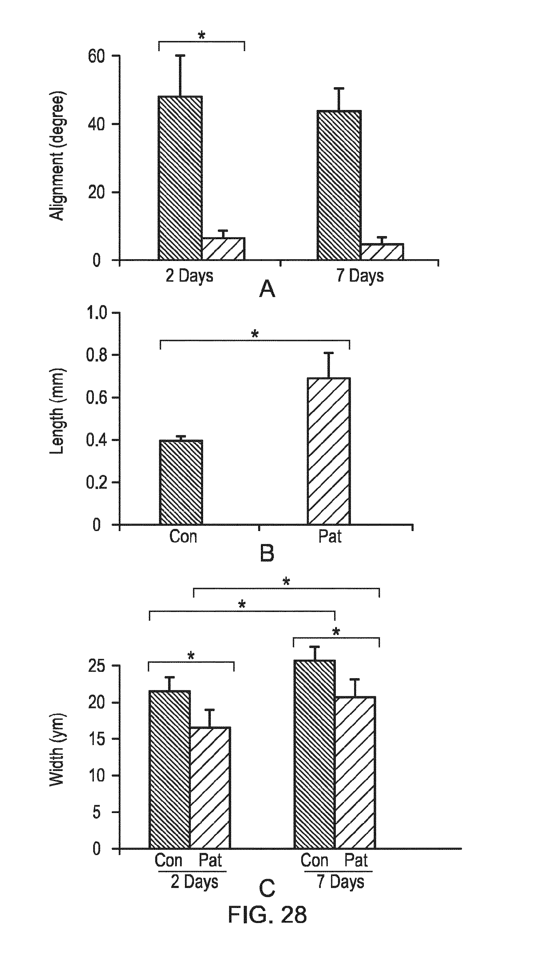

**FIG. 28** **Quantification of myotube organization and morphology on micropatterned membranes.** (A) Angle ofmyotube alignment in reference to the microgroove direction on non-patterned (Con) and micropatterned (Pat) membranes. (B) Myotube length after 7 days. (C) Myotube width after 7 days. * indicates statistically significant difference (P<0.05).

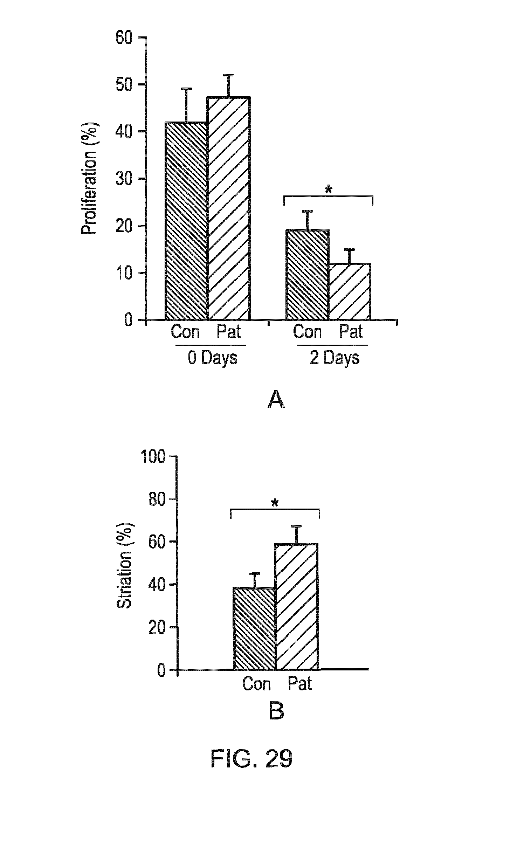

**FIG. 29** **Quantification of myoblast proliferation and striation on micropatterned PDMS membranes.** (A) BrdU incorporation for cell proliferation at the early stage of fusion on non-patterned (Con) and micropatterned (Pat) membranes. (B) Quantification of the percentage of striated cells after 7 days. * indicates statistically significant difference (P<0.05).

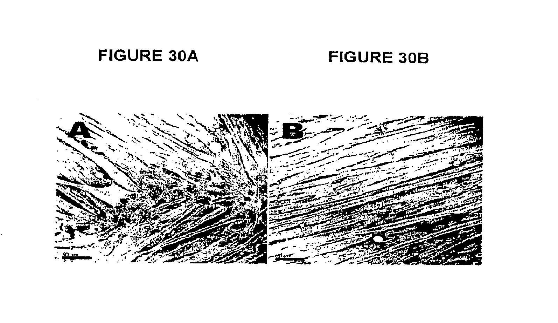

**FIG. 30** **SEM images of myotube formation on nanofibrous scaffolds.** Myotube alignment after 7 days in differentiation media on (A) randomly-oriented and (B) aligned PLLA nanofibrous scaffolds (Scale bar: 50 µm).

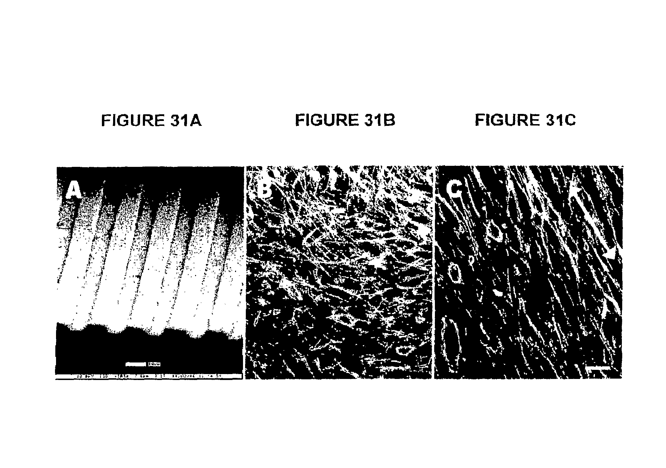

**FIG. 31** **Alignment of myoblasts on micropatterned biodegradable PLGC substrates.** (A) SEM image of topographically micropatterned grooves on a PLGC substrate. (B-C) F-actin staining of myoblasts after 5 days in differentiation media on non-patterned (B) and micropatterned (C) PLGC substrates. Note the aligned and well-organized actin stress fibers on micropatterned PLGC substrate. Scale bars are 10 µm (A) and 50 µm (B-C) respectively.

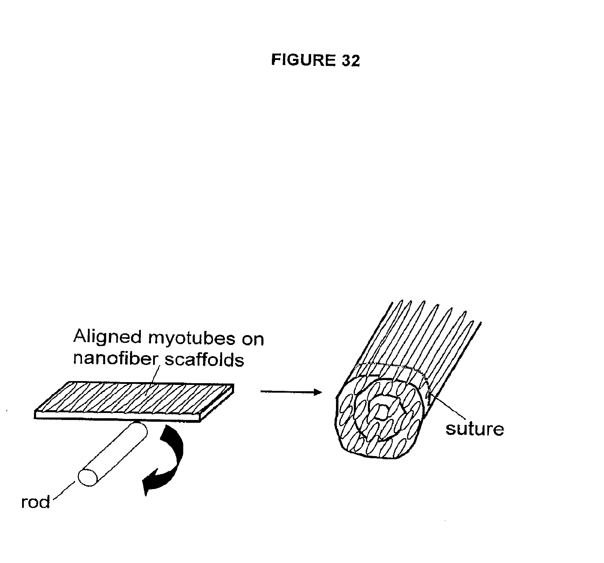

**FIG. 32** **Schematic diagram showing the rolling process for creating three-dimensional myofiber-seeded tubular scaffolds.** Myoblasts were differentiated into aligned myotubes on membranes of aligned nanofibers. After 7d of differentiation, the sheets were rolled into a tubular scaffold with a rod stem and suture-secured.

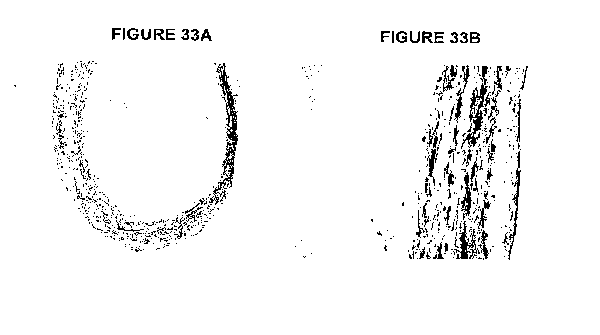

**FIG. 33** Hematoxylin and eosin (H&E) stain depicting organization of three-dimensional tubular nanofiber scaffold at low (left) and high (right) magnification.

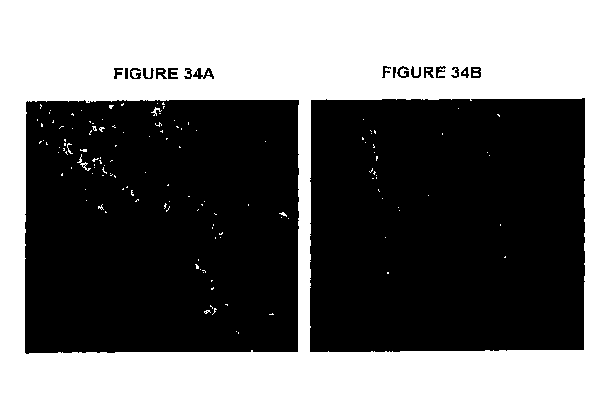

**FIG. 34** **Laser confocal microscopy depicting the cellular morphology of myoblasts and myotubes in three-dimensional tubular nanofiber scaffolds in cross-sectional (A) and long-axis (B) aspects.** The samples were immunofluorescently stained for F-actin (green) and nuclei (red).

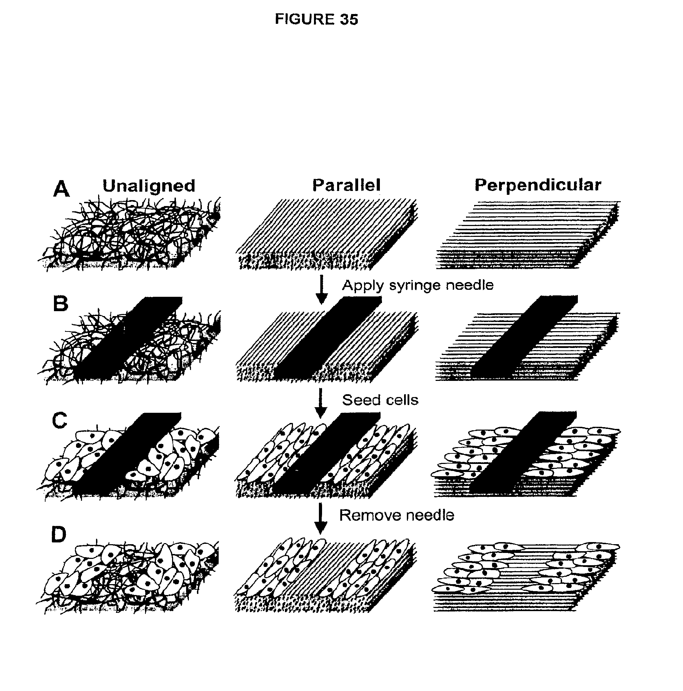

**FIG. 35** Schematic of *in vitro* wound healing model on aligned or unaligned fibers. (A) Micro/nanofibers are created as meshes with either unaligned fibers or aligned fibers that can be oriented parallel or perpendicular to the long edges of the wound. (B) A flattened 18 Gauge syringe needle is placed on the nanofiber meshes to block cell adhesion. (C) Cells are seeded on the nanofiber meshes. (D) After cells adhere to the nanofibers, the needle is removed to allow cell migration into the wound.

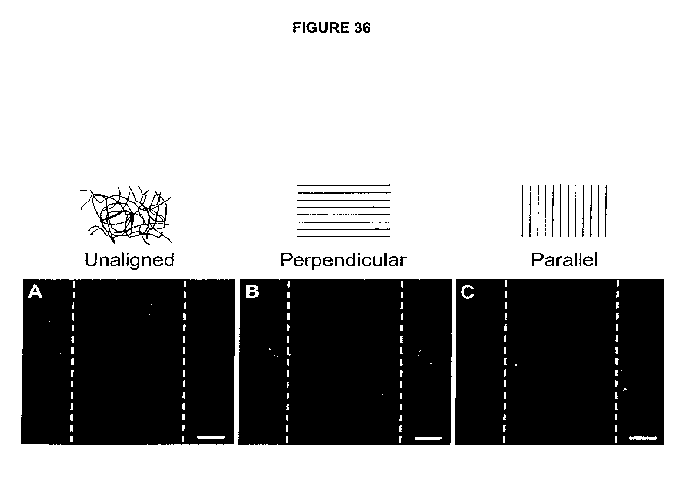

**FIG. 36** *In vitro* wound healing model with NHDFs on aligned vs. unaligned nanofibers. After 48 hours, NHDFs on unaligned micro/nanofibers (A) show moderate migration and wound coverage, and random cell alignment. When the fibers are aligned perpendicular to the edges of the wound (B), migration and wound coverage is greatly enhanced, and cells are aligned with fibers. When fibers are aligned parallel to the edges of the wound (C), wound coverage is greatly reduced. Stain is whole actin (green) and Hoechst nuclear stain (blue). Dotted white lines represent initial wound edges at 0 hours. Scale bars are 300 µm.

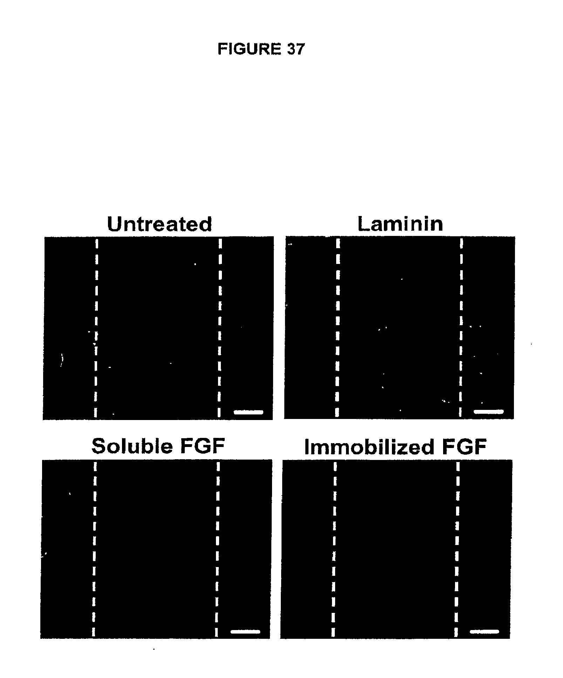

**FIG. 37** *In vitro* wound healing model with NHDFs on aligned micro/nanofibers with or without chemical modification. In all groups, micro/nanofibers were oriented perpendicular to the long edges of the wound. After 24 hours, NHDFs showed enhanced migration and wound coverage on fibers with additional chemical modification. On untreated fibers, cells did not completely cover the wound area. When laminin was added to the fibers, cells migrated more rapidly. Addition of bFGF enhanced migration even further, either in soluble form or immobilized to the micro/nanofibers. Stain is whole actin (green) and nuclei (blue). Dotted white lines represent initial wound edges at 0 hours. Scale bars are 300 µm.

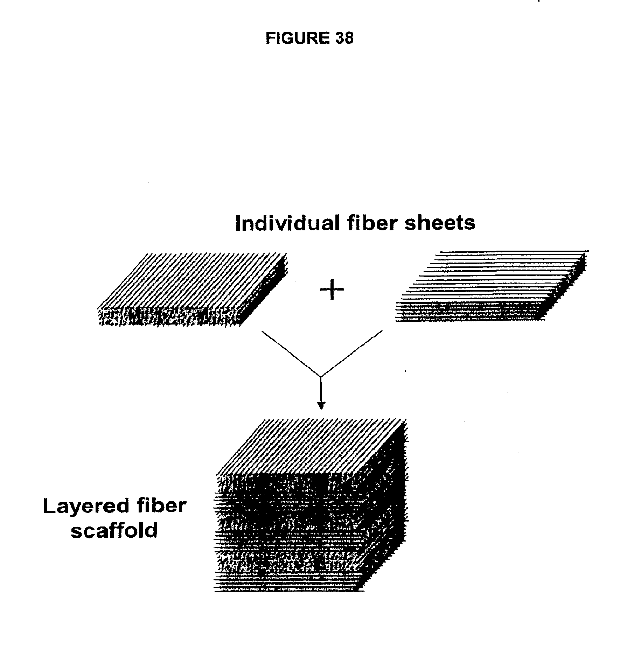

**FIG. 38** Assembly of multi-layered micro/nanofiber tissue graft. Individual micro/nanofiber sheets can be layered on top of each other to create constructs with complex architecture. This figure depicts the assembly of a graft with criss-cross fiber structure. Additional architectures can be created depending on the fiber orientation of each individual sheet.

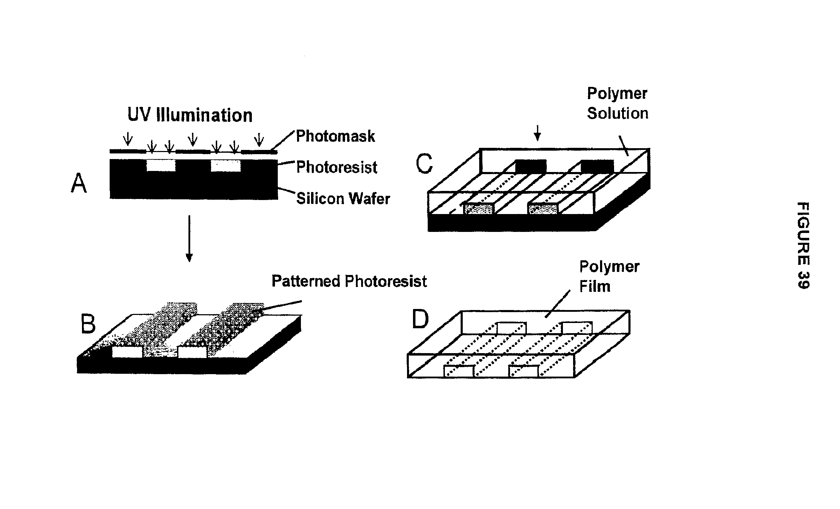

**FIG. 39** Fabrication of Micropatterned Polymer Films. (A) A negative photoresist was spin-coated on silicone wafer and exposed to UV light through a photomask. (B) Photoresist without UV-polymerization was developed away, leaving a patterned surface. (C) The polymer solution was cast onto the wafer, spin-coated, and allowed to polymerize. (D) The films were then peeled away from the silicon wafer.

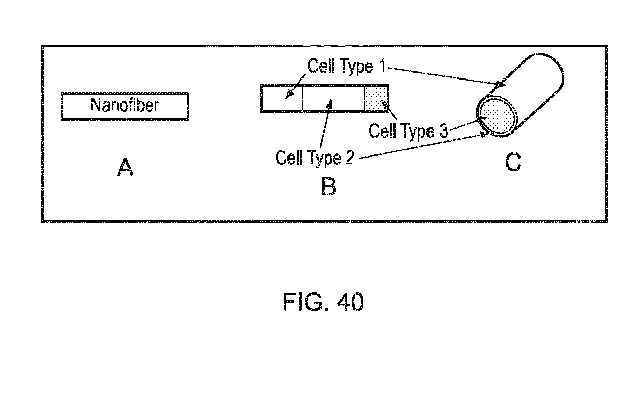

**FIG. 40** Multiple cell type graft. A) Aligned or randomly oriented nanofiber sheet. B) Seed multiple cell types on different areas of the sheet. C) Create a tubular construct with multiple cell types at different locations in the graft.

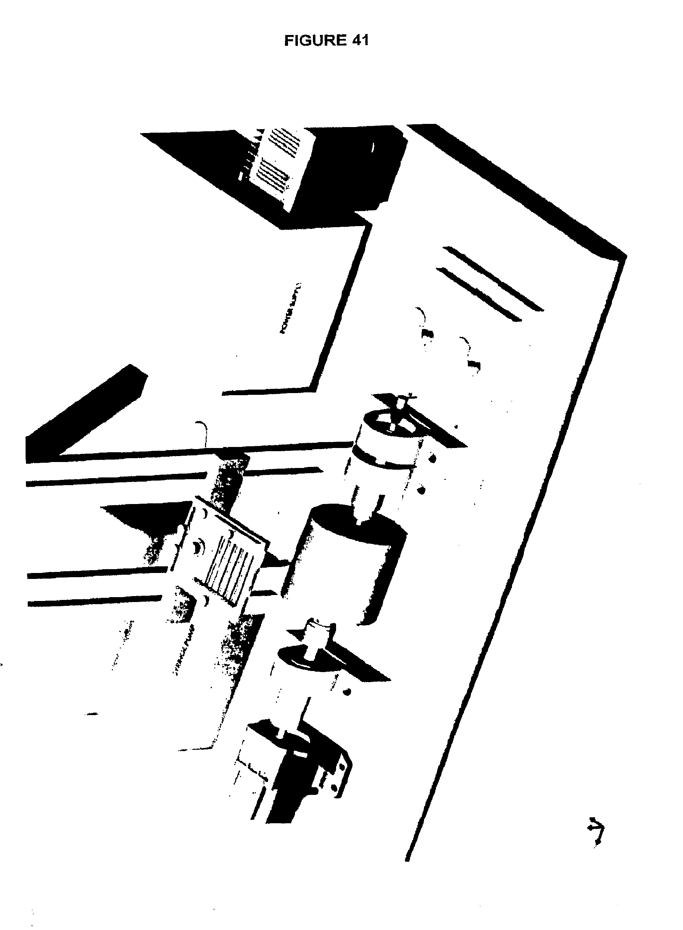

**FIG. 41** illustrates an electrospinning apparatus of the invention with a rotating drum collector.

### DETAILED DESCRIPTION OF THE PREFERRED EMBODIMENTS

### I. Definitions and Abbreviations

The abbreviations used herein generally have their conventional meaning within the chemical and biological arts.

As used herein the singular forms "a", "and", and "the" include *plural* referents unless the context clearly dictates otherwise.

As used herein, and unless otherwise indicated, a composition that is "essentially free" of a component means that the composition contains less than about 20% by weight, such as less than about 10% by weight, less than about 5% by weight, or less than about 3% by weight of that component.

"Peptide" refers to a polymer in which the monomers are amino acids and are joined together through amide bonds, alternatively referred to as a polypeptide. Additionally, unnatural amino acids, for example, β-alanine, phenylglycine and homoarginine are also included. Amino acids that are not gene-encoded may also be used in the present invention. Furthermore, amino acids that have been modified to include reactive groups, glycosylation sites, polymers, therapeutic moieties, biomolecules and the like may also be used in the invention. All of the amino acids used in the present invention may be either the D- or L-isomer. In addition, other peptidomimetics are also useful in the present invention. As used herein, "peptide" refers to both glycosylated and unglycosylated peptides. Also included are petides that are incompletely glycosylated by a system that expresses the peptide. For a general review, *see,* Spatola, A. F., in CHEMISTRY AND BIOCHEMISTRY OF AMINO ACIDS, PEPTIDES AND PROTEINS, B. Weinstein, eds., Marcel Dekker, New York, p. 267 (1983).

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g.*, hydroxyproline, γ-carboxyglutamate, and O-phosphoserine. Amino acid analogs refers to compounds that have the same basic chemical structure as a naturally occurring amino acid, *i.e.,* an α carbon that is bound to a hydrogen, a carboxyl group, an amino group, and an R group, *e.g.,* homoserine, norleucine, methionine sulfoxide, methionine methyl sulfonium. Such analogs have modified R groups (*e.g.*, norleucine) or modified peptide backbones, but retain the same basic chemical structure as a naturally occurring amino acid. Amino acid mimetics refers to chemical compounds that have a structure that is different from the general chemical structure of an amino acid, but that function in a manner similar to a naturally occurring amino acid.

As used herein, "nucleic acid" means DNA, RNA, single-stranded, doublestranded, or more highly aggregated hybridization motifs, and any chemical modifications thereof. Modifications include, but are not limited to, those providing chemical groups that incorporate additional charge, polarizability, hydrogen bonding, electrostatic interaction, points of attachment and functionality to the nucleic acid ligand bases or to the nucleic acid ligand as a whole. Such modifications include, but are not limited to, peptide nucleic acids (PNAs), phosphodiester group modifications (*e.g.*, phosphorothioates, methylphosphonates), 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, methylations, unusual base-pairing combinations such as the isobases, isocytidine and isoguanidine and the like. Nucleic acids can also include non-natural bases, such as, for example, nitroindole. Modifications can also include 3' and 5' modifications such as capping with a fluorophore (*e.g.*, quantum dot) or another moiety.

"Antibody," as used herein, generally refers to a polypeptide comprising a framework region from an immunoglobulin or fragments or immunoconjugates thereof that specifically binds and recognizes an antigen. The recognized immunoglobulins include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

As used herein, the term "copolymer" describes a polymer which contains more than one type of subunit. The term encompasses polymer which include two, three, four, five, or six types of subunits.

The term "isolated" refers to a material that is substantially or essentially free from components, which are used to produce the material. The lower end of the range of purity for the compositions is about 60%, about 70% or about 80% and the upper end of the range of purity is about 70%, about 80%, about 90% or more than about 90%.

"Hydrogel" refers to a water-insoluble and water-swellable cross-linked polymer that is capable of absorbing at least 3 times, preferably at least 10 times, its own weight of a liquid. "Hydrogel" and "thermo-responsive polymer" are used interchangeably herein.

The term "attached," as used herein encompasses interaction including, but not limited to, covalent bonding, ionic bonding, chemisorption, physisorption and combinations thereof.

The term "biomolecule" or "bioorganic molecule" refers to an organic molecule typically made by living organisms. This includes, for example, molecules comprising nucleotides, amino acids, sugars, fatty acids, steroids, nucleic acids, polypeptides, pcptidcs, peptide fragments, carbohydrates, lipids, and combinations of these (*e.g.*, glycoproteins, ribonucleoproteins, lipoproteins, or the like).

"Small molecule," refers to species that are less than 1 kD in molecular weight, preferably, less than 600 D.

"Composition of the invention," as used herein refers to the compositions discussed herein, pharmaceutically acceptable salts and prodrugs of these compositions.

Where substituent groups are specified by their conventional chemical formulae, written from left to right, they equally encompass the chemically identical substituents, which would result from writing the structure from right to left, *e.g.*, -CH₂O- is intended to also recite -OCH₂-.

By "effective" amount of a drug, formulation, or permeant is meant a sufficient amount of a active agent to provide the desired local or systemic effect. A "Topically effective," "Cosmetically effective," "pharmaceutically effective," or "therapeutically effective" amount refers to the amount of drug needed to effect the desired therapeutic result.

The term "pharmaceutically acceptable salts" is meant to include salts of the compounds of the invention which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, malcic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., "Pharmaceutical Salts", Journal of Pharmaceutical Science 66: 1-19 (1977)). Certain specific compounds of the present invention contain both basic and acidic functionalities that allow the compounds to be converted into either base or acid addition salts.

The neutral forms of the compounds are preferably regenerated by contacting the salt with a base or acid and isolating the parent compounds in the conventional manner. The parent form of the compound differs from the various salt forms in certain physical properties, such as solubility in polar solvents.

In addition to salt forms, the present invention provides compounds which are in a prodrug form. Prodrugs of the compounds or complexes described herein readily undergo chemical changes under physiological conditions to provide the compounds of the present invention. Additionally, prodrugs can be converted to the compounds of the present invention by chemical or biochemical methods in an *ex vivo* environment.

The compounds of the present invention may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present invention, whether radioactive or not, are intended to be encompassed within the scope of the present invention.

The term "pharmaceutically acceptable carrier" or "pharmaceutically acceptable vehicle" refers to any formulation or carrier medium that provides the appropriate delivery of an effective amount of a active agent as defined herein, does not interfere with the effectiveness of the biological activity of the active agent, and that is sufficiently non-toxic to the host or patient. Representative carriers include water, oils, both vegetable and mineral, cream bases, lotion bases, ointment bases and the like. These bases include suspending agents, thickeners, penetration enhancers, and the like. Their formulation is well known to those in the art of cosmetics and topical pharmaceuticals. Additional information concerning carriers can be found in Remington: The Science and Practice of Pharmacy, 21st Ed., Lippincott, Williams & Wilkins (2005) which is incorporated herein by reference.

"Pharmaceutically acceptable topical carrier" and equivalent terms refer to pharmaceutically acceptable carriers, as described herein above, suitable for topical application. An inactive liquid or cream vehicle capable of suspending or dissolving the active agent(s), and having the properties of being nontoxic and non-inflammatory when applied to the skin, nail, hair, claw or hoof is an example of a pharmaceutically-acceptable topical carrier. This term is specifically intended to encompass carrier materials approved for use in topical cosmetics as well.

The term "pharmaceutically acceptable additive" refers to preservatives, antioxidants, fragrances, emulsifiers, dyes and excipients known or used in the field of drug formulation and that do not unduly interfere with the effectiveness of the biological activity of the active agent, and that is sufficiently non-toxic to the host or patient. Additives for topical formulations are well-known in the art, and may be added to the topical composition, as long as they are pharmaceutically acceptable and not deleterious to the epithelial cells or their function. Further, they should not cause deterioration in the stability of the composition. For example, inert fillers, anti-irritants, tackifiers, excipients, fragrances, opacifiers, antioxidants, gelling agents, stabilizers, surfactant, emollients, coloring agents, preservatives, buffering agents, other permeation enhancers, and other conventional components of topical or transdermal delivery formulations as are known in the art.

As used herein, "administering" means oral administration, administration as a suppository, topical contact, intravenous, intraperitoneal, intramuscular, intralesional, intranasal or subcutaneous administration, or the implantation of a slow-release device *e.g.*, a mini-osmotic pump, to the subject.

The term "excipients" is conventionally known to mean carriers, diluents and/or vehicles used in formulating drug compositions effective for the desired use.

The term "autologous cells", as used herein, refers to cells which are a subject's own cells, or clones thereof.

The term "allogeneic cells", as used herein, refers to cells which are not a first subject's own cells, or clones thereof, but are cells, or clones thereof, derived from a second subject and this second subject is of the same species as the first subject.

The term "heterologous cells", as used herein, refers to cells which are not from a first subject's own cells, or clones thereof, but are cells, or clones thereof, derived from a second subject and this second subject is not the same species as the first subject.

The term "stem cells", as used herein, refers to cells capable of differentiation into other cell types, including those having a particular, specialized function (i.e., terminally differentiated cells, such as erythrocytes, macrophages, etc.). Stem cells can be defined according to their source (adult/somatic stem cells, embryonic stem cells), or according to their potency (totipotent, pluripotent, multipotent and unipotent).

The term "unipotent", as used herein, refers to cells can produce only one cell type, but have the property of self-renewal which distinguishes them from non-stem cells.

The term, "multipotent", or "progenitor", as used herein, refers to cells which can give rise to any one of several different terminally differentiated cell types. These different cell types are usually closely related (e.g. blood cells such as red blood cells, white blood cells and platelets). For example, mesenchymal stem cells (also known as marrow stromal cells) are multipotent cells, and are capable of forming osteoblasts, chondrocytes, myocytes, adipocytes, neuronal cells, and β-pancreatic islets cells. Another example is skeletal myoblasts, which preferentially give rise to skeletal muscle cells by a differentiation process involving fusion of individual cells into multinucleated myotubes.

The term "pluripotent", as used herein, refers to cells that give rise to some or many, but not all, of the cell types of an organism. Pluripotent stem cells are able to differentiate into any cell type in the body of a mature organism, although without reprogramming they are unable to de-differentiate into the cells from which they were derived. As will be appreciated, "multipotent"/progenitor cells (e.g., neural stem cells) have a more narrow differentiation potential than do pluripotent stem cells. Another class of cells even more primitive (i.e., uncommitted to a particular differentiation fate) than pluripotent stem cells are the so-called "totipotent" stem cells.

The term "totipotent", as used herein, refers to fertilized oocytes, as well as cells produced by the first few divisions of the fertilized egg cell (e.g., embryos at the two and four cell stages of development). Totipotent cells have the ability to differentiate into any type of cell of the particular species. For example, a single totipotent stem cell could give rise to a complete animal, as well as to any of the myriad of cell types found in the particular species (e.g., humans). In this specification, pluripotent and totipotent cells, as well as cells with the potential for differentiation into a complete organ or tissue, are referred as "primordial" stem cells.

The term "dedifferentiation", as used herein, refers to the return of a cell to a less specialized state. After dedifferentiation, such a cell will have the capacity to differentiate into more or different cell types than was possible prior to re-programming. The process of reverse differentiation (i.e., de-differentiation) is likely more complicated than differentiation and requires "re-programming" the cell to become more primitive. An example of dedifferentiation is the conversion of a myogenic progenitor cell, such as early primary myoblast, to a muscle stem cell or satellite cell.

A "normal" stem cell refers to a stem cell (or its progeny) that does not exhibit an aberrant phenotype or have an aberrant genotype, and thus can give rise to the full range of cells that be derived from such a stem cell. In the context of a totipotent stem cell, for example, the cell could give rise to, for example, an entire, normal animal that is healthy. In contrast, an "abnormal" stem cell refers to a stem cell that is not normal, due, for example, to one or more mutations or genetic modifications or pathogens. Thus, abnormal stem cells differ from normal stem cells.

A "growth environment" is an environment in which stem cells will proliferate in vitro. Features of the environment include the medium in which the cells are cultured, and a supporting structure (such as a substrate on a solid surface) if present.

"Growth factor" refers to a substance that is effective to promote the growth of cells and which, unless added to the culture medium as a supplement, is not otherwise a component of the basal medium. Put another way, a growth factor is a molecule that is not secreted by cells being cultured (including any feeder cells, if present) or, if secreted by cells in the culture medium, is not secreted in an amount sufficient to achieve the result obtained by adding the growth factor exogenously. Growth factors include, but are not limited to, basic fibroblast growth factor (bFGF), acidic fibroblast growth factor (aFGF), epidermal growth factor (EGF), insulin-like growth factor-I (IGF-I), insulin-like growth factor-II (TGF-II), platelet-derived growth factor-AB (PDGF), vascular endothelial cell growth factor (VEGF), activin-A, bone morphogenic proteins (BMPs), insulin, cytokines, chemokines, morphogens, neutralizing antibodies, other proteins, and small molecules.

The term "differentiation factor", as used herein, refers to a molecule that induces a stem cell or progenitor cell to commit to a particular specialized cell type.

"Extracellular matrix" or "matrix" refers to one or more substances that provide substantially the same conditions for supporting cell growth as provided by an extracellular matrix synthesized by feeder cells. The matrix may be provided on a substrate. Alternatively, the eomponent(s) comprising the matrix may be provided in solution. Components of an extracellular matrix can include laminin, collagen and fibronectin.

The term "regenerative capacity", as used herein, refers to the conversion of a stem cell into a dividing progenitor cell and a differentiated tissue-specific cell.

The term, "self renewal", as used herein, refers to proliferation without lineage specification.

The term, "aligned", as used herein, refers to the orientation of fibers in a fibrous polymer scaffold wherein at least 50% of the fibers are oriented in a general direction and their orientation forms an average axis of alignment. The orientation of any given fiber can deviate from the average axis of alignment and the deviation can be expressed as the angle formed between the alignment axis and orientation of the fiber: A deviation angle of 0° exhibits perfect alignment and 90° (or -90°) exhibits orthogonal alignment of the fiber with respect to the average axis of alignment. In an exemplary embodiment, the standard deviation of the fibers from the average axis of alignment can be an angle selected from between 0° and 1°, between 0° and 3°, between 0° and 5°, between 0° and 10°, between 0° and 15°, between 0° and 20°, or between 0° and 30°.

The term 'rod', as used herein, refers to a fibrous polymer scaffold which is essentially in the shape of a filled cylinder. Spaces and channels can be present between the individual fibers which compose the rod.

The term 'conduit', as used herein, refers to an object that is essentially cylindrical in shape. The conduit has an inner wall and an outer wall, an interior diameter, an exterior diameter, and an interior space which is defined by the inner diameter of the conduit as well as its length. Spaces and channels can be present between the individual fibers which compose the conduit.

The term 'filled conduit', as used herein, refers to a conduit in which a portion of the interior space is composed of filler material. This filler material can be a fibrous polymer scaffold. Spaces and channels can be present between the individual fibers which compose the filled conduit.

The term 'seam' or 'seamed', as used herein, refers to a junction formed by fitting, joining, or lapping together two sections. These two sections can be held together by mechanical means, such as sutures, or by chemical means, such as annealing or adhesives. For example, a seam is formed by joining one region of a sheet to another region.

The term 'seamless', as used herein, refers to an absence of a seam.

The term "cell" can refer to either a singular ("cell") or plural ("cells") situation.

The term "extracellular matrix component", as used herein, is a member selected from laminin, collagen, fibronectin and elastin.

The term "stent", as used herein, is a tube which can be made of, among other things, metal and organic polymers. When the stent is made of an organic polymer, the polymer is not a nanofibrous or microfibrous polymer scaffold as described herein. In other words, if the stent is made from a fibrous polymer scaffold, the average diameter of the fibers will be between 100 microns and about 50 centimeters. In some instances, the entire stent is capable of expanding from a first diameter to a second diameter, wherein the second diameter is greater than the first diameter.

### II. The Compositions

These compositions can comprise polymer scaffolds. These polymer scaffolds can be fibrous polymer scaffolds, such as microfiber polymer scaffolds or nanofiber polymer scaffolds. These polymer scaffolds can also be micropatterned polymer scaffolds. The compositions and/or polymer scaffolds of the invention can optionally be unaligned or they can be aligned, such as longitudinally or circumferentially. The compositions and/or polymer scaffolds of the invention can optionally be formed into a shape, such as a sheet, criss-cross sheet, conduit, rod or filled conduit. The compositions and/or polymer scaffolds of the invention can have a seam or they can be seamless. The compositions or polymers of the invention can also optionally include materials such as a cell, a biomolecule, or a pharmaceutically acceptable excipient. These alignments, shapes, and additional components can aid in the improvement or regeneration or replacement of biological function. The compositions of the invention do not include a stent. The compositions can be used in tissue engineering to improve, regenerate or replace biological functions.

### II. a) Fibrous Polymer Scaffolds

In a first aspect, the invention provides a composition which comprises a fibrous polymer scaffold. A fibrous polymer scaffold includes a fiber or fibers which can have a range of diameters. In an exemplary embodiment, the average diameter of the fibers in the fibrous polymer scaffold is from about 0.1 nanometers to about 50000 nanometers. In another exemplary embodiment, the average diameter of the fibers in the fibrous polymer scaffold is from about 25 nanometers to about 25,000 nanometers. In an exemplary embodiment, the average diameter of the fibers in the fibrous polymer scaffold is from about 50 nanometers to about 20,000 nanometers. In an exemplary embodiment, the average diameter of the fibers in the fibrous polymer scaffold is from about 100 nanometers to about 5,000 nanometers. In an exemplary embodiment, the average diameter of the fibers in the fibrous polymer scaffold is from about 1,000 nanometers to about 20,000 nanometers. In an exemplary embodiment, the average diameter of the fibers in the fibrous polymer scaffold is from about 10 nanometers to about 1,000 nanometers. In an exemplary embodiment, the average diameter of the fibers in the fibrous polymer scaffold is from about 2,000 nanometers to about 10,000 nanometers. In an exemplary embodiment, the average diameter of the fibers in the fibrous polymer scaffold is from about 0.5 nanometers to about 100 nanometers. In an exemplary embodiment, the average diameter of the fibers in the fibrous polymer scaffold is from about 0.5 nanometers to about 50 nanometers. In an exemplary embodiment, the average diameter of the fibers in the fibrous polymer scaffold is from about 1 nanometer to about 35 nanometers. In an exemplary embodiment, the average diameter of the fibers in the fibrous polymer scaffold is from about 2 nanometers to about 25 nanometers. In an exemplary embodiment, the average diameter of the fibers in the fibrous polymer scaffold is from about 90 nanometers to about 1,000 nanometers. In an exemplary embodiment, the average diameter of the fibers in the fibrous polymer scaffold is from about 500 nanometers to about 1,000 nanometers.

In an exemplary embodiment, the fibrous polymer scaffold is a member selected from a nanofiber polymer scaffold and a microfiber polymer scaffold. Microfiber polymer scaffolds have micron-scale features (an average fiber diameter between about 1,000 nanometers and about 50,000 nanometers, and especially between about 1,000 nanometers and about 20,000 nanometers), while nanofiber polymer scaffolds have submicron-scale features (an average fiber diameter between about 10 nanometers and about 1,000 nanometers, and especially between about 50 nanometers and about 1,000 nanometers). Each of these polymer scaffolds can resemble the physical structure at the area of treatment, such as native collagen fibrils or other extracellular matrices.

A variety of polymers from synthetic and/or natural sources can be used to compose these fibrous polymer scaffolds. A fiber can be made from one monomer or subunit. For example, lactic or polylactic acid or glycolic or polyglycolic acid can be utilized to form poly(lactide) (PLA) or poly(L-lactide) (PLLA) nanofibers or poly(glycolide) (PGA) nanofibers. Fibers can also be made from more than one monomer or subunit thus forming a co-polymer, terpolymer, etc. For example, lactic or polylactic acid and be combined with glycolic acid or polyglycolic acid to form the copolymer poly(lactide-co-glycolide) (PLGA). Other copolymers of use in the invention include poly(ethylene-co-vinyl) alcohol). In an exemplary embodiment, a fiber comprises a polymer or subunit which is a member selected from an aliphatic polyester, a polyalkylene oxide, polydimethylsiloxane, polyvinylalcohol, polylysine, collagen, laminin, fibronectin, elastin, alginate, fibrin, hyaluronic acid, proteoglycans, polypeptides and combinations thereof. In another exemplary embodiment, a fiber comprises two different polymers or subunits which are members selected from an aliphatic polyester, a polyalkylene oxide, polydimethylsiloxane, polyvinylalcohol, polylysine, collagen, laminin, fibronectin, elastin, alginate, fibrin, hyaluronic acid, proteoglycans, polypeptides and combinations thereof. In another exemplary embodiment, a fiber comprises three different polymers or subunits which are members selected from an aliphatic polyester, a polyalkylene oxide, polydimethylsiloxane, polyvinylalcohol, polylysine, collagen, laminin, fibronectin, elastin, alginate, fibrin, hyaluronic acid, proteoglycans, polypeptides and combinations thereof. In an exemplary embodiment, the aliphatic polyester is linear or branched. In another exemplary embodiment, the linear aliphatic polyester is a member selected from lactic acid (D- or L-), lactide, poly(lactic acid), poly(lactide) glycolic acid, poly(glycolic acid), poly(glycolide), glycolide, poly(lactide-co-glycolide), poly(lactic acid-co-glycolic acid), polycaprolactone and combinations thereof. In another exemplary embodiment, the aliphatic polyester is branched and comprises at least one member selected from lactic acid (D- or L-), lactide, poly(lactic acid), poly(lactide) glycolic acid, poly(glycolic acid), poly(glycolide), glycolide, poly(lactide-co-glycolide), poly(lactic acid-co-glycolic acid), polycaprolactone and combinations thereof which is conjugated to a linker or a biomolecule. In an exemplary embodiment, wherein said polyalkylene oxide is a member selected from polyethylene oxide, polyethylene glycol, polypropylene oxide, polypropylene glycol and combinations thereof.

In some embodiments, the fibrous polymer scaffold is composed of a single continuous fiber. In other embodiments, the fibrous polymer scaffold is composed of at least two, three, four, or five fibers. In an exemplary embodiment, the number of fibers in the fibrous polymer scaffolds is a member selected from 2 to 100,000. In an exemplary embodiment, the number of fibers in the fibrous polymer scaffolds is a member selected from 2 to 50,000. In an exemplary embodiment, the number of fibers in the fibrous polymer scaffolds is a member selected from 50,000 to 100,000. In an exemplary embodiment, the number of fibers in the fibrous polymer scaffolds is a member selected from 10 to 20,000. In an exemplary embodiment, the number of fibers in the fibrous polymer scaffolds is a member selected from 15 to 1,000.

The fibrous polymer scaffold can comprise a fiber of at least one composition. In an exemplary embodiment, the fibrous polymer scaffold comprises a number of different types of fibers, and this number is a member selected from one, two, three, four, five, six, seven, eight, nine and ten.

In another exemplary embodiment, the fiber or fibers of the fibrous polymer scaffold are biodegradable. In another exemplary embodiment, the fibers of the fibrous polymer scaffold comprise biodegradable polymers. In another exemplary embodiment, the biodegradable polymers comprise a monomer which is a member selected from lactic acid and glycolic acid. In another exemplary embodiment, the biodegradable polymers are poly(lactic acid), poly(glycolic acid) or a copolymer thereof Preferred biodegradable polymers are those which are approved by the FDA for clinical use, such as poly(lactic acid) and poly(glycolic acid). In another exemplary embodiment, biodegradable polymer scaffolds of the invention can be used to guide the morphogenesis of engineered tissue and gradually degrade after the assembly of the tissue. The degradation rate of the polymers can be tailored by one of skill in the art to match the tissue generation rate. For example, if a polymer that biodegrades quickly is desired, an approximately 50:50 PLGA combination can be selected. Additional ways to increase polymer scaffold biodegradability can involve selecting a more hydrophilic copolymer (for example, polyethylene glycol), decreasing the molecular weight of the polymer, as higher molecular weight often means a slower degradation rate, and changing the porosity or fiber density, as higher porosity and lower fiber density often lead to more water absorption and faster degradation. In another exemplary embodiment, the tissue is a member selected from muscle tissue, vascular tissue, nerve tissue, spinal cord tissue and skin tissue. In another exemplary embodiment, the biodegradable fibrous scaffolds can be used to guide the morphogenesis of engineered muscular tissue and gradually degrade after the assembly of myoblasts, myotubes, and skeletal muscle tissue.

### Methods of making a fibrous polymer scaffold

The polymer scaffolds ofthe invention can be produced in a variety of ways. In an exemplary embodiment, the polymer scaffold can be produced by electro spinning. Electrospinning is an atomization process of a conducting fluid which exploits the interactions between an electrostatic field and the conducting fluid. When an external electrostatic field is applied to a conducting fluid (e.g., a semi-dilute polymer solution or a polymer melt), a suspended conical droplet is formed, whereby the surface tension ofthe droplet is in equilibrium with the electric field. Electrostatic atomization occurs when the electrostatic field is strong enough to overcome the surface tension of the liquid. The liquid droplet then becomes unstable and a tiny jet is ejected from the surface of the droplet. As it reaches a grounded target, the material can be collected as an interconnected web containing relatively fine, i.e. small diameter, fibers. The resulting films (or membranes) from these small diameter fibers have very large surface area to volume ratios and small pore sizes. A detailed description of electrospinning apparatus is provided in Zong, *et al., Polymer,* 43(16):4403-4412 (2002); Rosen *et al., Ann Plast Surg.,* **25**:375-87 (1990) Kim, K., *Biomaterials* 2003, 24, (27), 4977-85; Zong, X., *Biomaterials* 2005, 26, (26), 5330-8. After electrospinninng, extrusion and molding can be utilized to further fashion the polymers. To modulate fiber organization into aligned fibrous polymer scaffolds, the use of patterned electrodes, wire drum collectors, or post-processing methods such as uniaxial stretching has been successful. Zong, X., *Biomaterials* 2005, 26, (26), 5330-8; Katta, P., *Nano Lett* 2004, 4, (11), 2215-2218; Li, D., *Nano Lett* 2005, 5, (5), 913-6.

The polymer solution can be produced in one of several ways. One method involves polymerizing the monomers and dissolving the subsequent polymer in appropriate solvents. This process can be accomplished in a syringe assembly or it can be subsequently loaded into a syringe assembly. Another method involves purchasing commercially available polymer solutions or commercially available polymers and dissolving them to create polymer solutions. For example, PLLA can be purchased from DuPont (Wilmington, DE), poly(lactide-co-glycolide) can be purchased from Ethicon (Somerville, NJ) and Birmingham Polymers (Birmingham, AL), Sigma-Aldrich (St. Louis, MO) and Polysciences (Warrington, PA). Other manufacturers include Lactel Absorbable Polymers (Pelham, AL). Additional polymer scaffold components of the invention, such as cells and biomolecules, are also commercially available from suppliers such as Invitrogen (San Diego, CA), Cambrcx (Walkcrsvillc, MD), Sigma-Aldrich, Peprotech (Rocky Hill, NJ), R&D Systems (Minneapolis, MN), ATCC (Manassas, VA), Pierce Biotechnology (Rockford, IL).

The polymer used to form the polymer scaffold is first dissolved in a solvent. The solvent can be any solvent which is capable of dissolving the polymer monomers and/or subunits and providing a polymer solution capable of conducting and being electrospun. Typical solvents include a solvent selected from N,N-Dimethyl formamide (DMF), tetrahydrofuran (THF), methylene chloride, dioxane, ethanol, hexafluoroisopropanol (HFIP), chloroform, water and combinations thereof.

The polymer solution can optionally contain a salt which creates an excess charge effect to facilitate the electrospinning process. Examples of suitable salts include NaCl, KH₂PO₄, K₂HPO₄, KIO₃, KCl, MgSO₄, MgCl₂, NaHCO₃, CaCl₂ or mixtures of these salts.

The polymer solution forming the conducting fluid will preferably have a polymer concentration in the range of about 1 to about 80 wt %, more preferably about 8 to about 60 wt %. The conducting fluid will preferably have a viscosity in the range of about 50 to about 2000 mPa X s, more preferably about 200 to about 700 mPa X s.

The electric field created in the electrospinning process will preferably be in the range of about 5 to about 100 kilovolts (kV), more preferably about 10 to about 50 kV. The feed rate of the conducting fluid to the spinneret (or electrode) will preferably be in the range of about 0.1 to about 1000 microliters/min, more preferably about 1 to about 250 microliters/min.

The single or multiple spinnerets sit on a platform which is capable of being adjusted, varying the distance between the platform and the grounded collector substrate. The distance can be any distance which allows the solvent to essentially completely evaporate prior to the contact of the polymer with the grounded collector substrate. In an exemplary embodiment, this distance can vary from 1 cm to 25 cm. Increasing the distance between the grounded collector substrate and the platform generally produces thinner fibers.

In electrospinning cases where a rotating mandrel is required, the mandrel is mechanically attached to a motor, often through a drill chuck. In an exemplary embodiment, the motor rotates the mandrel at a speed of between about 1 revolution per minute (rpm) to about 500 rpm. In an exemplary embodiment, the motor rotation speed of between about 200 rpm to about 500 rpm. In another exemplary embodiment, the motor rotation speed of between about 1 rpm to about 100 rpm.

Additional embodiments or modifications to the electrospinning process and apparatus are described herein.

### Variation of Electric/mechanical Properties of Conducting Fluid

The properties of the resulting membrane produced by electrospinning will be affected by the electric and mechanical properties of the conducting fluid. The conductivity of the polymer solution can be drastically changed by adding ionic inorganic/organic compounds. The magneto-hydrodynamic properties of the polymer solution can depend on a combination of physical and mechanical properties, (e.g., surface tension, viscosity and viscoelastic behavior of the fluid) and electrical properties (e.g., charge density and polarizability of the fluid). For example, by adding a surfactant to the polymer solution, the fluid surface tension can be reduced, so that the electrostatic fields can influence the jet shape and the jet flow over a wider range of conditions. By coupling a syringe pump that can control the flow rate either at constant pressure or at constant flow rate, the effect of viscosity of the conducting fluid can be alleviated.

### Electrode Design

In another embodiment for producing membranes according to the present invention, the jet formation process during electrospinning is further refined to provide better control over fiber size. Instead of merely providing a charged spinneret and a ground plate, as discussed above, a positively charged spinneret is still responsible for the formation of the polymer solution droplet and a plate electrode with a small exit hole in the center is responsible for the formation of the jet stream. This exit hole will provide the means to let the jet stream pass through the plate electrode. Thus, if the polymer droplet on the positively charged spinneret has a typical dimension of 2-3 mm and the plate electrode is placed at a distance of about 10 mm from the spinneret, a reasonable electrostatic potential can be developed. The short distance between the two electrodes implies that the electrostatic potential could be fairly low. However, the resultant electric field strength could be sufficiently strong for the electrospinning process. By varying the electric potential of the spinneret, the jet formation can be controlled and adjusted. Such an electrode configuration should greatly reduce the required applied potential on the spinneret from typically about 15 kilovolts (kV) down to typically about 1.5 to 2 kV (relative to the ground plate potential). The exact spinneret potential required for stable jet formation will depend on the electric/mechanical properties of the specific conducting fluid.

### Control of Jet Acceleration and Transportation

In another preferred embodiment for producing polymer scaffolds of the present invention, the jet stream flight is also precisely controlled. The jet stream passing through the plate electrode exit hole is positively charged. Although this stream has a tendency to straighten itself during flight, without external electric field confinement the jet will soon become unstable in its trajectory. In other words, the charged stream becomes defocused, resulting in loss of control over the microscopic and macroscopic properties of the fluid. This instability can be removed by using a carefully designed probe electrode immediately after the plate electrode and a series of (equally) spaced plate electrodes. The electrode assembly (or composite electrode), i.e., the probe electrode and the plate electrodes, can create a uniform distribution of electrostatic potential along the (straight) flight path. The acceleration potential is formed by placing the base potential of the spinneret at about +20 to +30 kV above the target (at ground potential) while the electrostatic potential of the probe electrode can be adjusted to slightly below the plate electrode base potential. The composite electrodes are capable of delivering the jet stream to a desired target area. The composite electrode can also be utilized to manipulate the jet stream. By changing the electrostatic potential, the jet stream acceleration is altered, resulting in varying the diameter of the formed polymer fiber. This electrostatic potential variation changes the jet stream stability, and therefore, corresponding changes in the composite electrode can be used to stabilize the new jet stream. Such a procedure can be used to fine-tune and to change the fiber diameter during the electrospinning process.

### Jet Manipulation

In yet another embodiment, the jet stream can be focused by using an "Alternating Gradient" (AG) technique, widely used in the accelerator technology of high-energy physics. The basic idea is to use two pairs of electrostatic quadrupole lenses. The second lens has the same geometric arrangement as the first lens with a reversed (alternate) electric gradient. The positively charged jet stream will be focused, for example, in the xz plane after the first lens and then be refocused in the yz plane after the second lens. It is noted that the z-direction represents the direction of the initial flight path. By applying an additional triangle-shaped waveform to the potential on one of the pairs of the quadrupole, the jet can be swept across the target area, allowing the control of the direction of the jet stream. Furthermore, with varying waveform of the 'sweep' potential, a desired pattern on the target can be formed.

### Electrospinning apparatus with unique mandrels

Electrospun polymer fibers can be deposited on a stationary or rotating substrate. In the past, a stationary metal collector has been used for the random deposition of electrospun fibers. A rotating metal mandrel has been used during electrospinning. A rotating metal mandrel causes random fiber deposition on the surface of the mandrel, which can produce a conduit when the mandrel is removed. Conduits with circumferential fiber alignment may also be produced by modifying this method and rotating the mandrel at a high speed (>100 rpm). If rotating drums with large diameters and/or lengths are used as collector substrates, sheets of unaligned and aligned fibrous polymer scaffolds may be produced upon cutting and removing the deposited fibrous polymer scaffolds from the drum. It has previously been shown that producing an air gap (hole) within a stationary metal collector induces alignment of the electrospun fibers deposited across the gap (Li, D., Wang Y. L., Xia, Y. N., *Electrospinning of Polymeric and Ceramic Nanofibers as Uniaxially Aligned Arrays.* Nano Letters, 2003. **3**(8): p. 1167-71). However, there is no description in Li, or any-where else, of fabricating three-dimensional conduits or rods with alignment or of directly electrospinning conduits or rods composed of longitudinally aligned fibers.

In another aspect, the invention includes a method to electrospin aligned fibrous polymer scaffolds on a rotating mandrel. These fibrous polymer scaffolds can be aligned in any orientation desired by the user. In an exemplary embodiment, the scaffolds are aligned in an essentially longitudinal or essentially circumferential direction. The fibrous polymer scaffolds created by this method can either have a seam or they can be seamless. In an exemplary embodiment, the fibrous polymer scaffolds are seamless. In another exemplary embodiment, the fibrous polymer scaffolds are seamless along an axis essentially parallel to the longitudinal axis of the polymer scaffolds.

In another aspect, the invention includes unique mandrels that allow for the electrospinning of seamless conduits, seamless filled conduits and seamless rods. In another exemplary embodiment, the seamless conduits, seamless filled conduits and seamless rods have an unaligned fiber orientation. In another exemplary embodiment, the fibers of the seamless conduits, seamless filled conduits and seamless rods are aligned. In another exemplary embodiment, the seamless conduits, seamless filled conduits and seamless rods have essentially longitudinally aligned fibers.

In another aspect, the invention includes unique mandrels that allow for the electrospinning of monolithically formed conduits, filled conduits and rods. In another exemplary embodiment, the monolithically formed conduits, filled conduits and rods have an unaligned fiber orientation. In another exemplary embodiment, the fibers of the monolithically formed conduits, filled conduits and rods are aligned. In another exemplary embodiment, the monolithically formed conduits, filled conduits and rods have essentially longitudinally aligned fibers.

In an exemplary embodiment, the mandrel is attached to a motor assembly that is capable of rotating the mandrel about its longitudinal axis. In the electrospinning apparatus, the rotating mandrel is grounded and placed below a spinneret. A polymer solution is delivered to the tip of the spinneret and is charged by a power supply. The electrical field created between the spinneret and the mandrel induces the charged polymer solution at the tip of the spinneret to form a jet. The jet sprays toward the mandrel. The polymer contacts one conducting region of the mandrel and then contacts a second conducting region of the mandrel, depositing the fiber across a non-conducting region or air gap ofthe mandrel. This results in the formation of aligned fibers deposited on the non-conducting region or in the air gap. By rotating the mandrel, the result is an evenly applied layer of aligned fibers. The deposited fibrous layers conform to the shape of the mandrel or the air gap between the mandrels, thus forming a sheet in some instances, a conduit in some instances or a rod in other instances. The fibers comprising the sheet, conduit or rod will be aligned along the length of the conduit or rod, thus forming a sheet, conduit or rod with longitudinally aligned fibers. In an exemplary embodiment, the conduit or rod will be seamless. In an exemplary embodiment, the conduit or rod will be seamless along an axis that is essentially parallel to the long axis of the conduit or rod.

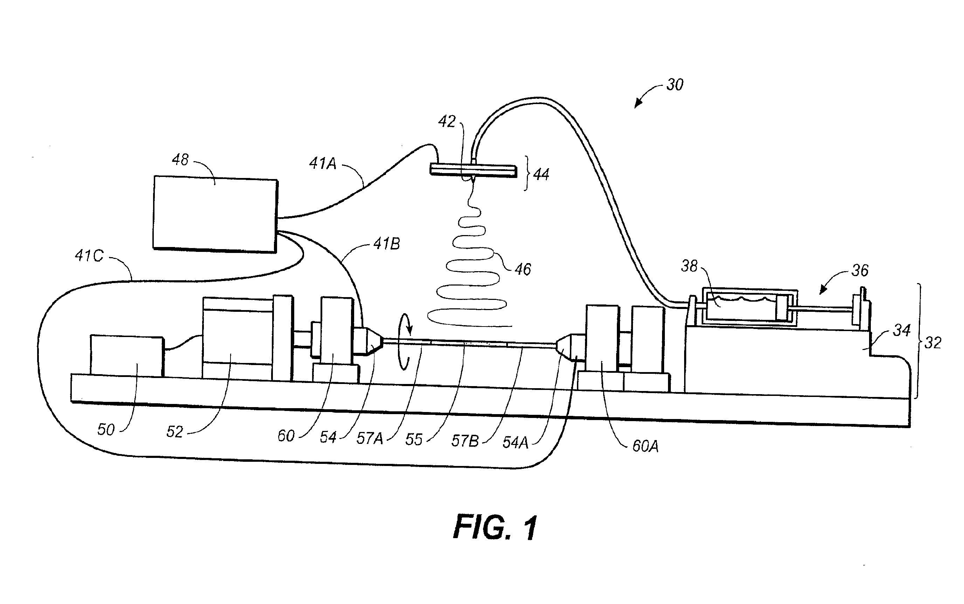

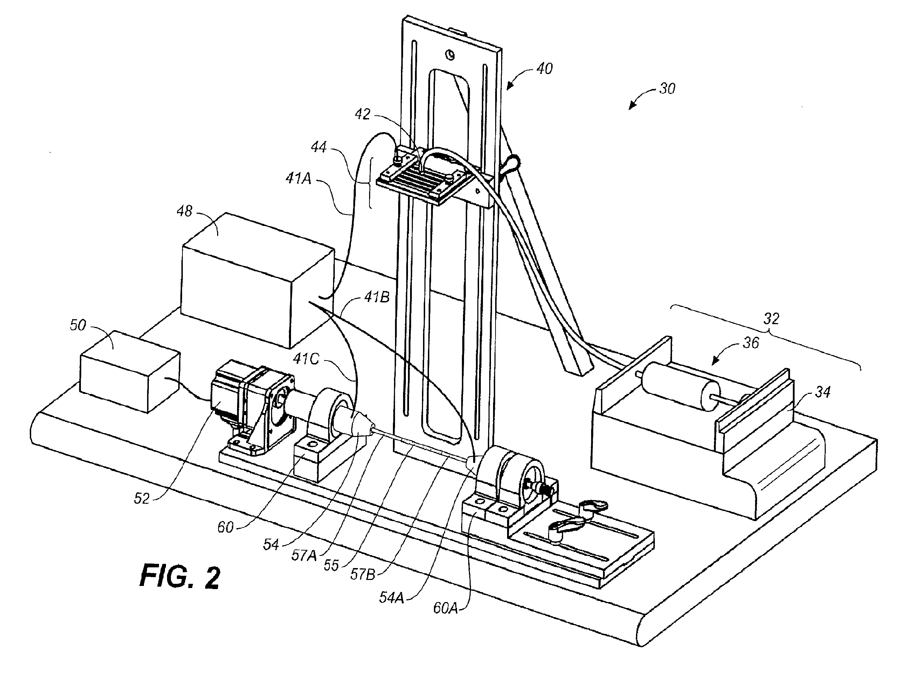

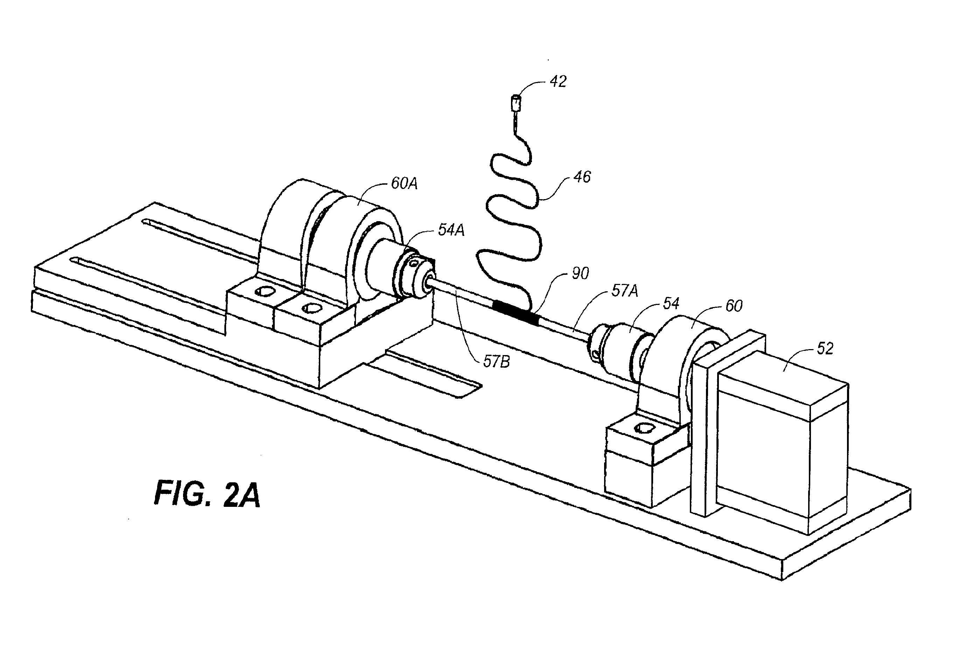

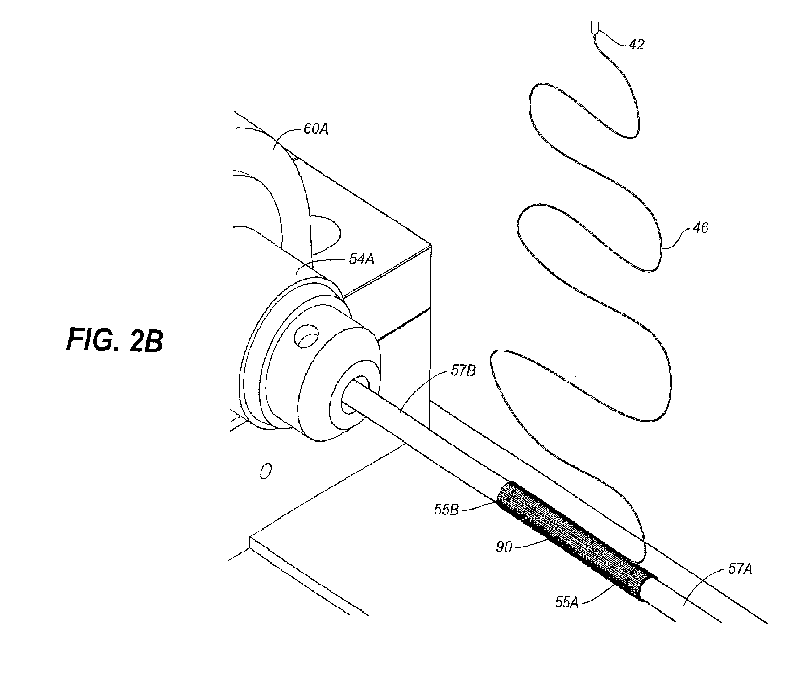

In one embodiment, the invention provides a mandrel with at least two conducting regions and at least one non-conducting region. Such a mandrel can be designed in a number of ways; an exemplary depiction of the mandrel is provided in **FIG. 3B** and an exemplary depiction of the mandrel as part of an apparatus for producing sheets and/or conduits of the invention is described in **FIGS. 1****,** **2****,** **2A****,** and **2B****.** In an exemplary embodiment, the electrically conducting material is a metal. In another exemplary embodiment, the metal is a member selected from steel and aluminum. In one instance, a region of a conducting mandrel can be covered with a non-electrically conducting material. An exemplary cross section of this mandrel is provided in **FIG. 3D****.** In an exemplary embodiment, the non-electrically conducting material is a member selected from tape, electrical tape, teflon and plastic. In another instance, a mandrel can be produced that has at least three sections, a non-electrically conducting region which interconnects two conducting mandrel regions. In another instance, a non-electrically conducting region is a discrete portion extending between two conducting mandrel regions. An exemplary cross section of this mandrel is provided in **FIG. 3C****.** Additional non-conducting regions can be added to the mandrel by either placing a non-conducting region over a conducting region of the mandrel, or by interconnecting a non-conducting region between two conducting regions of the mandrel. These additional non-conducting regions, ifused in conjunction with additional spinnerets, can facilitate the production of more than one conduit on the same mandrel at the same time. In one embodiment, the invention provides a mandrel with at least three conducting regions and at least two non-conducting regions. In one embodiment, the invention provides a mandrel with at least four conducting regions and at least three non-conducting regions. In one embodiment, the invention provides a mandrel with at least five conducting regions and at least four non-conducting regions.

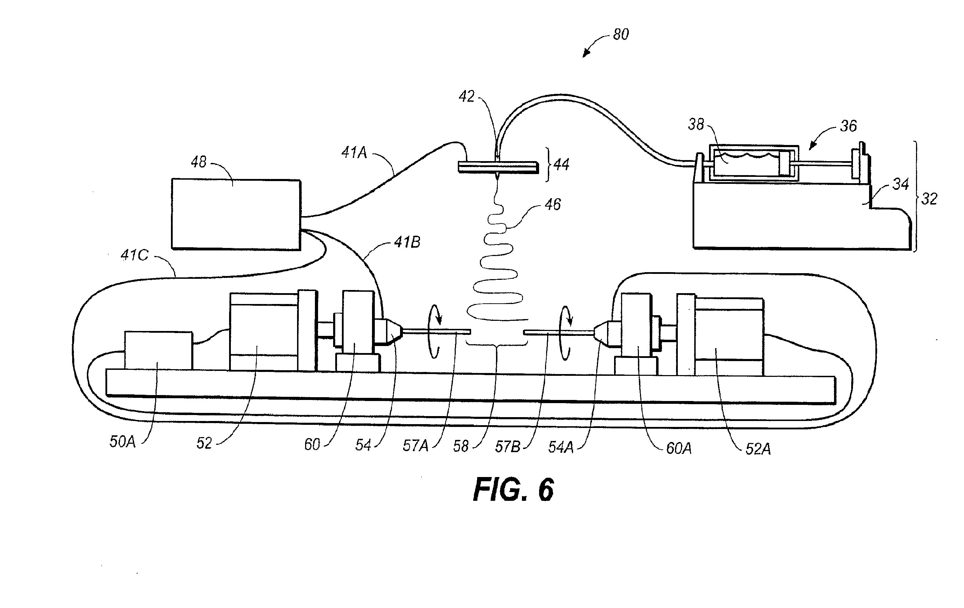

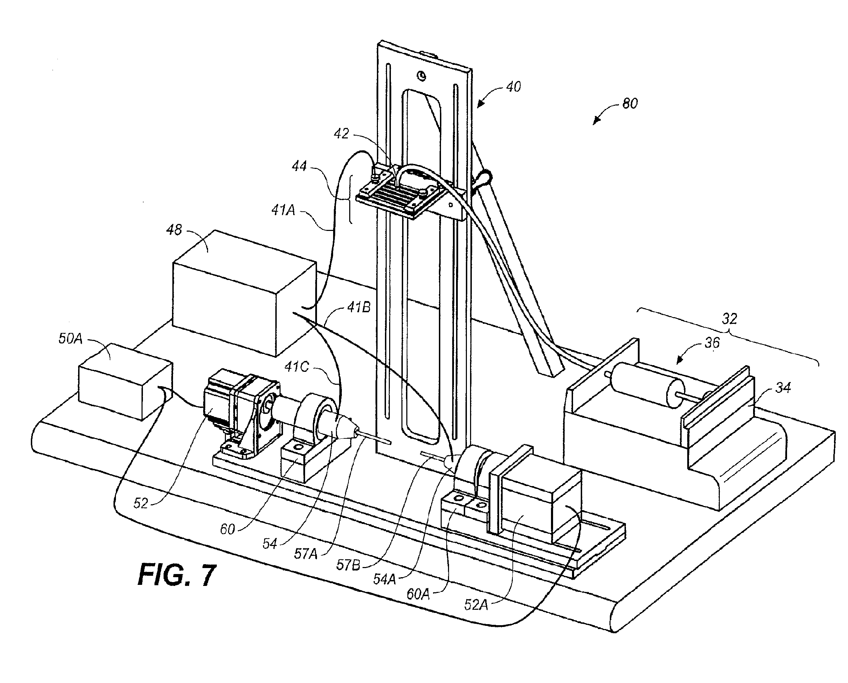

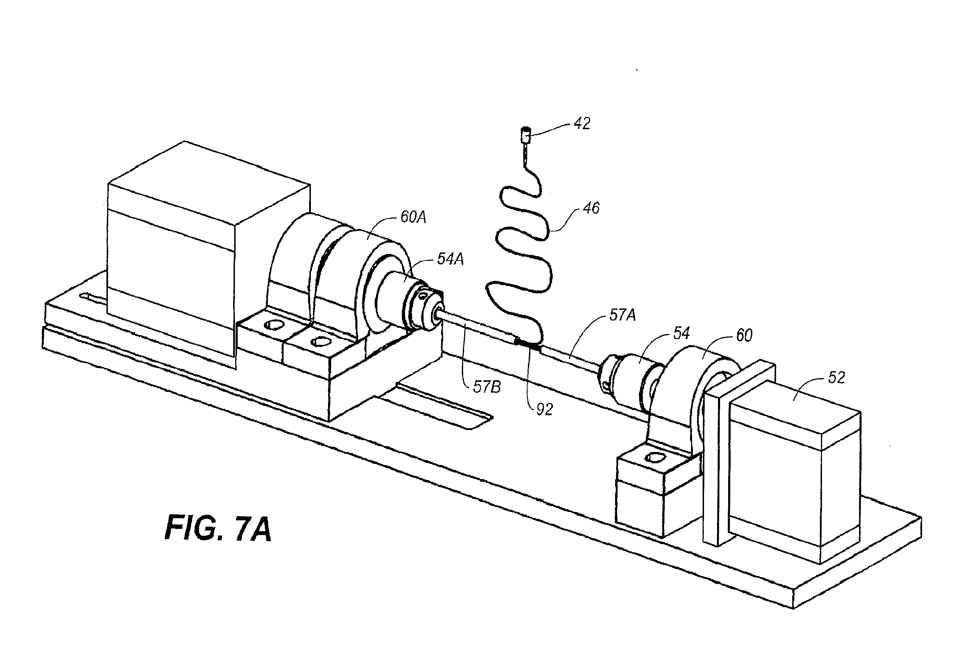

In one embodiment, the invention provides a mandrel with a first conducting region, a second conducting region, and an air gap between the first conducting region and the second conducting region. Such a mandrel can be designed in a number of ways; an exemplary depiction of the mandrel is provided in **FIG. 3E** and an exemplary depiction of the mandrel as part of an apparatus for producing rods of the invention is described in FIGS. 6, 7, and **7A****.** An embodiment with multiple spinnerets is provided is described in **FIG. 11****.** In an exemplary embodiment, the electrically conducting material is a metal. In another exemplary embodiment, the metal is a member selected from steel and aluminum. In an exemplary embodiment, each conducting region of the mandrel is aligned with the other. In an exemplary embodiment, each conducting region of the mandrel is attached to assemblies that are capable of rotating at the same speed. This can be accomplished by attaching motor assemblies to each conducting region of the mandrel and ensuring that each motor runs at the same speed. This can also be accomplished by ensuring that each conducting region of the mandrel is connected to the same motor.

After electrospinning is complete, the polymer scaffolds of the invention are removed from the mandrel. For sheet polymer scaffolds, the sheet can be peeled away from the mandrel. For conduit polymer scaffolds, the mandrel can be taken out ofthe motor assembly and the conduit can then be removed. In some embodiments, removal can also be accomplished by disconnecting the mandrel in the middle or also by cutting the conduit. For rod polymer scaffolds, the rod can be peeled away from the metal ends of the conducting regions. In some instances, the lengths of the deposited fibers will not be equal, resulting in jagged edges at the end or ends of the polymer scaffold. Optionally, the ends of the polymer scaffolds can be cut in order to create polymer scaffolds with lengths of essentially the same size. This cutting can occur when the polymer scaffold is on the mandrel, or after it has been removed from the mandrel.

The characteristics of the polymer scaffolds described herein can be changed by altering various parameters. For example, there are several methods which either alone or in combination can decrease the average diameter of the fibers in the fibrous polymer scaffold. One method is to add more salt to the polymer solution. Using a more polar solvent in the polymer solution also tends to decrease the average fiber diameter, as does increasing the distance between the spinneret and the mandrel. Additional methods of decreasing the scaffold diameter include increasing the apparatus voltage and increasing the polymer concentration.

Multi-layered polymer scaffolds can be formed by the methods described herein by completing several mandrel rotations. For example, a multilayered conduit can be formed by completing several mandrel rotations. Additional polymer scaffolds with more than one type of layer can also be produced. In an exemplary embodiment, the circumferential alignment of the fibers can also be adjusted or varied by altering the speed by which the mandrel rotates. Thus a polymer scaffold with an inner longitudinally aligned layer and an outer circumferentially aligned layer can be produced. In order to fabricate a multi-layered hollow conduit scaffold with each layer having specific alignment, various mandrels and rotation speeds may be used. In an exemplary embodiment, a hollow conduit shaped fibrous scaffold is produced with a luminal layer composed of longitudinally aligned fibers and an outer layer with circumferentially aligned fibers. One method to produce such a scaffold involves using a mandrel with a non-conducting region as described previously. The mandrel is rotated at a slow speed allowing for the formation of a conduit shaped fibrous scaffold composed of longitudinally aligned fibers. The rotation speed of the mandrel is then increased, which causes the electrospun fibers to align in a circumferential direction around the longitudinally aligned fibrous conduit. In another exemplary embodiment, the inner layer is aligned longitudinally while the outer layer is composed of randomly aligned fibers. This can be achieved using the same setup as described previously except when forming the outer layer, the mandrel is rotated at an intermediate speed that prevents both longitudinal and circumferential alignment of fibers.

### II. b) Micropatterned Polymer Scaffolds

In a second aspect, the invention provides a composition which comprises a micropattemed polymer scaffold. With micropatterning, soft lithography is used to topographically or chemically alter the spatial and geometric organization of the polymer and create micron-scale features on substrate surfaces. Taylor, A.M., *Nat Methods* 2005, 2, (8), 599-605; Dow, J.A., *J Cell Sci Suppl* 1987, 8, 55-79; Kane, R.S., *Biomaterials* 1999, 20, (23-24), 2363-76. The polymer scaffolds created by this technique can be used to control many aspects of cellular behavior, including cell size, shape, spatial organization, proliferation and survival. Chen, C.S., *Science* 1997, 276, (5317), 1428-8; Bhatia, S.N., *Faseb J* 1999, 13, (14), 1883-900; Deutsch, J., *J Biomed Mater Res* 2000, 53, (3), 267-76; Folch, A., *Annu Rev Biomed Eng* 2000, 2, 227-56; Whitesides, G.M., *Annu Rev Biomed Eng* 2001, 3, 335-73. Poly(dimethylsiloxane) (PDMS) is an elastomer that can be micropattemed with high reproducibility and provides a flexible substrate for cell attachment. Wang, N., *Cell Motil Cytoskeleton* 2002, 52, (2), 97-106.

### II. c) Alignment of the Polymer Scaffolds

The polymer scaffolds of the invention can have an aligned orientation or a random orientation. In an aligned orientation, at least 50% of the fibers comprising the polymer scaffold are oriented along an average axis of alignment.

In an exemplary embodiment, the composition has an alignment which is a member selected from essentially longitudinal, essentially circumferential, and 'criss-cross'. A longitudinal alignment is present when the fibers are aligned in the direction of the long axis of the conduit, filled conduit or rod shaped polymer scaffolds. A circumferential alignment is present when the fibers are aligned along the short axis of the polymer scaffold. A criss-cross alignment is present when the fibers of one polymer scaffold in the composition are aligned in such a manner that the average alignment axis of a first polymer scaffold is at an angle relative to the average alignment axis of a second polymer scaffold which is adjacent to the first polymer scaffold. A longitudinally aligned or circumferentially aligned polymer scaffold can have more than one layer of fibers. A criss-cross aligned polymer scaffold requires more than one layer of fibers.

In another exemplary embodiment, the polymer fibers can have a standard deviation from the central axis of the fiber bundle. In an exemplary embodiment, the standard deviation of the fiber is a member selected from between about 0° and about 1°, between about 0° and about 3°, between about 0° and about 5°, between about 0° and about 10°, between about 0° and about 15°, between about 0° and about 20°, and between about 0° and about 30°.

Aligned polymer scaffolds have profound effects on cell cytoskeletal alignment, cell migration and cellular function. Aligned polymer scaffolds can induce and direct cell migration thus enhancing tissue regeneration. Such scaffolds are a promising solution for a variety of tissue regeneration, such as muscle, skin, vascular tissue, nerve and spinal cord regeneration. For example, the longitudinally aligned fibrous polymer scaffolds can enhance and specifically direct nerve, skin, muscle and/or vascular tissue growth across an injury gap.

The direction in which the aligned polymer scaffold is situated may affect the biological function that the aligned polymer scaffold is replacing or improving. For instance, when an aligned polymer scaffold is situated in a wound, wound healing is more rapid when the aligned polymer scaffold is perpendicular, rather than parallel, to the long axis of the wound. In an exemplary embodiment, the central long axis of the bundle of an aligned polymer scaffold is situated perpendicular to the direction of the material which the aligned polymer scaffold is improving or replacing. In another exemplary embodiment, the central long axis of the bundle of an aligned polymer scaffold is situated parallel to the direction of the material which the aligned polymer scaffold is improving or replacing.

In another exemplary embodiment, the aligned compositions of the invention (such as polymer scaffolds) can comprise biodegradable polymers. These compositions can be used to guide the morphogenesis of other types of tissues with anisotropic structure, e.g., nerve, skin, blood vessel, skeletal muscle, cardiac muscle, tendon and ligament. These aligned, biodegradable compositions of the invention can also be used in the development of three-dimensional tissues. Using electrospun biodegradable fibrous polymer scaffolds, three-dimensional constructs of nerve tissue, spinal cord tissue, skin tissue, vascular tissue and muscle tissue can be created.

In an exemplary embodiment, the compositions described herein can comprise more than one polymer scaffold. Each ofthose polymer scaffolds can have an alignment which is the same or different from the other polymer scaffold or scaffolds in the composition.

In an exemplary embodiment, the composition comprises two polymer scaffolds. The first polymer scaffold has the shape of a conduit and is longitudinally aligned. The second polymer scaffold surrounds the exterior of the first polymer scaffold and has an orientation which is a member selected from random, circumferential, criss-cross, and longitudinal. In an exemplary embodiment, the orientation of the second polymer scaffold is a member selected from random and circumferential.

### II. d) Shapes of the Polymer Scaffolds/Methods of Making the Polymer-Scaffolds

The polymer scaffolds of the invention can be formed into a variety of shapes, depending on the nature of the problem to be solved.

The compositions and/or polymer scaffolds of the invention can have a variety of dimensions. In an exemplary embodiment, the polymer scaffold is 0.1 mm to 50 cm long. In another exemplary embodiment, the polymer scaffold is 0.1 mm to 1 mm long. In another exemplary embodiment, the polymer scaffold is 1mm to 1 cm long. In another exemplary embodiment, the polymer scaffold is 1 cm to 10 cm long. In another exemplary embodiment, the polymer scaffold is 10 cm to 50 cm long. In another exemplary embodiment, the polymer scaffold is 1 cm to 5 cm long. In another exemplary embodiment, the polymer scaffold is 2.5 cm to 15 cm long. In another exemplary embodiment, the polymer scaffold is 5mm to 6 cm long. In another exemplary embodiment, the polymer scaffold is 8mm to 3 cm long. In another exemplary embodiment, the polymer scaffold is 10 cm to 25 cm long. In another exemplary embodiment, the polymer scaffold is 0.5 cm to 2 cm long. In another exemplary embodiment, the polymer scaffold is 0.1 cm to 2 cm long.

The compositions and/or polymer scaffolds of the invention can be composed of a variety of fibrous layers. In an exemplary embodiment, the composition has between about 1 and about 2,000 fibrous layers. In an exemplary embodiment, the composition has between about 1 and about 1,000 fibrous layers. In an exemplary embodiment, the composition has between about 1 and about 500 fibrous layers. In an exemplary embodiment, the composition has between about 1 and about 20 fibrous layers. In an exemplary embodiment, the composition has between about 1 and about 10 fibrous layers. In an exemplary embodiment, the composition has between about 5 and about 25 fibrous layers. In an exemplary embodiment, the composition has between about 500 and about 1,500 fibrous layers. In an exemplary embodiment, the composition has between about 10 and about 20 fibrous layers. In an exemplary embodiment, the composition has between about 35 and about 80 fibrous layers. In an exemplary embodiment, the composition has between about 10 and about 100 fibrous layers. In an exemplary embodiment, the composition has between about 5 and about 600 fibrous layers. In an exemplary embodiment, the composition has between about 10 and about 80 fibrous layers. In an exemplary embodiment, the composition has between about 2 and about 12 fibrous layers. In an exemplary embodiment, the composition has between about 60 and about 400 fibrous layers. In an exemplary embodiment, the composition has between about 1,200 and about 1,750 fibrous layers.

In an exemplary embodiment, the polymer scaffold has the shape of a sheet or membrane. Polymer scaffold membranes can be made through electrospinning. The individual fibers within the membrane can be aligned either during electrospinning using a rotating drum as a collector or after by mechanical uniaxial stretching.

In another exemplary embodiment, the polymer scaffold has the shape of a 'criss-cross' sheet. To form a criss-cross sheet, layers of aligned polymer sheets or membranes can be arranged in relation to each other, at an angle which is a member selected from greater than 20 degrees but less than 160 degrees, greater than 30 degrees but less than 150 degrees, greater than 40 degrees but less than 140 degrees, greater than 50 degrees but less than 130 degrees, greater than 60 degrees but less than 120 degrees, greater than 70 degrees but less than 110 degrees, and greater than 80 degrees but less than 100 degrees.

There are a variety of ways to make a 'criss-cross' sheet. In one exemplary embodiment, a rotating metal drum collector is used that does not contain a non-conducting region. An aligned layer of fibers is created on the drum, which is then peeled off the drum. The aligned layer is rotated 90 degrees and then placed back on the drum. Next an additional layer of electrospun fibers is added while the drum rotates at a high speed. Additional criss-cross layers can be added by repeating these steps. In another exemplary embodiment, a drum is used that has a non-conducting region. Here, the drum is rotated slowly for a first period of time so the fibers deposit and align longitudinally on the non-conducting section. Then the drum is spun fast so the fibers are forced to align circumferentially. Additional criss-cross layers can be added by repeating these steps.

### Conduit

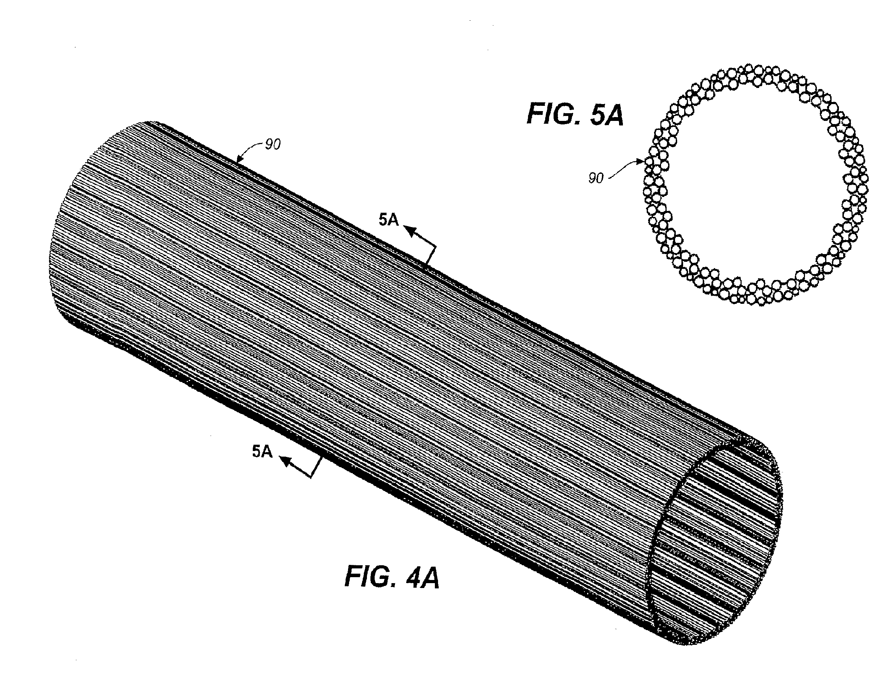

In another exemplary embodiment, the polymer scaffold has the shape of a conduit. An exemplary depiction of a conduit is provided in **FIG. 4A** and an exemplary depiction of a cross-sectional view of the conduit is provided in **FIG. 5A****.** A conduit can have a variety of sizes, depending on its length, as well as its inner diameter and outer diameters. In an exemplary embodiment, the interior space of the conduit is essentially free of a fibrous polymer scaffold. These parameters can be varied to accommodate, for example, various tissue sizes and applications. In an exemplary embodiment, the conduit wall is comprised of aligned fibers. In another exemplary embodiment, the fibers are longitudinally aligned or circumferentially aligned. In another exemplary embodiment, the conduit has a seam. In another exemplary embodiment, the seam of the conduit is essentially parallel to the longitudinal axis of the conduit. In another exemplary embodiment, the conduit is seamless. In another exemplary embodiment, the conduit is essentially seamless parallel to the longitudinal axis of the conduit. In another exemplary embodiment, the inner wall of the conduit consists of a layer of longitudinally aligned fibers while the outer wall of the conduit is composed of unaligned fibers. In another exemplary embodiment, the conduit is seamless parallel to the longitudinal axis of the conduit, and the inner wall of the conduit consists of a layer of longitudinally aligned fibers while the outer wall of the conduit is composed of unaligned fibers. The conduit defined in this instance is designed to display greater structural integrity due to the presence of randomly oriented fibers as an outer sheath. In another exemplary embodiment, the inner wall of the conduit is composed of unaligned randomly oriented fibers while the outer wall of the conduit is composed of a layer of longitudinally aligned fibers. In another exemplary embodiment, this conduit is seamless parallel to the longitudinal axis of the conduit. In another exemplary embodiment, the inner wall of the conduit is composed of longitudinally aligned fibers while the outer wall of the conduit is composed of circumferentially aligned fibers. In another exemplary embodiment, this conduit is seamless along an axis essentially parallel to the longitudinal axis of the conduit.

In an exemplary embodiment, the distance between the inner wall and the outer wall ofthe conduit is from about 1nm to 50,000 nm. In another exemplary embodiment, the distance between the inner wall and the outer wall of the conduit is from about 1nm to 10,000 nm. In another exemplary embodiment, the distance between the inner wall and the outer wall of the conduit is from about 1nm to 5,000 nm. In another exemplary embodiment, the distance between the inner wall and the outer wall of the conduit is from about 1nm to 500 nm. In another exemplary embodiment, the distance between the inner wall and the outer wall of the conduit is from about 1nm to 50 nm. In another exemplary embodiment, the distance between the inner wall and the outer wall of the conduit is from about 1nm to 5 nm. In another exemplary embodiment, the distance between the inner wall and the outer wall of the conduit is from about 10nm to 500 nm. In another exemplary embodiment, the distance between the inner wall and the outer wall of the conduit is from about 100nm to 1,000 nm. In another exemplary embodiment, the distance between the inner wall and the outer wall of the conduit is from about 5,000nm to 15,000 nm. In another exemplary embodiment, the distance between the inner wall and the outer wall of the conduit is from about 20,000nm to 50,000 nm. In another exemplary embodiment, the distance between the inner wall and the outer wall of the conduit is from about 75nm to 600 nm. In another exemplary embodiment, the distance between the inner wall and the outer wall ofthe conduit is from about 2,000 nm to 7,000 nm.

In an exemplary embodiment, the inner diameter of the conduit is from about 1nm to 50,000 nm. In another exemplary embodiment, the inner diameter of the conduit is from about 1nm to 10,000 nm. In another exemplary embodiment, the inner diameter of the conduit is from about 1nm to 5,000 nm. In another exemplary embodiment, the inner diameter of the conduit is from about 1nm to 500 nm. In another exemplary embodiment, the inner diameter of the conduit is from about 1nm to 50 nm. In another exemplary embodiment, the inner diameter of the conduit is from about 1nm to 5 nm. In another exemplary embodiment, the inner diameter of the conduit is from about 10nm to 500 nm. In another exemplary embodiment, the inner diameter of the conduit is from about 100nm to 1,000 nm. In another exemplary embodiment, the inner diameter ofthe conduit is from about 5,000nm to 15,000 nm. In another exemplary embodiment, the inner diameter of the conduit is from about 20,000nm to 50,000 nm. In another exemplary embodiment, the inner diameter of the conduit is from about 75nm to 600 nm. In another exemplary embodiment, the inner diameter of the conduit is from about 2,000 nm to 7,000 nm.

The conduits described herein can be produced in a number of ways. In an exemplary embodiment, the conduit is not electrospun. In another exemplary embodiment, the conduit is composed of random unoriented fibers or a random unoriented polymer scaffold.

In an exemplary embodiment, a fibrous polymer scaffold sheet is rolled to fabricate a conduit with a seam. First, a fibrous polymer scaffold sheet is/are electrospun. The fibers comprising the sheet can be aligned during electrospinning. Some methods which produce aligned electrospun fibers include the use either of a rotating drum as a grounded collector substrate or by using a mandrel described herein. In an exemplary embodiment, the mandrel is mandrel **56A.** The fibers comprising the sheet can also be aligned after electrospinning by mechanical uniaxial stretching. The aligned fibrous polymer scaffold sheet is then rolled around a mandrel to form a conduit. The mandrel can either be removed either before or after the conduit is fastened. In an exemplary embodiment, the two ends of the sheet which are parallel to the longitudinal axis of the polymer scaffold are then fastened together to produce a longitudinally aligned seamed conduit. In an exemplary embodiment, the sheet is rolled around the mandrel more than once and one end of the sheet is fastened to a part of the conduit to create a longitudinally aligned seamed conduit. The fastening can be accomplished by annealing (heat), adhesion or by sutures. Examples of adhesion involve solvents or biological adhesives such as fibrin sealant and collagen gels.

Reference will now be made in detail to several embodiments ofthe invention, examples of which are illustrated in the accompanying drawings. While the invention will be described in conjunction with the subsequent embodiments, it will be understood that they are not intended to limit the invention to those embodiments. On the contrary, the invention is intended to cover alternatives, modifications and equivalents, which may be included within the spirit and scope of the invention as defined by the appended claims.

In another exemplary embodiment, the invention provides a seamless conduit. **FIG. 1** refers to an electrospinning apparatus **30** for producing such a structure. The polymer solution **38,** which contains the polymer dissolved in a solvent, is contained within the syringe assembly **36.** The syringe assembly **36** is part of a syringe pump assembly **32** in which a computer **34** controls the rate at which the polymer solution exits the syringe by controlling pressure or flow rate. Optionally, different flow rates can be provided and controlled to selected spinnerets. The flow rate will change depending on the desired physical characteristics of the polymer scaffold, i.e., membrane thickness, fiber diameter, pore size, membrane density, etc.

The syringe pump assembly **32** feeds the polymer solution to a spinneret **42** that sits on a platform **44.** The spinneret has a tip geometry which allows for jet formation and transportation, without interference. A charge in the range of about 10 to about 30 kV is applied to the spinneret by a high voltage power supply **48** through wire **41A.**

A mandrel **56A** (which, as mentioned in **FIG. 3B****,** includes **55, 57A** and **57B)** is positioned below the spinneret **42** such that an electric field is created between the charged spinneret and the mandrel **56A.** The electric field causes a jet ofthe polymer solution to be ejected from the spinnerets and spray towards the mandrel **56A,** forming micron or nanometer diameter filaments or fibers **46.** The drill chucks are grounded using ground wires **41B** and **41C.**

The mandrel **56A** is attached to a first drill chuck **54** (attached to a non-conducting bearing **60)** and a second drill chuck **54A** (attached to a non-conducting bearing **60A)** which is connected to a motor **52.** The motor **52** is linked to a speed control **50** which controls the rate at which the motor spins the mandrel **56A.** Optionally, different spin rates can be provided. The spin rate will change depending on the desired physical characteristics ofthe polymer scaffold, i.e., membrane thickness, fiber diameter, pore size, membrane density, etc.

In another exemplary embodiment, the invention provides a seamless conduit produced via the electrospinning apparatus of **FIG. 2****.** This apparatus is similar to the apparatus of **FIG.1** but also comprises a tower **40** which holds the platform **44.**

Conduits with multiple layers of polymer scaffolds can be produced in a variety of ways. In an exemplary embodiment, additional polymer scaffold sheets can be wrapped around the outside or the inside of a conduit described herein. In an exemplary embodiment, a longitudinally aligned fibrous polymer scaffold conduit is created, either seamless or with a seam. Then a fibrous polymer scaffold sheet of unaligned micro/nanofibers is placed around the longitudinally aligned conduit to form a two layer conduit with an inner longitudinally aligned fibrous layer and an outer unaligned fibrous layer. Conduits with additional layers (three, four, five, six, etc.) are possible extensions of these methods. Sutures or adhesives can optionally be added to the polymer to maintain this structure.

In another exemplary embodiment, a longitudinally aligned fibrous polymer scaffold conduit is created, either seamless or with a seam. Then a circumferentially aligned fibrous polymer scaffold sheet is placed around the longitudinally aligned conduit to form a two layer conduit with an inner longitudinally aligned fibrous layer and an outer circumferentially aligned fibrous layer. Sutures or adhesives can optionally be added to the seamed polymer scaffold to maintain this structure.

In another exemplary embodiment, a seamless fibrous polymer scaffold conduit is created with an inner wall composed of longitudinally aligned fibers and an outer wall composed of circumferentially aligned fibers. The mandrel described herein with two conducting regions flanking a non-conducting region is used during electrospinning. The mandrel is rotated at a slow speed to allow for the even deposition of longitudinally aligned fibers. The mandrel is then rotated at a high speed to allow for the even deposition of circumferentially aligned fibers. The result is a seamless fibrous polymer scaffold conduit with an inner longitudinally aligned fiber layer and an outer circumferentially aligned fiber layer.

In another exemplary embodiment, a seamless fibrous polymer scaffold conduit is created with an inner wall composed of longitudinally aligned fibers and an outer wall composed of unaligned fibers. The specialized mandrel described above with two conducting regions flanking a non-conducting region is used during electrospinning. The mandrel is rotated at a slow speed to allow for the even deposition of longitudinally aligned fibers. The mandrel is then rotated at an intermediate speed that prevents both longitudinal and circumferential alignment of the deposited fibers. The result is a seamless fibrous polymer scaffold conduit with an inner longitudinally aligned fiber layer and an outer randomly aligned fiber layer.

### Rod

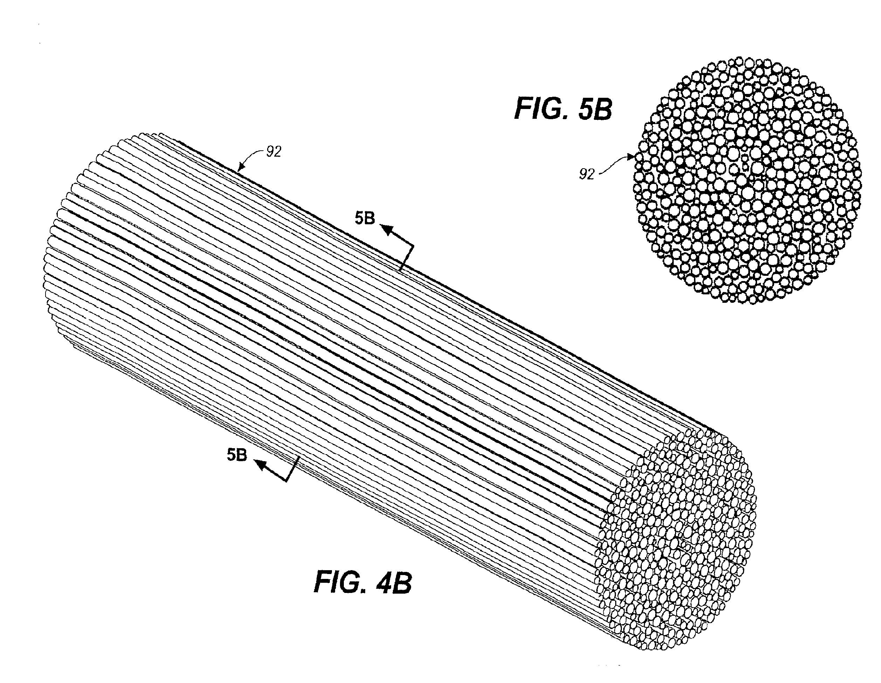

In another exemplary embodiment, the polymer scaffold has the shape of a rod. An exemplary depiction of a rod is provided in **FIG. 4B** and an exemplary depiction of a cross-sectional view of the rod is provided in **FIG. 5****B.** A rod can have a variety of sizes, depending on its length, as well as its diameter. The number the fibers within the rods can also be varied which will affect the density of the rod. These parameters can be varied to accommodate, for example, various tissue sizes and applications. In an exemplary embodiment, the rod is comprised of aligned fibers. In another exemplary embodiment, the fibers are longitudinally aligned or circumferentially aligned. In another exemplary embodiment, the rod has a seam. In another exemplary embodiment, the seam of the rod is essentially parallel to the longitudinal axis of the rod. In another exemplary embodiment, the rod is seamless. In another exemplary embodiment, the rod is essentially seamless parallel to the longitudinal axis of the conduit. In another exemplary embodiment, the rod includes a layer of longitudinally aligned fibers, which is covered by a conduit which is composed of unaligned fibers. In another exemplary embodiment, the rod is seamless parallel to its longitudinal axis, and the rod includes a layer of longitudinally aligned fibers which is covered by a conduit which is composed of unaligned fibers. The material defined in this instance is designed to display greater structural integrity due to the presence of randomly oriented fibers as an outer sheath. In another exemplary embodiment, rod is composed of unaligned randomly oriented fibers while the conduit is composed of a layer of longitudinally aligned fibers. In another exemplary embodiment, the rod is seamless parallel to its longitudinal axis. In another exemplary embodiment, the rod is composed of longitudinally aligned fibers which is covered by a conduit which is composed of circumferentially aligned fibers. In another exemplary embodiment, this rod is seamless parallel to its longitudinal axis.

In an exemplary embodiment, the diameter of the rod is from about 1nm to 50,000 nm. In another exemplary embodiment, the diameter of the rod is from about 1nm to 10,000 mn. In another exemplary embodiment, the diameter of the rod is from about 1nm to 5,000 mm. In another exemplary embodiment, the diameter ofthe rod is from about 1nm to 500 nm. In another exemplary embodiment, the diameter of the rod is from about 1nm to 50 nm. In another exemplary embodiment, the diameter of the rod is from about 1nm to 5 nm. In another exemplary embodiment, the diameter of the rod is from about 10nm to 500 nm. In another exemplary embodiment, the diameter of the rod is from about 100nm to 1,000 nm. In another exemplary embodiment, the diameter of the rod is from about 5,000nm to 15,000 nm. In another exemplary embodiment, the diameter of the rod is from about 20,000nm to 50,000 nm. In another exemplary embodiment, the diameter of the rod is from about 75nm to 600 nm. In another exemplary embodiment, the diameter of the rod is from about 2,000 nm to 7,000 nm.

The rods described herein can be produced in a number of ways. In an exemplary embodiment, the rod is not electrospun. In another exemplary embodiment, the rod is composed of random unoriented fibers or a random unoriented polymer scaffold.

In an exemplary embodiment, a fibrous polymer scaffold sheet is rolled to fabricate a rod with a seam. First, a fibrous polymer scaffold sheet is/are electrospun. The fibers comprising the sheet can be aligned during electrospinning. Some methods which produce aligned electrospun fibers include the use either of a rotating drum as a grounded collector substrate or by using a mandrel described herein. In an exemplary embodiment, the mandrel is mandrel **56B**. The fibers comprising the sheet can also be aligned after electrospinning by mechanical uniaxial stretching. The aligned fibrous polymer scaffold sheet is then rolled over itself to form a rod. An end of the polymer scaffold sheet is then fastened to a part of the rod to create a longitudinally aligned seamed rod. The sheet can be fastened together by annealing (heat), adhesion or by sutures. Examples of adhesion involve solvents or biological adhesives such as fibrin sealant and collagen gels.

Reference will now be made in detail to several embodiments of the invention, examples of which are illustrated in the accompanying drawings. While the invention will be described in conjunction with the subsequent embodiments, it will be understood that they are not intended to limit the invention to those embodiments. On the contrary, the invention is intended to cover alternatives, modifications and equivalents, which may be included within the spirit and scope of the invention as defined by the appended claims.

In another exemplary embodiment, the invention provides a seamless rod. **FIG. 6** refers to an electrospinning apparatus **80** for producing such a structure. The polymer solution **38,** which contains the polymer dissolved in a solvent, is contained within the syringe assembly **36.** The syringe assembly **36** is part of a syringe pump assembly **32** in which a computer **34** controls the rate at which the polymer solution exits the syringe by controlling pressure or flow rate. Optionally, different flow rates can be provided and controlled to selected spinnerets. The flow rate will change depending on the desired physical characteristics of the polymer scaffold, i.e., membrane thickness, fiber diameter, pore size, membrane density, etc.

The syringe pump assembly **32** feeds the polymer solution to a spinneret **42** that sits on a platform **44.** The spinneret has a tip geometry which allows for jet formation and transportation, without interference. A charge in the range of about 10 to about 30 kV is applied to the spinneret by a high voltage power supply **48** through wire **41A.**

A mandrel **56B** (which, as mentioned in **FIG. 3C****,** includes **57A, 57B** and **58)** is positioned below the spinneret **42.** The mandrel **56B** has a first electrically conducting region **57A** and a first electrically conducting face **57C,** a second electrically conducting region **57B** and a second electrically conducting face **57D,** such that an electric field is created between the charged spinneret and the mandrel **56B.** The electric field causes a jet of the polymer solution to be ejected from the spinnerets and spray towards the mandrel **56B,** forming micron or nanometer diameter filaments or fibers within **58.** The drill chucks are grounded using ground wires **41B** and **41C.**

The first electrically conducting region **57A** is attached to a first drill chuck **54** (attached to a non-conducting bearing **60)** and the second electrically conducting region **57B** is attached to a second drill chuck **54A** (attached to a non-conducting bearing **60A)** which is connected to a motor **52A.** Motor **52** and **52A** are linked to a speed control **50A** which controls the rate at which the motor spins the mandrel **56B**. Optionally, different spin rates can be provided. The spin rate will change depending on the desired physical characteristics of the polymer scaffold, i.e., membrane thickness, fiber diameter, pore size, membrane density, etc.

In another exemplary embodiment, the invention provides a seamless rod produced via the electrospinning apparatus of **FIG. 7****.** This apparatus is similar to the apparatus of **FIG.6** but also comprises a tower **40** which holds the platform **44.**

Rods with multiple layers of polymer scaffolds can be produced in a variety of ways. In an exemplary embodiment, additional polymer scaffold sheets can be wrapped around the outside of the rod described herein. In an exemplary embodiment, a longitudinally aligned fibrous polymer scaffold rod is created, either seamless or with a seam. Then a fibrous polymer scaffold sheet of unaligned micro/nanofibers is placed around the longitudinally aligned rod to form a two layer polymer scaffold with an inner longitudinally aligned fibrous layer and an outer unaligned fibrous layer. Rods with additional layers (three, four, five, six, etc.) are possible extensions of these methods. Sutures or adhesives can optionally be added to the polymer to maintain this structure. Some of these multiple layered rod embodiments may also be termed 'filled conduits'.

In another exemplary embodiment, a longitudinally aligned fibrous polymer scaffold rod is created, either seamless or with a seam. Then a circumferentially aligned fibrous polymer scaffold sheet is placed around the longitudinally aligned rod to form a two layer rod with an inner longitudinally aligned fibrous layer and an outer circumferentially aligned fibrous layer. Sutures or adhesives can optionally be added to the seamed polymer scaffold to maintain this structure.

In another exemplary embodiment, a seamless fibrous polymer scaffold has a interior rod composed of longitudinally aligned fibers and an exterior conduit or sleeve composed of circumferentially or randomly aligned fibers. A seamless longitudinally aligned fibrous polymer rod scaffold is fabricated as described herein. In order to form the exterior conduit or sleeve of circumferentially aligned fibers, the rotation of the mandrels is increased to a high speed to allow for the even deposition of circumferentially aligned fibers around the longitudinally aligned fibrous polymer rod. Alternatively, to form the exterior conduit or sleeve of randomly aligned fibers, the rotation of the mandrels is increased to an intermediate speed that prevents both longitudinal and circumferential alignment of fibers that are deposited on the longitudinally aligned fibrous polymer rod. Upon removal of the scaffold rod from the mandrels, the result is a seamless fibrous polymer scaffold with an interior rod composed of longitudinally aligned fibers and an exterior conduit or sleeve composed of either circumferentially aligned or unaligned fibers.

### Filled conduit

In an exemplary embodiment, the polymer scaffold has the shape of a filled conduit. The filled conduit can be produced as follows: (1) a conduit is formed as described herein; and (2) filler material for the filled conduit is composed of longitudinally aligned fibers. This filler material can be a loose, highly porous material. In an exemplary embodiment, the filler material is electrospun as a thin membrane of aligned fibers. The material is then directly inserted within the conduit described herein with the orientation of the aligned fibers parallel to the long axis of the conduit. In another instance, a rod of longitudinally aligned fibers is produced as described herein. This rod is then either: (1) directly inserted within a fully formed conduit or (2) used as a mandrel around which a fibrous sheet is rolled and then sealed with sutures or adhesive to form a filled conduit.

### II. f) Additional Composition or Polymer Scaffold Components

### II. f1) Cell

In an exemplary embodiment, the compositions and/or polymer scaffolds described herein further comprise a cell. The cell can be on the surface or embedded or entangled within the compositions and/or polymer scaffold. In an exemplary embodiment, the cell is covalently attached or non-covalently associated with the compositions and/or polymer scaffolds of the invention. In some embodiments, the cell is utilized to promote the growth of new tissue. In an exemplary embodiment, the cell is a member selected from autologous (donor and recipient are the same individual), allogeneic (donor and recipient are not the same individual, but are from the same species) and heterologous (donor and recipient are from different species). In an exemplary embodiment, the cell is not a stem cell. In an exemplary embodiment, the cell is a stem cell. In an exemplary embodiment, the cell is a member selected from an adult stem cell and an embryonic stem cell. In another exemplary embodiment, the adult stem cells can be mesenchymal stem cells (MSC) (derived from bone marrow) or adipose derived adult stem cells (ADAS). In another exemplary embodiment, the stem cell is a member selected from unipotent, multipotent, pluripotent and totipotent. In an exemplary embodiment, the cell is a progenitor cell. In another exemplary embodiment, the progenitor cells can be fibroblasts, myoblasts, neural progenitor cells, hematopoietic progenitor cells, and endothelial progenitor cells. In another exemplary embodiment, the cell is a member selected from myoblasts and muscular progenitor cells. In another exemplary embodiment, the cell is a member selected from an adult muscle cell, a muscle progenitor cell, a muscle stem cell or combinations thereof. In another exemplary embodiment, the cell is a member selected from an adult vascular cell, a vascular progenitor cell, a vascular stem cell or combinations thereof. In another exemplary embodiment, the cell is a member selected from adult neural cells, glial cells, neural progenitor cells, glial progenitor cells, neural stem cells, neuroepithelial cells or combinations thereof. In another exemplary embodiment, the cell is a member selected from a Schwann cell, a fibroblast and a vascular cell. In another exemplary embodiment, the cell is a member selected from an adult skin cell, a skin progenitor cell, and a skin stem cell. Recently, the feasibility of nanofiber substrates for guidance of cell growth, function, and organization has been demonstrated for fibroblasts, vascular cells, and mesenchymal stem cells. Zong, X., *Biomacromoleucles* 2003, 4, (2), 416-23; Li, D., *Adv Mater* 2004, 16, (4), 1151-1170; Boland, E.D., *Front Biosci* 2004, 9, 1422-32; Bhattarai, S.R., *Biomaterials* 2004, 25, (13), 2592-602; Yoshimoto, H., *Biomaterials* 2003, 24, (12), 2077-82.

In another embodiment, the cell-embedded compositions and/or polymer scaffolds described herein can be used in the development of three-dimensional tissues. In another exemplary embodiment, myoblast-embedded polymer scaffolds can be used to develop muscle tissue, neural cell-embedded polymer scaffolds can be used to develop nervous tissue, vascular cell-embedded polymer scaffolds can be used to develop vascular tissue, spinal cord cell-embedded polymer scaffolds can be used to develop spinal cord tissue and skin cell embedded polymer scaffolds can be used to develop skin tissue.

Cells can be incorporated within the compositions and/or polymer scaffolds after electrospinning or post-fabrication.

### II. f2) Biomolecules

A biomolecule (such as a nucleic acid, amino acid, sugar or lipid) can be covalently attached or non-covalently associated with the composition and/or polymer scaffolds described herein. In an exemplary embodiment, the biomolecule is a member selected from a receptor molecule, extracellular matrix component or a biochemical factor. In another exemplary embodiment, the biochemical factor is a member selected from a growth factor and a differentiation factor. In an exemplary embodiment, the biomolecules is a member selected from glycosaminoglycans and proteoglycans. In an exemplary embodiment, the biomolecule is a member selected from heparin, heparan sulfate, heparan sulfate proteoglycan and combinations thereof.

In another exemplary embodiment, a first molecule (which may or may not be a biomolecule) is covalently attached to the composition and/or polymer scaffold of the invention. This first molecule can be used to interact with a second biomolecule. In an exemplary embodiment, the first molecule is a linker, and the second biomolecule is a member selected from a receptor molecule, biochemical factor, growth factor and a differentiation factor. In an exemplary embodiment, the first molecule is a member selected from heparin, heparan sulfate, heparan sulfate proteoglycan, and combinations thereof. In an exemplary embodiment, the second biomolecule is a member selected from a receptor molecule, biochemical factor, growth factor and a differentiation factor. In another exemplary embodiment, the first molecule is covalently attached through a linker, and said linker is a member selected from di-amino poly(ethylene glycol), poly(ethylene glycol) and combinations thereof. For biomolecules that do not bind to heparin, direct conjugation to the polymer scaffold or through a linker (such as PEG, amino-PEG and di-amino-PEG) is also feasible. In another exemplary embodiment, the biomolecule is an extracellular matrix component which is a member selected from laminin, collagen, fibronectin, elastin, vitronectin, fibrinogen, polylysine, other cell adhesion promoting polypeptides and combinations thereof. In another exemplary embodiment, the biomolecule is a growth factor which is a member selected from acidic fibroblast growth factor, basic fibroblast growth factor, nerve growth factor, brain-derived neurotrophic factor, insulin-like growth factor, platelet derived growth factor, transforming growth factor beta, vascular endothelial growth factor, epidermal growth factor, keratinocyte growth factor and combinations thereof. In another exemplary embodiment, the biomolecule is a differentiation factor which is a member selected from stromal cell derived factor, sonic hedgehog, bone morphogenic proteins, notch ligands, Wnt and combinations thereof.

The first molecules which are covalently attached to the polymer scaffold of the invention can be used to interact with a biomolecule (for example, a growth factor and/or ECM component) in order to stimulate neurite growth. In another exemplary embodiment, the polymer scaffold can be used for wound healing, and the biomolecule which is a member selected from an extracellular matrix component, growth factors and differentiation factors. Examples of potential factors for wound healing enhancement include epidermal growth factor (EGF), vascular endothelial growth factor (VEGF), basic fibroblast growth factor (bFGF) and platelet-derived growth factor (PDGF).

Biomolecules can be incorporated within the compositions of the invention during electrospinning or post-fabrication. These biomolecules can be incorporated via blending, covalent attachment directly or through various linkers or by adsorption.

### II. f3) Pharmaceutically Acceptable Excipients/Pharmaceutical Formulations

A pharmaceutically acceptable excipient can also be included in compositions with the polymer scaffolds of the invention. In an exemplary embodiment, the invention provides a composition which is a pharmaceutical formulation comprising: a) a polymer scaffold of the invention; and b) pharmaceutically acceptable excipient. In an exemplary embodiment the pharmaceutical formulation is a polymer scaffold in which a pharmaceutically acceptable excipient is present. In an exemplary embodiment, the pharmaceutically acceptable excipient is a member selected from inert diluents, granulating and disintegrating agents, binding agents, lubricating agents, and a time delay material.

The pharmaceutical formulations of the invention can take a variety of forms adapted to the chosen route of administration. Those skilled in the art will recognize various synthetic methodologies that may be employed to prepare non-toxic pharmaceutical formulations incorporating the compounds described herein. Those skilled in the art will recognize a wide variety of non-toxic pharmaceutically acceptable solvents that may be used to prepare solvates of the compounds of the invention, such as water, ethanol, propylene glycol, mineral oil, vegetable oil and dimethylsulfoxide (DMSO).

The compositions of the invention may be administered through surgical incision, topically, or parenterally in dosage unit formulations containing conventional non-toxic pharmaceutically acceptable carriers, adjuvants and vehicles.

In another exemplary embodiment, the compositions and/or polymer scaffolds described herein are part of a kit. This kit can comprise an instruction manual that teaches a method of the invention and/or describes the use of the components of the kit.

### III. Uses for the Compositions

In another aspect, a composition of the invention (such as a polymer scaffold) can be used in a subject in order to replace, regenerate or improve a biological function. In an exemplary embodiment, the composition replaces, regenerates or improves nerve function or muscle function or skin function or vascular function in a subject. In another aspect, the invention provides a method of treating an injury in a subject, said method comprising: (a) contacting said subject with a therapeutically effective amount of the composition of the invention, sufficient to treat the injury. In an exemplary embodiment, the composition contacts the subject at the site of the injury. In another exemplary embodiment, the injury is a member selected from a severed nerve, a damaged nerve, a severed muscle, a damaged muscle, a severed blood vessel, a damaged blood vessel, a skin wound and bruised skin. In another aspect, the invention provides a method of growing tissue in a subj ect, said method comprising: (a) contacting said subject with a therapeutically effective amount of the composition of the invention, sufficient to facilitate growth of said tissue. In an exemplary embodiment, the tissue is a member selected from muscle tissue, vascular tissue, nerve tissue and skin tissue. The compositions can be used *in vitro* or *in vivo* to test for their efficacy. In another exemplary embodiment, the subject is an animal. In another exemplary embodiment, the animal is a member selected from a human, a dog, a cat, a horse, a rat and a mouse.

The following are examples of the uses of the compositions ofthe invention.

### III a) Uses Involving Nerves

In an exemplary embodiment, the compositions described herein are used to replace severed or damaged nerves. One use is for the regeneration of damaged peripheral nerves. Peripheral nerve damage can be caused by trauma, autoimmune disease, diabetes, etc. Peripheral nerves are composed of nerve fibers that run from the spinal cord to various end targets throughout the body. Peripheral nerve injuries result in at least partial loss of motor and sensory function at the nerve's end targets. In the most severe forms of injury, the nerve is completely severed and a large injury gap forms between the proximal and distal nerve stumps. The nerve fibers at the proximal end are capable of regeneration but are unable to do so efficiently over gaps longer than a few millimeters. Thus it is imperative to bridge the injury gap with materials that efficiently guide regenerating nerve fibers from the proximal nerve segment to the distal nerve segment.

In a clinical situation where the peripheral nerve is damaged and is no longer in continuity, at least one ofthe longitudinally aligned fibrous polymer scaffolds described herein may be used. The polymer scaffold can be in the shape of a conduit, a filled conduit or a rod. Injuries that result in nerve discontinuity commonly occur at the limbs. The aligned fibrous scaffold can be implanted in these regions to enhance nerve regeneration across the injury gap and allow for the return of motor and sensory function to the limbs. The scaffolds may be used in all situations involving discontinuous damaged nerves where the gap between the nerve stumps is large enough to prevent direct reattachment. In each case, the scaffold would bridge the two ends of the nerve, be sutured to the nerve segments and enhance and direct the regeneration of nerve fibers from the proximal nerve segment to the distal nerve segment. The longitudinally aligned fibers that compose the scaffold would be aligned in essentially the same orientation as the nerve fibers. Thus the aligned scaffold fibers could provide specific guidance cues that direct the regenerating nerve fibers efficiently across the injury gap. The longitudinally aligned conduit polymer scaffolds may function by first promoting directed outgrowth of the nerve fibers positioned along the periphery of the proximal stump. The rod shaped and filled conduit shaped longitudinally aligned polymer scaffolds may be capable of continuously guiding all nerve fibers from the proximal to the distal nerve segments. The polymer scaffolds may also be loaded with biomolecules such as extracellular matrix proteins, polypeptides, growth factors and/or differentiation factors to further enhance and guide nerve fiber regeneration. Extracellular matrix proteins which can be added to the polymer scaffolds include collagen, laminin, and fibronectin. Growth factors which can be added to the polymer scaffolds include basic fibroblast growth factor, nerve growth factor and vascular endothelial growth factor. Polypeptides which can be added to the polymer scaffold include polylysine, RGD based polypeptides and laminin mimetic polypeptides. Other biomolecules which can be added to the polymer scaffolds include heparin and heparan sulfate proteoglycan. These biomolecules can also be used to non-covalently entrap laminin, VEGF and BFGF onto the fibrous polymer scaffolds.

The scaffolds can be shaped and sized to match the specific requirements of the patients' nerve injuries. For instance, the inner diameters of the conduits, filled conduits and the overall diameters of the rod shaped polymer scaffolds can be from about 1mm to about 20 mm. In another exemplary embodiment, the diameters can be from about 2mm to about 8 mm. In another exemplary embodiment, the diameters can be from about 5mm to about 10 mm. In another exemplary embodiment, the diameters can be from about 2mm to about 15 mm. In another exemplary embodiment, the diameters can be from about 12mm to about 18 mm. In another exemplary embodiment, the diameters can be about 1mm, 1.5mm, 2mm, 2.5mm, 3mm, 3.5mm, 4mm, 4.5mm, 5mm, 5.5mm, 6mm, 6.5mm, 7mm, 7.5mm, 8mm, 8.5mm, 9mm, 9.5mm, 10mm, 10.5mm, 11mm, 11.5mm, 12mm, 12.5mm, 13mm, 13.5mm, 14mm, 14.5mm, 15mm, 15.5mm, 16mm, 16.5mm, 17mm, 17.5mm, 18mm, 18.5mm, 19mm, 19.5mm, 20mm, 21mm for specific size matching with injured nerves. The lengths of the scaffolds can vary from 1cm to about 50 cm to accommodate a large range of injury gaps. In another exemplary embodiment, the scaffold length can be from about 4cm to about 15 cm. In another exemplary embodiment, the scaffold length can be from about 14cm to about 30 cm. In another exemplary embodiment, the scaffold length can be from about 1cm to about 5 cm. In another exemplary embodiment, the scaffold length can be from about 2cm to about 8cm. In another exemplary embodiment, the scaffold length can be about 1cm, 1.5cm, 2cm, 2.5cm, 3cm, 3.5cm, 4cm, 4.5cm, 5cm, 5.5cm, 6cm, 6.5cm, 7cm, 7.5cm, 8cm, 8.5cm, 9cm, 9.5cm, 10cm, 10.5cm, 11cm, 11.5cm, 12cm, 12.5cm, 13cm, 13.5cm, 14cm, 14.5cm, 15cm, 15.5cm, 16cm, 16.5cm, 17cm, 17.5cm, 18cm, 18.5cm, 19cm, 19.5cm, 20cm, 20.5cm. All forms of the longitudinally aligned fibrous scaffolds can serve as a replacement for nerve autografts, currently the most widely used but far from perfect form of treatment for nerve injuries. The scaffolds may also be used to bridge long injury gaps beyond the range covered by current synthetic nerve guidance products. For example, injury gaps to be bridged can be over 3 cm. In an exemplary embodiment, a subject has a long injury gap, and a rod shaped polymer scaffold or a filled conduit polymer scaffolds may be the most preferred scaffold shapes for nerve regeneration across long injury gaps.

In a clinical situation where a peripheral nerve is damaged but not severed, the longitudinally aligned fibrous scaffolds described in this invention can be shaped as a sheet and used as a wrap around the nerve and/or can be shaped as rods or filled conduits or conduits and inserted directly into the damaged region. The longitudinally aligned fibrous scaffolds can also be loaded with similar biomolecules as described herein.

Another use of scaffolds described in this invention is for the regeneration of damaged spinal cords. Aligned fibrous polymer scaffolds can be inserted into the damaged region to bridge spinal cord tissue. The aligned fibrous polymer scaffolds can enhance and direct the regeneration of spinal nerve fibers. The scaffolds can also be loaded with biomolecules and/or cells. Biomolecules could include extracellular matrix proteins, polypeptides, growth factors and/or differentiation factors as listed above and could initiate and enhance spinal nerve regeneration. Cells could include neural stem cells, glial cells, and/or neural progenitors. The cells could replace lost neurons and glial cells and/or could support and enhance the growth of spinal nerves.

In another exemplary embodiment, a longitudinally aligned polymer conduit scaffold is used as a nerve guidance conduit to promote nerve regeneration across an injury gap.

### III b) Uses Involving Skin

Polymer scaffolds ofthe invention described can be useful for clinical and personal wound care and soft tissue regeneration. In one aspect of the invention, polymer scaffold sheets are used as a wound dressing or graft for external skin wounds. In a clinical setting, these sheets can be used to treat wounds resulting from trauma, burns, ulcers, abrasions, lacerations, surgery, or other damage. Surgeons can use these grafts to cover and protect the wound area, to temporarily replace lost or damaged skin tissue, and to guide new tissue generation and wound healing into the damaged area. In a clinical setting, micro/nanofiber sheets may be secured to the wound area using sutures, adhesives, or overlaying bandages. These micro/nanofiber wound dressings may be cut to match the size of the wound, or may overlap the wound edges.

In another aspect of the invention, the polymer scaffold sheets may be tailored for personal/home care by combining the sheet with an adhesive backing to create a polymer scaffold bandage. The adhesive section will hold the polymer scaffold sheet in place on a wounded area and can be removed when the fibers degrade or fuse with the tissue. The polymer scaffold sheet may also be secured with a liquid or gel adhesive.

In another aspect of the invention, large polymer scaffold sheets can be used as gauze to absorb fluid and protect large wounds. This polymer scaffold gauze can be wrapped around a wounded area or secured with tape.

In another aspect of the invention, polymer scaffold sheets can be used to treat internal soft tissue wounds such as wounds in the amniotic sac, ulcers in the gastrointestinal tract or mucous membranes, gingival damage or recession, internal surgical incisions or biopsies, etc. Again, the polymer scaffold grafts can be sutured or adhered into place to fill or cover the damaged tissue area.

Polymer scaffold have numerous characteristics that are useful for wound healing. First, the polymer scaffolds described herein that include nanofibers are both nano-porous and breathable. They can prevent microbes and infectious particles from crossing through, but they allow air flow and moisture penetration which are critical in natural wound healing.

Second, the fibers in this invention are biodegradable, which allows for temporary wound coverage followed by eventual ingrowth of new tissue. The choice of material for polymer scaffold wound dressings can be determined to match the natural tissue characteristics including mechanical strength and rate of degredation/tissue regeneration.

Third, the polymer scaffolds may be embedded or conjugated with various factors which may be released upon degredation. These factors may include, but are not limited to epidermal growth factor (EGF), platelet derived growth factor (PDGF), basic fibroblast growth factor (bFGF), transforming growth factor-β (TGF-β), and tissue inhibitors of metalloproteinases (TIMP), which have been shown to be beneficial in wound healing Fu, X. *et al., Wound Repair Regen,* **13(2):**122-30 (2005). Additional wound healing factors such as antibiotics, bacteriocides, fungicides, silver-containing agents, analgesics, and nitric oxide releasing compounds can also be incorporated into the polymer scaffold wound dressings or grafts.

Fourth, polymer scaffold grafts for wound healing may be seeded with cells for faster tissue regeneration and more natural tissue structure. These cells may include, but are not limited to fibroblasts, keratinocytes, epithelial cells, endothelial cells, mesenchymal stem cells, and/or embryonic stem cells.

Fifth, the nano-scale architecture of the nanofibrous polymer scaffolds closely mimics that of the extracellular matrix (ECM) of many common soft tissues. For example, the nano-scale fibers are structurally similar to collagen fibrils found in skin and other tissues. This architecture may prevent scar formation by providing an organized scaffold for cells to migrate into a wound. In this aspect of the invention, alignment of the polymer scaffold (as opposed to randomly oriented fibers) is important to keep cells aligned and organized, rather than allowing them to arrange randomly as in the formation of scar tissue. Aligned polymer scaffolds may be oriented with respect to a given axis of the wound to allow faster tissue ingrowth and wound coverage.

Polymer scaffold alignment can also be used to closely match the architecture of natural tissue ECM. This may include fiber alignment in a single direction, criss-cross alignment in orthogonal directions, or more complicated fiber architecture. In this instance of the invention, the polymer scaffold includes multiple layers of fibers with specific fiber orientation in each layer. Similarly, each individual polymer scaffold layer may also contain a specific factor or cell type such as the ones listed previously. This allows for creation of polymer scaffolds that can closely match natural tissue architecture and composition. For example, a simple polymer scaffold wound dressing or graft might include a single layer of aligned fibers. On the other hand, a more complex polymer scaffold skin graft might include multiple aligned fiber sheets layered in a criss-cross pattern with fibroblasts in the bottom sheets and keratinocytes in the top sheet, as well as bFGF in the bottom sheets and an antimicrobial agent in the top sheet. Other such combinations are possible, depending on the specific needs of the patient.

### III. c) Uses involving the Vascular System

The polymer scaffolds described herein can be used to replace or bypass a variety of damaged, severed or altered blood vessels. In an exemplary embodiment, the conduit or filled conduit polymer scaffolds are used in coronary artery bypass surgery. In addition, these grafts can be used to support and stabilize blood vessel aneurysms (ie - abdominal aortic aneurysms typically require synthetic polymer replacement grafts, such as ePTFE or Dacron) by either complete replacement of the vessel with the polymer scaffold or by creating a sheath like encasement. Other reinforcement techniques involve wrapping polymer scaffold sheets around the aneurysm site. Their uses are not limited to lower body vessel replacement, but may include other common sites of aneurysms; for example - the Circle of Willis, involving any of the local arteries, including the internal carotid, posterior communicating, posterior cerebral, etc.

In an exemplary embodiment, the polymer scaffold which is surrounded by a sleeve is used to replace or regenerate a blood vessel. A sleeve can be made to surround the polymer scaffold to improve its mechanical strength, rigidity, compliance or any other physical or chemical property. This sleeve can be placed around a nanofibrous polymer scaffold conduit, for example, such that the underlying nanofibers will have a particular direction of alignment and the sleeve may have the same or different direction of alignment. Unaligned or randomly aligned micro or nanofibers can also serve as the sleeve material or the underlying nanofiber construct. Multiple sleeves can be used to create a multi-layered construct with different physical or chemical properties.

### Multiple cell type graft

A tubular construct can be made by seeding different regions of the polymer scaffold with different cell types. For example, **FIG. 40** shows a graft with cell type1 lining the lumen and cell type 2 in the medial portions of the graft and cell type 3 used to encase the graft by seeding on another region of the graft.

### Modifications to tubular constructs of nanofibers

In an exemplary embodiment, the invention provides a polymer scaffold for use in the vascular system which has no biochemical or cellular modifications prior to implantation. In another exemplary embodiment, the invention further comprises poly(ethylene glycol) or similar biochemical modification to create a non-fouling, non-thrombogenic brush layer which prevents platelets from adhering to the nanofibers. This brush layer can be covalently grafted onto the nanofibrous polymer scaffold for thrombosis reduction. In another exemplary embodiment, the polymer scaffold further comprises heparin, hirudin or combinations thereof Heparin is capable of binding to anti-thrombin III, which can block Factor Xa and thrombin in the bloodstream. Hirudin is an inhibitor of thrombin. Heparin and hirudin can be covalently grafted onto the polymer scaffold by using a di-amino poly(ethylene glycol) linker. In an exemplary embodiment, the polymer scaffold contains PLLA fibers. The polymer scaffolds of the invention which are used in connection with the vascular system of a subject reduce thrombosis and increase graft patency.

In another exemplary embodiment, the polymer scaffolds used in connection with the vascular system further comprise human bone marrow-derived mesenchymal stem cells. In an exemplary embodiment, the mesenchymal stem cells will be seeded at least twenty-four hours prior to implantation. In another exemplary embodiment, the cells will be seed onto the graft two days prior to implantation.

Decellularization - Human bone marrow-derived mesenchymal stem cells or any cell type will be seeded as described above. Several hours prior to implantation, the cells will be killed, while leaving the cellular structure and cell surface intact.

In another exemplary embodiment, the polymer scaffolds used in connection with the vascular system further comprise endothelial precursor cells. In an exemplary embodiment, the endothelial precursor cells will be seeded 2 days onto the graft two days prior to implantation. These endothelial cells can prevent platelet adhesion/activation, thrombosis and fibrin formation via a highly active cell membrane surface involving heparan sulfate proteoglycans and several factors secreted by the cells themselves.

In another exemplary embodiment, the polymer scaffolds used in connection with the vascular system further comprise human embryonic stem cell-derived vascular progenitor cells. These embryonic stem cells can differentiate into a vascular progenitor lineage through collagen IV integrin signaling and are capable of fully differentiating into endothelial and smooth muscle cells upon growth factor stimulation.

### III d) Uses Involving Muscles

A muscle graft involving a polymer scaffold of the invention can be therapeutically implanted intramuscularly for enhancing muscle regeneration due to significant muscle loss after acute injury. Growth factors and other biomolecules can be incorporated into the muscle graft to stimulate muscle cell proliferation and differentiation as well as vascularization, which will all accelerate muscle healing. The growth factors can include insulin-like growth factor-1 (IGF-1), basic growth factor (bFGF), vascular endothelial growth factor (VEGF), and platelet-derived growth factor (PDGF). The growth factors can be incorporated into the graft by covalent binding or physiological coating. A related approach involves genetically modifying the cells to overexpress genes for such proteins.

Another therapeutic application of the graft is for genetic modification of animals or humans with muscular diseases such as muscular dystrophy which are characterized by genetic mutations of specific genes. For this type of gene therapy application, myoblasts with the normal gene can be delivered within the graft to the muscle. Alternatively, the gene can be directly conjugated onto the scaffold in the form of plasmid DNA. Engraftment of the scaffold within the body can lead to genetic modification to help the tissue develop aspects of normal muscle function. For the purpose of gene therapy, the polymer scaffold provides a matrix for the survival and growth of implanted cells and/or serves as a delivery vchiele for the release and subsequent uptake of plasmid DNA.

The graft can be in the shape of a patch or a conduit. In both cases the fiber direction is parallel to the direction of the muscle. For rodents, the physical dimension of the scaffold ranges from 0.5-5.0 X 0.5-5.0 X 0.1-0.5 cm. For human grafts, the scaffold size ranges from 1.0-50.0 X 1.0-50.0 X 0.1-5.0 cm.

The muscles most suited for this type of therapeutic treatment are those that have an aligned geometry and are close to vasculature to nourish the graft. Such muscles include the biceps, quadriceps, anterior tibialis, and gastrocnemius.

In an exemplary embodiment, a conduit embedded with myoblasts can be used to grow skeletal muscle. In another exemplary embodiment, a conduit embedded with mesenchymal stem cells can be used as a vascular graft. In another exemplary embodiment, a conduit embedded with neural stem cells can be used as a nerve graft. In another exemplary embodiment, the invention provides methods of making the composition which comprises (i) seeding the polymer with the cells; (ii) rolling the product around a mandrel, thus forming a tubular shape for the polymer; and (iii) removing the mandrel. In another exemplary embodiment, the invention provides (iv) attaching a first portion of the polymer to a second portion of the polymer. This attaching can be accomplished with the use of annealing (heat), adhesion (such as biological adhesives) or sutures.

### III. e) Muscle Grafts with Micropatterned Polymer Scaffolds

The muscle graft applications with micropatterned polymer scaffolds are similar to that of nanofibrous scaffolds as described above, with the exception that the micropatterned polymer scaffolds would be in the shape of a sheet.

The invention is further illustrated by the Examples that follow. The Examples are not intended to define or limit the scope of the invention.

### EXAMPLES

### EXAMPLE 1

### Myoblast Preparation

Murine C2C12 myoblasts (ATCC, Manassas, VA) were used to study cell organization and assembly. The myoblasts were cultured in growth media that consisted of Dulbecco's Modified Eagle's Medium (DMEM), 10% fetal bovine serum, and 1% penicillin/streptomycin. To initiate myoblast differentiation and fusion, the growth media was replaced with differentiation media that consisted of DMEM, 5% horse serum, and 1% penicillin/streptomycin after 24 h when samples were confluent. In all experiments, time points were denoted by the incubation time in differentiation media.

### EXAMPLE 2

### PLLA Nanofiber Scaffold Preparation

Biodegradable poly(L-lactide) (PLLA) (Lactel Absorbable Polymers, Pelham, AL, 1.09 dL/g inherent viscosity) was used to fabricate nanofiber scaffolds by electrospinning. (Zong, X., *Biomacromolecules,* **4(2)**: 416-23 (2003)). Briefly, the PLLA solution (10% w/v in chloroform) was delivered by a programmable pump to the exit hole of the electrode at a flow rate of 25 µL/minute. A high-voltage supply (Glassman High Voltage Inc., High Bridge, NJ) was used to apply the voltage at 20 kV. The collecting plate was on a rotating drum that was grounded and controlled by a stepping motor. To align the nanofibers, the electrospun scaffold was stretched uniaxially to 200% engineering strain at 60 °C. Nanofibrous scaffolds were approximately 150 µm in thickness. The surface of the nanofibrous scaffold was coated with 2% gelatin or fibronectin (5 µg/cm²) before cell seeding. No significant difference in cell adhesion and morphology was detected between gelatin and fibronectin coating. Randomly-oriented scaffolds were used as controls.

Scanning electron microscopy (SEM) was used to visualize nanofiber alignment after uniaxial stretching. SEM images show that uniaxial stretching resulted in aligned nanofibers (**FIG. 24A-B****).** The average nanofiber diameter in the scaffold was approximately 500 nm with an average gap size of approximately 4 µm.

### EXAMPLE 3

### Growing/Characterization of Myoblasts on Nanofiber Scaffolds under Differentiation Media

Confluent myoblasts were grown on the nanofibrous scaffolds in differentiation media for up to 7 days. To determine cell organization and cytoskeletal structure on random-oriented and aligned nanofibrous scaffolds, fluorescence staining of F-actin, myosin heavy chain (MHC) and cell nuclei were performed (Supporting Information). F-actin was stained using fluorescein (FITC)-conjugated phalloidin (Molecular Probes, Eugene, OR). MHC was immunostained with a mouse anti-skeletal fast MHC antibody (Sigma, St. Louis, MO). Cell nuclei were stained with ToPro dye (Molecular Probes). Fluorescence microscopy was performed by using a Nikon TE300 microscope and Leica TCS SL confocal microscope.

### EXAMPLE 4

### Microfabrication of Micropatterned Substrates

Micropatterned films composed of polydimethylsiloxane (PDMS) or poly-L-lactide-co-glycolide-co-ε-caprolactone (PLGC) polymers were fabricated from a silicon wafer inverse template, by a process modified from Thakar, R.G., *Biochem Biophys Res Commun,* **307(4)**: 883-90 (2003). To create the micropatterned template, wafers were cleaned in acetone, followed by a 4:1 wash solution of sulfuric acid to hydrogen peroxide (Piranha acid). The wafers were dried and coated with hexamethyldisilazane (HMDS) for 5 minutes to improve photoresist adhesion to the substrate. A mask with patterned emulsion strips was first generated. The parallel strips were 10-µm wide, 10-µm apart, and 4-cm in length. To transfer the pattern to the silicon wafer, I-line (OCG OiR 897-10i, Arch Chemicals, Norwalk, CT) photoresist was spun onto the wafers at 820 RPM for one minute, producing approximately a 2.8-µm thick layer of photoresist. The wafer was then baked at 90°C for 60 seconds to harden the photoresist, before exposing the photoresist to UV Light with a KS Aligner (Karl Suss, MJB3, Germany). A post-exposure bake was performed for 60 seconds at 120°C to help the photoresist set and drive the diffusion of the photoproducts. The exposed photoresist was developed for 60 seconds using a photoresist developer solution OPD 4262. The wafer with unexposed photoresist was placed into the primer oven for 15 minutes at 120 °C to allow the remaining photoresist to set. At this point, the wafers were ready to be used for the preparation of PDMS films. Templates for the fabrication of PLCG films required an additional step of etching with the STS Deep Reactive Ion Etcher for 2-3 minutes to create microgrooves approximately 2 µm deep.

Micropatterned and non-patterned PDMS films were prepared as directed by the manufacturer (Sylgard 184, Dow Corning, MI). After degassing under vacuum, 15 g of PDMS was poured onto the wafer. The wafer with PDMS was placed on photoresist spinner and spun at 100 rpm for 30 seconds, followed by spinning at 200 rpm for 2 minutes, which formed PDMS films with uniform thickness of approximately 350 µm. The wafer with spin-coated PDMS was kept at room temperature for 10 min before placing in an oven at 80 °C for 15 minutes to allow polymerization of PDMS. After baking, the PDMS was cooled to room temperature and then removed from the wafer. After the fabrication process, the PDMS membranes were cleaned by sonication in water. To sterilize and promote cell adhesion, the membranes were treated with oxygen plasma and coated with 2% gelatin for 30 minutes before cell seeding.

For biodegradable micropatterned films, a triblock copolymer PLGC (Aldrich, St. Louis, MO) at a 70:10:20 component ratio (Mₙ ∼100,000) was used. PLGC solution was prepared in chloroform at a concentration of 50 mg/mL and agitated on a stirplate until dissolved. The solution was then poured onto the silicon mold and allowed for the solvent to evaporate, forming thin polymer films. After fabrication, the PLGC films were sterilized in 70% ethanol for 2 hours and rinsed in PBS. Prior to cell seeding, the films were coated with 2% gelatin for 30 minutes to enhance cell attachment.

### EXAMPLE 5

### Characterization of the compositions by Scanning Electron Microscopy (SEM)

Compositions or samples containing cells were processed for SEM by fixation in 2% glutaraldehyde in 0.1 M sodium cacodylate buffer. After ethanol dehydration series, the samples were dried and sputter coated with either iridium or gold:palladium (40:60) particles to a thickness of 10-15 nm. Samples were visualized under an Environmental Scanning Electron Microscope (Philips XL-30).

### EXAMPLE 6

### Immunofluorescent Staining

Samples were fixed in 4% paraformaldehyde for 15 minutes, permeabilized with 0.5% Triton X-100 for 10 minutes, and pretreated with 1% bovine serum albumin (BSA) for 30 minutes. F-actin assembly was stained by fluorescein (FITC)-conjugated phalloidin (5 U/mL, Molecular Probes) incubation for 1 h. For myosin heavy chain staining, samples were incubated with mouse anti-skeletal fast myosin heavy chain, (87 µg/mL, Sigma), followed by the incubation with FITC-conjugated donkey anti-mouse (6.25 µg/mL, Jackson ImmunoResearch, West Grove, PA) antibody. Cell nuclei were stained with ToPro (1µM, Molecular Probes) before visualizing with a Nikon TE300 microscope or Leica TCS SL confocal microscope. Confocal images represent two-dimensional projections of three-dimensional stacked images.

### EXAMPLE 7

### BrdU Incorporation

For cell cycle analysis, samples were pulsed for 2 h with bromodeoxyuridine (BrdU, 1:1000, Amersham, Piscataway, NJ) in growth media before fixing in 4% paraformaldehyde and washing in phosphate buffered saline (PBS). The samples were pretreated with 50% methanol for 30 minutes, then permeabilized in 0.5% Triton-X-100 for 10 minutes, followed by 2N HCl treatment for 30 minutes. Afterwards, samples were incubated with mouse anti-BrdU (2.5 µg/mL, BD Biosciences, Bedford, MA), followed by FITC-conjugated donkey anti-mouse (6.25 µg/mL) antibodies. Cell nuclei were stained with propidium iodide (1 µg/mL, Molecular Probes).

### EXAMPLE 8

### Analysis of Cell Fusion and Proliferation

Immunofluorescent images of anti-skeletal myosin staining were taken under at least 5 representative high-power (40X) and low-power (10X) ficlds. Using SPOT 4.0.5 software (Diagnostic Instruments, Sterling Heights, MI), myotube width, length, and alignment in reference to the axis of the aligned nanofibers or microgrooves were quantified using high and low magnification, respectively. The minimum myotube alignment value of 0 degrees denoted parallel alignment from the axis of the nanofibers and the maximum of 90 degrees represented perpendicular alignment. For alignment analysis ofrandomly-oriented nanofibrous scaffolds and non-patterned PDMS substrates, an arbitrary axis of alignment was used. In addition, the percentages of fused nuclei, BrdU-positive cells, and striated myotubes were quantified and averaged at high power. The average width were quantified and averaged. Serial images under low magnification were merged to quantify myotube length. All data was expressed as mean ± standard deviation (n≥3). Statistical significance was calculated by a student's t-test for two groups or analysis of variance (ANOVA) with Holm's adjustment for multiple comparisons.

### EXAMPLE 9

### Conduits

Myoblasts were differentiated for 7 days on rectangular sheets of aligned nanofibers. To create three-dimensional structures, the nanofiber sheets were rolled around a 1-2 mm diameter steel rod (**FIG. 27**). The tubular structures were secured by 7-0 Ticron sutures on both ends of the tubular constructs. The samples were then cryosectioned for histological analysis. The cryosectioned samples were analyzed by routine hematoxylin and eosin (H&E) staining and by immunofluorescent staining of F-actin.

The fabricated tubular constructs were approximately 2-3 mm in diameter and 10mm in longitudinal length. H&E staining for the cross-sectional gross morphology of these constructs demonstrated that tubular structure of the constructs could be successfully fabricated (**FIG. 28**). Distinctive purple-colored nuclei could be seen throughout the layers of the scaffold, although more cells were visible at the surface of each layer. Confocal microscopy of immunofluorescently stained F-actin demonstrated cell penetration within the multiple layers of the construct (**FIG. 29**). The cells adopted elongated morphology and aligned according to the direction of the nanofibers. These results demonstrate the feasibility of engineering aligned three-dimensional skeletal muscle using nanofiber polymers.

### EXAMPLE 10

### Nanofiber Polymer Production

Biodegradable poly(L-lactide) (PLLA) (Lactel Absorbable Polymers, Pelham, AL, 1.09 dL/g inherent viscosity) was used to fabricate nanofibrous scaffolds by electrospinning as described previously by Rosen *et al., Ann Plast Surg.,* **25**:375-87 (1990). The PLLA solution (10% w/v in chloroform) was delivered by a programmable pump to a grounded collecting plate in a high electric field, resulting in random nanofibers. To align the nanofibers, the electrospun scaffold was stretched uniaxially to 200% strain at 60 °C. Nanofibrous scaffolds were approximately 150 µm in thickness. Scanning electron microscopy (SEM) was used to visualize nanofiber alignment after uniaxial stretching. SEM images show that uniaxial stretching resulted in highly aligned nanofibers (**FIG. 12A-B**). The average nanofiber diameter was approximately 500 nm.

To chemically modify nanofibers, one ECM protein (laminin) and one neurotrophic factor (basic fibroblast factor or bFGF) were selected as representative examples. Laminin and bFGF have both been shown to promote neurite extension in vitro. Manthorpe *et al., J Cell Biol.,* **97**:1882-90 (1983); Rydel *et al., J Neurosci.,* 7:3639-53 (1.987). Moreover, both proteins associate with heparin via their heparin binding domains. Heparin has also been shown to protect bFGF from degradation and plays a key role in the bFGF cell signaling pathway. Gospodarowicz et al., *J Cell Physiol.,* **128:**475-84 (1986); Saksela et al., *J Cell Biol.,* **107**:743-51 (1988); Yayon et al., *Cell.,* **64**:841-8 (1994).

Heparin functionalized nanofibers were created by using di-amino-poly(ethylene glycol) (di-NH₂-PEG) as a linker molecule (**FIG. 12C**). First, the density of reactive carboxylic groups on the PLLA nanofibers was increased by treating the scaffolds with 0.01N NaOH (Sigma, St. Louis, MO). Di-NH₂-PEG (MW 3400, Sigma) molecules were then covalently attached to the carboxylic groups on the PLLA nanofibers using the zero-length cross linkers 1-ethyl-3-(3-dimethylaminopropyl)carbodiimide hydrochloride (EDC) and N-hydroxysulfosuccinimide (Sulfo-NHS) (Pierce Biotechnology, Rockford, IL). Heparin (Sigma) molecules were then covalently attached to the free amines on the di-NH₂-PEG molecules via EDC and sulfo-NHS. Any remaining reactive sites on the nanofibrous scaffolds were blocked by incubating the samples in 10% w/v glycine in phosphate buffer saline (PBS) solution. Then bFGF (50ng/cm², Peprotech, Rocky Hill, NJ)) and laminin (10 µg/cm², Sigma) in PBS solution were incubated with the nanofibrous scaffold sequentially to allow their binding to heparin and immobilization on the surface of nanofibers.

Attachment ofbFGF molecules to heparin functionalized PLLA nanofibers was verified by using a modified ELISA technique. To compare the efficiency of different ways to immobilize bFGF, three nanofibrous scaffolds with same size, untreated, di-NH₂-PEG modified and heparin functionalized nanofiber membranes, were incubated with bFGF in PBS. All membranes were then incubated with 1% bovine serum albumin (BSA) in PBS solution to minimize passive adsorption of antibodies. Subsequently, the samples were incubated with HRP-conjugated anti-bFGF mouse monoclonal IgG antibody (R&D Systems, Minneapolis, MN). The membranes were washed thoroughly, transferred to Eppendorf tubes and incubated with HRP substrate solution (hydrogen peroxide and chromagen, tetramethylbenzidine). The reaction was then stopped using 2N sulfuric acid. The absorbances of the solutions were then read using a spectrophotometer (450 nm wavelength). Nonspecific adsorption of the bFGF antibody was tested on negative control samples and found to be negligible (data not shown). The results (**FIG. 12D**) indicate that bFGF coating was significantly more efficient on nanofibers functionalized with heparin. These results were further verified on tissue culture polystyrene substrates coated with poly(acrylic acid) and conjugated with di-NH₂-PEG and heparin in the same manner as that used on the PLLA nanofibers (**FIG. 12E**).

DRG tissue harvested from P4-P5 rats was used to study neurite extension on the nanofiber scaffolds. The DRG tissue was cultured in neurobasal medium supplemented with B27 and 0.5 mM L-glutamine (Invitrogen, Carlsbad, CA) for 6 days on the following aligned and unaligned PLLA nanofiber scaffolds: untreated, heparin functionalized with laminin (LAM), and heparin functionalized with laminin and bFGF (LAM+bFGF). After 6 days of *ex vivo* culture, neurite extension was analyzed using immunofluorescent staining. All samples were first fixed with 4% paraformaldehyde, and then cell membranes were permeabilized with 0.5% Triton-X100 in PBS solution. The samples were incubated with goat polyclonal anti-neurofilament-M (NFM) IgG antibody (Santa Cruz Biotechnology, Santa Cruz, CA) and subsequently with FITC-conjugated donkey anti-goat IgG antibody (Jackson Immunoresearch, West Grove, PA). The samples were mounted on glass slides and visualized using a Zeiss fluorescence microscope and a Leica confocal fluorescence microscope.

Neurite extension from DRG tissue was not observed on unaligned untreated nanofiber scaffolds (**FIG. 13** **Untreated**). Neurite extension from DRG tissue cultured on unaligned LAM nanofibers was minimal **(****FIG. 13** **LAM**). Various neurites were observed extending from DRG tissue cultured on unaligned LAM+bFGF nanofibers (**FIG. 13** **LAM+bFGF**). The neurites extended outward in a radial fashion from the DRG tissue and lacked uniformity in alignment. Neurite outgrowth from DRG tissue was about 1mm.

In contrast to unaligned untreated nanofibers, neurite extension was observed on untreated aligned nanofibers. The neurites extended from two distinct regions of the DRG tissue and were aligned in the direction of the nanofibers. **FIG. 14** shows the extension of neurites from one of two regions of the tissue. Neurite outgrowth on untreated nanofibers was about 0.7mm. These results indicate that aligned nanofibers provide guidance to induce neurite extension from sensory neurons in DRG tissues. Similarly aligned but longer neurites were observed on aligned LAM nanofibers (**FIG. 14** **LAM**), suggesting that ECM proteins can further enhance the guidance of aligned nanofibers. Neurite outgrowth on LAM nanofibers was about 1.3mm. The longest and most dense neurite extension was observed on aligned LAM+bFGF nanofibers (**FIG. 14** **LAM+bFGF**). Neurite outgrowth on LAM+bFGF outgrowth was about 4.8mm. These results clearly show that chemical cues from laminin and bFGF and the physical cues from aligned nanofiber substrates, individually or in combination, greatly enhance and direct neurite extension from sensory neurons. High-magnification confocal microscopy demonstrated that extending neurites followed the guidance of bioactive nanofibers, and formed distinct patterns on random and aligned nanofibers (**FIG. 15**).

### EXAMPLE 11

### Longitudinally Aligned Polymer Scaffold Conduit

Biodegradable poly(lactic-co-glycolic-acid) (PLGA) (Lactel Absorbable Polymers, Pelham, AL, 0.82 dL/g inherent viscosity) was used to fabricate nanofiber scaffolds by electrospinning. The PLGA solution (20% w/v in HFIP) was delivered by a programmable syringe pump to the exit hole of the electrode at a flow rate of 1 mL/hour. A high-voltage supply was used to apply the voltage at 11 kV. The collector substrate was a grounded steel mandrel attached to a motor capable of rotated the mandrel around its long axis. Teflon tape was wrapped around a section of the mandrel to create a non-conducting region. The mandrel was rotated at a slow speed (<15 rpm) as the PLGA fibers were electrospun. The jet alternated between the two sections of the mandrel separated by the non-conducting Teflon tape region resulting in deposition of PLGA fibers that were aligned parallel to the long axis of the mandrel. The rotation of the mandrel ensured even coverage of PLGA fibers around the mandrel. After electrospinning was completed, the edges of electrospun PLGA fibrous conduit were made blunt with a scalpel and the conduit was then removed from the Teflon tape and mandrel.

In order to verify fiber alignment, the tube was cut along its long axis and the fiber morphology was visualized with a light microscope. A majority of the fibers were aligned in the longitudinal direction (ie along the long axis of the conduit).

### EXAMPLE 12

### Longitudinally Aligned Polymer Scaffold Rod

Biodegradable poly(lactic-co-glycolic-acid) (PLGA) (Lactel Absorbable Polymers, Pelham, AL, 0.82 dL/g inherent viscosity) can be used to fabricate nanofiber scaffolds by electrospinning. The PLGA solution (20% w/v in HFIP) can be delivered by a programmable syringe pump to the exit hole of the electrode at a flow rate of 1 mL/hour. A high-voltage supply can be used to apply the voltage at 11 kV. In order for the electrospinning process to form longitudinally aligned fibers in the shape of a rod, a specialized collector substrate can be used. The collector substrate can consist of two grounded metal mandrels arranged end to end with an air gap in the middle (ie 2 cm) and can be placed below (i.e. 15 cm) the exit hole of the electrode. Each mandrel can be attached to electronically controlled motors that can rotate the mandrels around their long axes in a synchronized manner. During the electrospinning process, the mandrels can be rotated at a slow speed (<10 rpm) to ensure even deposition of electrospun polymer fibers. During the electrospinning process, the polymer solution will form a jet that travels toward the collecting substrate. In this case, the jet will traverse the air gap between the ends of the two mandrels forming fibers that are aligned along the length of the gap (and have the same orientation as the long axis of the mandrels). Upon completion of electrospinning, the electrospun polymer material can be separated from the ends of the two metal mandrels by using a scalpel and cutting along the edges of the mandrels. The result is a rod shaped electrospun fibrous polymer scaffold with fibers aligned along its long axis.

### EXAMPLE 13

### Longitudinally Aligned Filled Conduit Fibrous Polymer Scaffold

To form a conduit composed of longitudinally aligned fibers and filled with longitudinally aligned polymer fibers, the following method can be used. First, a hollow conduit with longitudinally aligned fibers is electrospun. To produce a 2 cm long longitudinally aligned filled conduit with an inner diameter of 0.5 cm, the following conditions may be used. First, a longitudinally aligned hollow conduit can be electrospun as described herein using a mandrel with a non-conducting region of at least 2cm in length and 0.5 cm in diameter. The longitudinally aligned hollow conduit can then be removed from the mandrel and cut to size. The filler material is electrospun as a highly porous sheet of aligned polymer fibers as described herein. The sheet of aligned nanofibers can be shaped to the proper dimensions of the lumen of the hollow conduit. The sheet of aligned fibers is then inserted into the conduit to produce a conduit scaffold filled with polymer fibers aligned along its long axis.

The filler material can be produced as either a rolled sheet composed of aligned fibers or a rod composed of longitudinally aligned fibers. To produce the filler material using a rolled sheet composed of aligned fibers a method described herein may be used. An aligned fibrous polymer membrane with 50 micron thickness can be electrospun and a 2cm x 2cm square section can be cut. The sheet can then be rolled onto itself in such a manner that the aligned fibers run along the length of the rolled sheet. The sheet can be rolled until the diameter of the formed rod is 0.5 cm. Excess material can then be cut. The rolled rod shaped filler material can then be inserted into the longitudinally aligned hollow conduit with forceps to form the longitudinally aligned filled conduit. To produce filler material, a rod shaped fibrous polymer scaffold composed of longitudinally aligned fibers according to a method described herein may be used. In order to produce the properly sized rod, the two collector mandrels can be 0.5 cm in diameter and be spaced at least 2 cm apart to create at least a 2 cm air gap. After the rod is electrospun as in Example 12, it can be removed using a scalpel and cutting along the edges of the mandrels. The rod shaped scaffold can then be inserted within the hollow conduit using forceps.

### EXAMPLE 14

### PLGA Nanofiber Scaffold Preparation

Biodegradable poly(lactic-co-glycolic-acid) (PLGA) (Lactel Absorbable Polymers, Pelham, AL, 1.09 dL/g inherent viscosity) was used to fabricate nanofiber scaffold membranes by electrospinning. The PLGA solution (20% w/v in HFIP) was delivered by a programmable syringe pump to the exit hole of the electrode at a flow rate of 1 mL/hour. A high-voltage supply was used to apply the voltage at 11 kV. The fibers were deposited on a rotating steel drum (see **FIG. 41**) covered (diameter: 10cm, length: 10cm) with aluminum foil that was grounded and controlled by a stepping motor. For aligned nanofibers, the collector drum was rotated at 400 rpm. For unaligned fibers, the collecting drum was rotated at <30 rpm. Electrospinning was conducted until fibrous scaffolds were approximately 130 µm in thickness composed of 500 nm diameter fibers. In order to remove, the fibrous polymer sheet from the drum, the polymer layer and foil were cut along the length of the drum and unwrapped from the drum. The fibrous polymer layer was then peeled from the aluminum foil to produce a fibrous polymer sheet approximately 30 cm in length and 10 cm in width.

Alignment of fibers was checked using a phase contrast microscope (Carl Zeiss), and the fibrous polymer sheets were cut to desired dimensions to fit a given application. For wound healing, nanofiber scaffold pieces can be cut to match the dimensions of the wound, or to expand beyond the wound edges. The orientation of the fibers can be chosen to be aligned at a specific angle with respect to a given axis of the wound.

### EXAMPLE 15

### Criss-cross Nanofiber Scaffold Preparation

A fibrous polymer scaffold with a criss-cross pattern of fibers can be formed through several methods. In one example, electrospinning can be used to create aligned fibrous polymer sheets as previously described in this patent by collecting the fibers on a rotating drum or by stretching unaligned fibers. These aligned polymer sheets are then removed from the collector drum and can be layered on top of each other with the fibers in each sheet aligned orthogonally to the fibers in the sheets above and below (**FIG. 38**). Additionally, the sheets can be sutured together for mechanical strength and stability.

In a second example, the criss-cross pattern can be formed by utilizing a conducting drum with a non-conducting section in the center for electrospinning of longitudinal fibers along the drum. A steel drum 10 cm in diameter and 10 cm in length can be secured to a motor. Teflon tape can be rolled around the drum to cover a 4 cm width it. To create the criss-cross pattern, the drum will first spin slowly (<30 rpm) to create fibers aligned along the longitudinal axis across the non-conducting Teflon tape region. This will be followed by rotating the drum rapidly (>100rpm) to create fibers aligned orthogonally to the first layer. Switching between slow and rapid rotation of the drum will create a series of aligned fiber layers deposited on the Teflon tape region that yield a criss-cross pattern. Alternatively, a drum can be constructed where a non-conducting region is sandwiched between two conducting metal regions similar to the design of the mandrel described previously in this invention.

In a third example, a fibrous polymer scaffold sheet with criss-cross alignment of fibers can be electrospun using a rotating drum as a collector substrate. The electrospinning setup can be assembled as described herein for electrospun aligned fibrous polymer sheets. A layer of aligned fibers can be deposited on the rotating drum. Then the layer of fibers can be peeled from the drum, rotated 90° and placed back onto the drum collector. The drum can again be rotated at a high speed (<100 rpm) to allow for the deposition of aligned fibers. This process can be repeated many times to produce a criss-cross aligned fibrous polymer scaffold sheet in which any given layer of fibers is aligned orthogonally relative to the fiber layers adjacent to it.

### EXAMPLE 16

### Micro/nanofiber Wound Healing

PLLA micro/nanofiber sheets were created as described in Example 10 with either aligned or unaligned fibers. An artificial wound or gap defect was created in a monolayer of normal human dermal fibroblasts (NHDFs) on these fiber sheets as follows. First, an 18 Gauge syringe needle was flattened using a hand vice. Second, nanofibrous PLLA meshes were cut to dimensions of 1 x 1 cm. The flattened needle and the mesh were sterilized in 70% isopropyl alcohol and UV light for 30 min, and the needle was laid securely across the nanofibers in the desired orientation with respect to the fiber alignment - fibers were either parallel, perpendicular, or unaligned with respect to the wound axis **(****FIG. 35**). NHDFs were seeded at confluency over the entire area, but the needle prevented cells from binding underneath (**FIG. 35**). After the cells adhered, the needle was removed, leaving a "wound" in its place. The cultures were kept in a humidified incubator at 37°C with 5% CO₂ for 1-2 days to allow time for cell migration and wound coverage. Before seeding, NHDFs were stained briefly with DiI cell tracker (1:2000 dilution, 10 min) to observe initial wound size and to monitor progression of cell infiltration into the wound.

NHDFs on micro/nanofibers were fixed with 4% paraformaldehyde, permeabilized with 0.1% Triton X-100, and blocked with 1% BSA. For cytoskeletal staining, samples were incubated with anti-whole actin primary antibody for 60 min, followed by incubation with FITC-conjugated anti-goat IgG secondary antibody (Jackson ImmunoResearch) for 60 min. Cytoskeletal features were used to determine cell orientation and morphology, as well as overall organization and wound coverage of the NHDF monolayer. NHDF nuclei were stained with Hoechst for 5 min to allow cell counting. On unaligned fibers, cell migration and wound coverage was only moderate after 24 hours, but was greatly enhanced when the fibers were oriented perpendicularly to the long edges of the wound (**FIG. 36**). Also, NHDFs remained randomly oriented on unaligned fibers, but oriented with the fiber direction on aligned fibers.

### EXAMPLE 17

### Wound Healing

PLLA fibrous polymer scaffold sheets were created for wound healing as described in Example 16 using only aligned fibers. Before cell seeding, the fibrous polymer scaffold sheets were functionalized with laminin and/or bFGF as described in example 10. bFGF was either immobilized to the polymer scaffold sheets as described, or presented in soluble form in the media. Again, an artificial wound or gap defect was created in a monolayer of normal human dermal fibroblasts (NHDFs) on these fiber sheets as described in Example 16. Fiber alignment for all samples was perpendicular with respect to the long axis of the wound. Cell migration and wound coverage was analyzed with immunostaining and microscopy as described in Example 16. NHDFs on untreated fibers did not fully cover the wound area after 24 hours, whereas NHDFs on treated fibers (laminin or bFGF) migrated more quickly into the wound and showed enhanced wound coverage **(****FIG. 37**).

### EXAMPLE 18

### Nanofiber Wound Healing in Animals

Aligned biodegradable polymer nanofiber sheets will be created using a rotating drum collector as described in Example 14. These sheets will be used as wound dressings to aid dermal tissue repair in animals. Surgeons will cut a full thickness gap defect on the backs of rats. The micro/nanofiber sheets will be cut to the dimensions of the wound and sutured into the dermal layer to aid fibroblast migration into the gap. Wound healing and tissue regeneration will be monitored using digital photography, histology, and immunohistochemistry.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference for all purposes.
The invention further includes the subject matter of the claims of PCT/US2007/061253 from which this application is derived, the content of which is reproduced below as numbered paragraphs.
Paragraph 1. A composition comprising a first fibrous polymer scaffold, wherein the fiber or fibers of the first fibrous polymer scaffold are aligned.
Paragraph 2. The composition of paragraph 1, wherein the first fibrous polymer scaffold has a length which is a member selected from about 0.01 cm to about 20 cm, about 0.05 cm to about 5 cm, about 0.5 cm to about 5 cm, about 1 cm to about 5 cm, about 2 cm to about 5 cm, about 1 cm to about 3 cm, about 2 cm to about 10 cm, and about 5 cm to about 15 cm.
Paragraph 3. The composition of paragraph 1, wherein the composition has a shape which is a member selected from a sheet, a conduit, a filled conduit and a rod.
Paragraph 4. The composition of paragraph 1, wherein the composition has a shape which is a member selected from a conduit, a filled conduit and a rod.
Paragraph 5. The composition of paragraph 1, wherein the composition has a rod shape.
Paragraph 6. The composition of paragraph 1, wherein said first fibrous polymer scaffold is essentially aligned in a direction which is a member selected from longitudinal and circumferential.
Paragraph 7. The composition of paragraph 1, wherein said first fibrous polymer scaffold has a seam.
Paragraph 8. The composition of paragraph 1, wherein said first fibrous polymer scaffold is seamless.
Paragraph 9. The composition of paragraph 1, wherein said first fibrous polymer scaffold is monolithically formed.
Paragraph 10. The composition of paragraph 1, wherein at least one of the fibers of the first fibrous polymer scaffold comprises a polymer or subunit which is a member selected from an aliphatic polyester, a polyalkylene oxide, polydimethylsiloxane, polycaprolactone, polylysine, collagen, laminin, fibronectin, elastin, alginate, fibrin, hyaluronic acid, proteoglycans, polypeptides and combinations thereof.
Paragraph 11. The composition of paragraph 10, wherein the aliphatic polyester is a member selected from lactic acid (D- or L-), lactide, poly(lactic acid), poly(lactide) glycolic acid, poly(glycolic acid), poly(glycolide), glycolide, poly(lactide-co-glycolide), poly(lactic acid-co-glycolic acid) and combinations thereof.
Paragraph 12. The composition of paragraph 1, wherein at least one of the fibers of the first fibrous polymer scaffold comprises poly(lactide-co-glycolide) (PLGA).
Paragraph 13. The composition of paragraph 10, wherein the polyalkylene oxide is a member selected from polyethylene oxide, polypropylene oxide and combinations thereof.
Paragraph 14. The composition of paragraph 1, further comprising a cell.
Paragraph 15. The composition of paragraph 14, wherein said cell is embedded within, or is on the surface of the first fibrous polymer scaffold.
Paragraph 16. The composition of paragraph 14, wherein said cell is a member selected from a stem cell and a progenitor cell.
Paragraph 17. The composition of paragraph 14, wherein said cell is a member selected from an adult vascular cell, vascular progenitor cell, vascular stem cell, adult muscle cell, muscle progenitor cell, muscle stem cell, adult neural cell, neural progenitor cell, neural stem cell, Schwann cell, fibroblast cell, adult skin cell, skin progenitor cell, and skin stem cell.
Paragraph 18. The composition of paragraph 1, further comprising a molecule which is covalently attached, either directly or through a linker, to said first fibrous polymer scaffold, and said molecule is capable of either covalently or non-covalently attaching to a member selected from an extracellular matrix component, a growth factor, a differentiation factor and combinations thereof.
Paragraph 19. The composition of paragraph 18, wherein the molecule is covalently attached through a linker, and said linker is a member selected from di-amino poly(ethylene glycol), poly(ethylene glycol) and combinations thereof.
Paragraph 20. The composition of paragraph 18, wherein the molecule is a member selected from heparin, heparan sulfate, heparan sulfate proteoglycan, and combinations thereof.
Paragraph 21. The composition of paragraph 18, wherein the extracellular matrix component is a member selected from laminin, collagen, fibronectin, elastin, vitronectin, fibrinogen, polylysine and combinations thereof.
Paragraph 22. The composition of paragraph 18, wherein the growth factor is a member selected from acidic fibroblast growth factor, basic fibroblast growth factor, nerve growth factor, brain-derived neurotrophic factor, insulin-like growth factor, platelet derived growth factor, transforming growth factor beta, vascular endothelial growth factor, epidermal growth factor, keratinocyte growth factor and combinations thereof.
Paragraph 23. The composition of paragraph 18, wherein the differentiation factor is a member selected from stromal cell derived factor, sonic hedgehog, bone morphogenic proteins, notch ligands, Wnt and combinations thereof.
Paragraph 24. The composition of paragraph 1, wherein said first fibrous polymer scaffold has a conduit, filled conduit or rod shape, and wherein said polymer is seamless.
Paragraph 25. The composition of paragraph 1, produced by applying a polymer solution comprising a polymer to a rotating mandrel.
Paragraph 26. The composition of paragraph 1, wherein said polymer scaffold has a sheet, conduit or filled conduit shape and is produced by an electrospinning process comprising a rotating mandrel with at least one non-conducting region.
Paragraph 27. The composition of paragraph 1, wherein said polymer scaffold has a rod shape and is produced by an electrospinning process comprising a rotating mandrel with an air gap.
Paragraph 28. A pharmaceutical composition comprising:
   (a) the composition of paragraph 1; and
   (b) a pharmaceutically acceptable excipient.
Paragraph 29. The composition of paragraph 1, wherein the composition is a rod or a conduit and wherein at least one of the fibers of the first fibrous polymer scaffold comprises poly(lactide-co-glycolide) (PLGA).
Paragraph 30. The composition of paragraph 29, wherein the composition has a length of between about 0.5 cm and 50 cm.
Paragraph 31. The composition of paragraph 29, further comprising a sleeve which surrounds the first fibrous polymer scaffold.
Paragraph 32. The composition of paragraph 31, wherein said sleeve comprises a second fibrous polymer scaffold, and said second fibrous polymer scaffold is aligned or has a random orientation.
Paragraph 33. The composition of paragraph 31, further comprising a first sleeve which surrounds a first end of the first fibrous polymer scaffold and a second sleeve which surrounds a second end of the first fibrous polymer scaffold.
Paragraph 34. A method of treating an injury in a subject, said method comprising:
   (i) applying the composition of paragraph 1 to a site of interest for said subject, in an amount, and under conditions, sufficient to treat said injury.
Paragraph 35. The method of paragraph 34, wherein said injury is a member selected from a severed nerve, a damaged nerve, a severed muscle, a damaged muscle, a severed blood vessel, a damaged blood vessel, a skin wound and bruised skin.
Paragraph 36. The method of paragraph 35, wherein said injury involves a severed nerve, said first fibrous polymer scaffold has a conduit, filled conduit or rod shape comprising a first end and a second end, and said severed nerve comprises a first nerve stump and a second nerve stump, said applying comprises:
   (ii) attaching said first end of said composition to said first nerve stump; and
   (iii) attaching said second end of said composition to said second nerve stump.
Paragraph 37. The method of paragraph 35, wherein said injury involves a damaged nerve, and said applying comprises a member selected from:
   (ii) wrapping the composition of paragraph 1 around said damaged nerve, wherein said composition has a sheet shape.
Paragraph 38. The method of paragraph 35, wherein said injury involves a damaged nerve, and said applying comprises a member selected from:
   (ii) inserting the composition into said damaged nerve, wherein said first fibrous polymer scaffold has a rod, conduit or filled conduit shape.
Paragraph 39. A method of enhancing nerve growth in a subject, said method comprising:
   (i) applying the composition of paragraph 1 to a nerve site of interest in said subject, in an amount, and under conditions, sufficient to enhance nerve growth.
Paragraph 40. The method of paragraph 35, wherein said injury involves cut skin or bruised skin, said first fibrous polymer scaffold has a sheet shape, and said applying comprises:
   (i) attaching said composition to said cut skin; thereby treating said injury.
Paragraph 41. A method of enhancing skin growth in a subject, wherein said first fibrous polymer scaffold has a sheet shape, said method comprising:
   (i) applying the composition of paragraph 1 to a skin site of interest in said subject, in an amount, and under conditions, sufficient to enhance skin growth.
Paragraph 42. The method of paragraph 35, wherein said injury involves a severed blood vessel, said first fibrous polymer scaffold has a conduit or filled conduit shape comprising a first end and a second end, and said severed blood vessel comprises a first vessel stump and a second vessel stump, said applying comprises:
   (ii) attaching said first end of said composition to said first vessel stump; and
   (iii) attaching said second end of said composition to said second vessel stump.
Paragraph 43. A method of enhancing blood vessel growth in a subject, said method comprising:
   (i) applying the composition of paragraph 1 to a vessel site of interest in said subject, in an amount, and under conditions, sufficient to enhance blood vessel growth.
Paragraph 44. The method of paragraph 35, wherein said injury involves a severed muscle, said first fibrous polymer scaffold has a conduit, filled conduit or rod shape comprising a first end and a second end, and said severed muscle comprises a first muscle stump and a second muscle stump, said applying comprises:
   (ii) attaching said first end of said composition to said first muscle stump; and
   (iii) attaching said second end of said composition to said second muscle stump.
Paragraph 45. The method of paragraph 35, wherein said injury involves a damaged muscle, and said applying comprises a member selected from:
   (ii) wrapping the composition of paragraph 1 around said damaged muscle, wherein said composition has a sheet shape.
Paragraph 46. The method of paragraph 35, wherein said injury involves a damaged muscle, and said applying comprises a member selected from:
   (ii) inserting the composition into said damaged muscle, wherein said first fibrous polymer scaffold has a rod, conduit or filled conduit shape.
Paragraph 47. A method of enhancing muscle growth in a subject, said method comprising:
   (i) applying the composition of paragraph 1 to a muscle site of interest in said subject, in an amount, and under conditions, sufficient to enhance muscle growth.
Paragraph 48. A method of making the composition of paragraph 1.
Paragraph 49. A method of making the composition of paragraph 1, said method comprising:
   (i) subjecting a fiber or fibers to an electrospinning process, thereby making said composition.
Paragraph 50. The method of paragraph 49, wherein said electrospinning process comprises a rotating mandrel having an air gap or at least one non-conducting region.
Paragraph 51. A mandrel for an electrospinning apparatus, comprising:
   a first electrically conducting region;
   a second electrically conducting region; and
   a non-electrically conducting region extending between the first and the second electrically conducting region,
   wherein the non-electrically conducting region is dimensioned and configured to receive a fibrous polymer for the formation of a first fibrous polymer scaffold.
Paragraph 52. The mandrel of paragraph 51, wherein said non-electrically conducting region is a sleeve which is placed around the mandrel.
Paragraph 53. The mandrel of paragraph 52, wherein said non-electrically conducting region is a member selected from tape, electrical tape, teflon, and plastic.
Paragraph 54. The mandrel of paragraph 51, wherein said non-electrically conducting region interconnects the two conducting mandrel regions.
Paragraph 55. The mandrel of paragraph 51, wherein said non-electrically conducting region is a discrete portion extending between the two conducting mandrel regions.
Paragraph 56. The mandrel of paragraph 52, wherein said non-electrically conducting region is a member selected from teflon and plastic.
Paragraph 57. The mandrel of paragraph 51, wherein said non-electrically conducting region has a diameter that is a member selected from larger and smaller than said electrically conducting region.
Paragraph 58. A mandrel for an electrospinning apparatus, comprising:
   a first electrically conducting region and a second electrically conducting region, wherein an air gap located between the first and the second electrically conducting region forms a non-conducting region between the first and the second electrically conducting region.
Paragraph 59. The mandrel of paragraph 58, further comprising:
   a first non-electrically conducting sleeve which is positioned over at least part of the first electrically conducting portion, and
   a second non-electrically conducting sleeve which is positioned over at least part of the second electrically conducting portion.
Paragraph 60. The mandrel of paragraph 51, in combination with an electrospinning system.
Paragraph 61. The mandrel of paragraph 58, in combination with an electrospinning system.
Paragraph 62. An electrospinning system, comprising a rotating mandrel with a non-conducting region or an air gap.
Paragraph 63. The electrospinning system of paragraph 62, wherein a polymer solution is directed through a spinneret and deposited on said mandrel.

## Claims

1. A composition comprising a seamless first fibrous polymer scaffold, said composition having a shape selected from a conduit and a rod, wherein an average diameter of a fiber is from about 100 nm to about 5,000 nm, wherein the fibers of the first fibrous polymer scaffold are aligned at a standard deviation from the longitudinal axis of the composition between 0 degrees and 20 degrees.

2. The composition of claim 1, wherein said first fibrous polymer scaffold has a rod shape and is produced by an electrospinning process comprising a rotating mandrel with an air gap.

3. The composition of claim 1, wherein at least one of the fibers of the first fibrous polymer scaffold comprises a polymer selected from an aliphatic polyester, a polyalkylene oxide, polydimethylsiloxane, polycaprolactone, polylysine, collagen, laminin, fibronectin, elastin, alginate, fibrin, hyaluronic acid, proteoglycans, polypeptides and combinations thereof, optionally:
(i) wherein the aliphatic polyester is a member selected from lactic acid (D- or L-), lactide, poly(lactic acid), poly(lactide) glycolic acid, poly(glycolic acid), poly(glycolide), glycolide, poly(lactide-co-glycolide), poly(lactic acid-co-gycolic acid) and combinations thereof; or
(ii) wherein the polyalkylene oxide is a member selected from polyethylene oxide, polypropylene oxide and combinations thereof.

4. The composition of claim 1:
(a) wherein the composition has a length of between about 0.5 cm and 50 cm; or
(b) further comprising a sleeve which surrounds the first fibrous polymer scaffold, optionally wherein said sleeve comprises a second fibrous polymer scaffold, and fibers of said second fibrous polymer scaffold are aligned at a standard deviation from the longitudinal axis of the composition between 0 degrees and 20 degrees or have a random orientation; or
(c) further comprising a first sleeve which surrounds a first end of the first fibrous polymer scaffold and a second sleeve which surrounds a second end of the first fibrous polymer scaffold.

5. The composition of claim 1, further comprising a cell, optionally:
(a) wherein said cell is embedded within, or is on the surface of the first fibrous polymer scaffold, or
(b) wherein said cell is a member selected from a stem cell and a progenitor cell, or
(c) wherein said cell is a member selected from an adult vascular cell, vascular progenitor cell, vascular stem cell, adult muscle cell, muscle progenitor cell, muscle stem cell, adult neural cell, neural progenitor cell, neural stem cell, Schwann cell, fibroblast cell, adult skin cell, skin progenitor cell, and skin stem cell.

6. The composition of claim 1, further comprising a molecule which is covalently attached, either directly or through a linker, to said first fibrous polymer scaffold, and said molecule is capable of either covalently or non-covalently attaching to a member selected from an extracellular matrix component, a growth factor, a differentiation factor and combinations thereof, optionally
(a) wherein the molecule is covalently attached through a linker, and said linker is a member selected from di-amino poly(ethylene glycol), poly(ethylene glycol) and combinations thereof, or
(b) wherein the growth factor is a member selected from acidic fibroblast growth factor, basic fibroblast growth factor, nerve growth factor, brain-derived neurotrophic factor, insulin-like growth factor, platelet derived growth factor, transforming growth factor beta, vascular endothelial growth factor, epidermal growth factor, keratinocyte growth factor and combinations thereof.

7. The composition of claim 1, produced by applying a polymer solution comprising a polymer to a rotating mandrel, or by an electrospinning process comprising a rotating mandrel with at least one non-conducting region.

8. A pharmaceutical composition comprising:
(a) the composition of claim 1; and
(b) a pharmaceutically acceptable excipient.

9. The composition of claim 1 for use in treating an injury in a subject, wherein
the composition is for application to a site of interest for said subject, in an amount, and under conditions, sufficient to treat said injury.

10. Composition for use according to claim 9 wherein said injury is a member selected from a severed nerve, a damaged nerve, a severed muscle, a damaged muscle, a severed blood vessel, and a damaged blood vessel; optionally
(i) wherein said injury involves a severed nerve, said first fibrous polymer scaffold has a first end and a second end, said nerve comprises a first nerve stump and a second nerve stump, and said application comprises:
(a) attaching said first end of said composition to said first nerve stump; and
(b) attaching said second end of said composition to said second nerve stump; or
(ii) wherein said injury involves a damaged nerve, and said application comprises inserting the composition into an injury gap of said damaged nerve.

11. The composition of claim 1 for use in enhancing nerve growth in a subject; wherein the composition is for application to a nerve site of interest in said subject, in an amount, and under conditions, sufficient to enhance nerve growth.

12. The composition of claim 1, wherein
(a) said fibers of said first fibrous polymer scaffold have an average diameter from about 500 nm to about 1000 nm; or
(b) the composition further comprises a sleeve which surrounds the first fibrous scaffold, wherein the sleeve comprises a second fibrous polymer scaffold comprising a plurality of unaligned fibers, optionally
(i) wherein the said first fibrous polymer scaffold and said second fibrous polymer scaffold comprise the same polymer selected from an aliphatic polyester, polyalkylene oxide, polydimethylsiloxane, polycaprolactone, polylysine, collagen, laminin, fibronectin, elastin, alginate, fibrin, hyaluronic acid, proteoglycans, polypeptides and combinations thereof; or
(ii) wherein the said first fibrous polymer scaffold and said second fibrous polymer scaffold comprise different polymers, wherein said polymer is independently selected from an aliphatic polyester, polyalkylene oxide, polydimethylsiloxane, polycaprolactone, polylysine, collagen, laminin, fibronectin, elastin, alginate, fibrin, hyaluronic acid, proteoglycans, polypeptides and combinations thereof; or
(iii) wherein the shape of said first fibrous polymer scaffold is a rod, optionally
wherein said first fibrous polymer scaffold and said second fibrous polymer scaffold comprise the same polymer selected from an aliphatic polyester, polyalkylene oxide, polydimethylsiloxane, polycaprolactone, polylysine, collagen, laminin, fibronectin, elastin, alginate, fibrin, hyaluronic acid, proteoglycans, polypeptides and combinations thereof; or
wherein said first fibrous polymer scaffold and said second fibrous polymer scaffold comprise different polymers, each said polymer is independently selected from an aliphatic polyester, polyalkylene oxide, polydimethylsiloxane, polycaprolactone, polylysine, collagen, laminin, fibronectin, elastin, alginate, fibrin, hyaluronic acid, proteoglycans, polypeptides and combinations thereof.

13. The composition of claim 1, wherein
(a) said first fibrous polymer scaffold has a length of between about 0.5 cm and 50 cm; or
(b) said first fibrous polymer scaffold has the shape of a conduit and an inner diameter between 1 and 20 mm; or
(c) said first fibrous polymer scaffold has an inner diameter between 1 and 20 mm; or
(d) wherein said first fibrous polymer scaffold is composed of between 1 and 2,000 layers of fibers.

14. The composition of claim 1, wherein at least one of the fibers of the said first fibrous polymer scaffold comprises a biodegradable polymer, optionally wherein the polymer is selected from poly(lactic acid), poly(glycolic acid) or a copolymer thereof.

15. A method of making the composition of claim 1, comprising:
(i) spinning a rotational mandrel assembly comprising a first electrically conducting region, a second electrically conducting region and a third non-electrically conducting region extending between the first and second conducting region; and
(ii) releasing the polymer from a charged spinnerette onto said mandrel assembly, thereby making the composition of claim 1,
optionally wherein the third region of the rotational mandrel assembly is an air gap.
